(19) Europäisches Patentamt
European Patent Office
Office européen des brevets

(11) EP 3 747 879 A1

(12) EUROPEAN PATENT APPLICATION
published in accordance with Art. 153(4) EPC

(43) Date of publication:
**09.12.2020 Bulletin 2020/50**

(21) Application number: **19747944.7**

(22) Date of filing: **30.01.2019**

(51) Int Cl.:
***C07D 401/12*** (2006.01) ***A61K 31/454*** (2006.01)
***A61K 31/4709*** (2006.01) ***A61K 31/4725*** (2006.01)
***A61P 17/06*** (2006.01) ***A61P 37/02*** (2006.01)
***A61P 43/00*** (2006.01)

(86) International application number:
**PCT/JP2019/003046**

(87) International publication number:
**WO 2019/151270 (08.08.2019 Gazette 2019/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2018 JP 2018014813**

(71) Applicant: **Toray Industries, Inc.**
**Tokyo, 103-8666 (JP)**

(72) Inventors:
- **OSUMI, Kazuya**
  **Kamakura-shi, Kanagawa 248-8555 (JP)**
- **MATSUMURA, Yuki**
  **Kamakura-shi, Kanagawa 248-8555 (JP)**
- **HAYASHI, Shinnosuke**
  **Kamakura-shi, Kanagawa 248-8555 (JP)**
- **HOSHI, Masaki**
  **Kamakura-shi, Kanagawa 248-8555 (JP)**
- **VALLET, Martial**
  **Iyo-gun, Ehime 791-3193 (JP)**
- **YOKOSAKA, Shinya**
  **Kamakura-shi, Kanagawa 248-8555 (JP)**
- **AOKI, Takumi**
  **Tokyo 103-8666 (JP)**
- **MEGURO, Hiroyuki**
  **Kamakura-shi, Kanagawa 248-8555 (JP)**
- **KAINO, Mie**
  **Kamakura-shi, Kanagawa 248-8555 (JP)**
- **TAKAGAKI, Kozue**
  **Kamakura-shi, Kanagawa 248-8555 (JP)**
- **SASAKI, Rie**
  **Kamakura-shi, Kanagawa 248-8555 (JP)**

(74) Representative: **Kador & Partner PartG mbB**
**Corneliusstraße 15**
**80469 München (DE)**

# (54) CYCLIC AMINE DERIVATIVE AND PHARMACEUTICAL USE THEREOF

(57) An object of the present invention is to provide a novel compound which has retinoid-related orphan receptor γ antagonist activity and shows a therapeutic effect or a preventive effect on autoimmune diseases, such as psoriasis or alopecia areata, or allergic diseases, such as allergic dermatitis. The present invention provides a cyclic amine derivative represented by the following formula or a pharmacologically acceptable salt thereof.

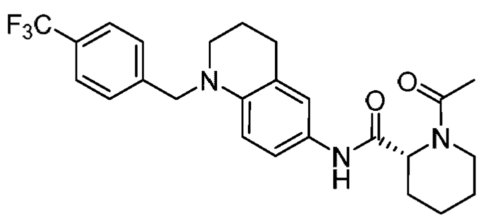

EP 3 747 879 A1

## Description

BACKGROUND OF THE INVENTION

### Field of the Invention

The present invention relates to a cyclic amine derivative and medical use thereof.

### Background Art

An autoimmune disease is a general term for diseases in which excessive immune responses attack an individual's own normal cells and tissues, resulting in symptoms, and examples thereof include multiple sclerosis, psoriasis, rheumatoid arthritis, systemic lupus erythematosus, inflammatory bowel disease, ankylosing spondylitis, uveitis, or polymyalgia rheumatica.

An allergic disease is a disease derived from excessive immune responses to specific antigens, and examples thereof include allergic dermatitis, atopic dermatitis, allergic rhinitis (pollinosis), allergic conjunctivitis, allergic gastroenteritis, bronchial asthma, childhood asthma, or food allergy.

Various mechanisms have been proposed for the onset and progress of autoimmune diseases and allergic diseases. As one of these mechanisms, it is known that Th17 cells, which is one of a subset of helper T cells, and IL-17, which is an inflammatory cytokine produced by Th17 cells, play an important role in the onset and progress of autoimmune diseases (Non-Patent Documents 1 and 2).

IL-17 acts on various cells, such as fibroblasts, epithelial cells, vascular endothelial cells, and macrophages, and is involved in the induction of inflammatory cytokines, chemokines, metalloproteases and other inflammatory mediators and the migration of neutrophils. Therefore, it is considered that potent anti-inflammatory effects are shown if the production or function of IL-17 can be suppressed, and clinical studies of anti-IL-17 antibodies with indications for various autoimmune diseases have been conducted.

Recently, it becomes clear that retinoid-related orphan receptor γ (hereinafter referred to as RORγ), which is a nuclear receptor, functions as a transcription factor essential for the differentiation and proliferation of Th17 cells and the expression of IL-17 (Non-Patent Document 3), and it was shown that suppression of the expression or function of RORγ results in suppression of the differentiation and activation of Th17 cells and the production of IL-17 (Non-Patent Document 4).

It has been reported that the expression level of RORγ in peripheral blood mononuclear cells or skin tissue in patients with autoimmune diseases (multiple sclerosis, psoriasis, systemic lupus erythematosus, etc.) or patients with allergic diseases (allergic dermatitis, etc.) is higher than that of healthy individuals (Non-Patent Documents 5, 6 and 10). It has been reported that, in a knockout mouse of RORγ, the pathological state of a mouse experimental autoimmune encephalomyelitis model, which is an animal model of multiple sclerosis, is suppressed and that symptoms of autoimmune diseases, such as colitis, and symptoms of allergic diseases, such as asthma, are suppressed (Non-Patent Documents 3, 7 and 11).

Furthermore, it is suggested that binding between RORγ and a coactivator is necessary for RORγ to function as a transcription factor (Non-Patent Document 8). Therefore, an RORγ antagonist, which is a compound that inhibits the binding between RORγ and a coactivator, is expected to be useful as a therapeutic agent or preventive agent for autoimmune diseases.

On the other hand, as the RORγ antagonist, N-(5-(N-(4-(1,1,1,3,3,3-hexafluoro-2-hydroxypropan-2-yl)phenyl)s ulfamoyl)-4-methylthiazol-2-yl)acetamide (Non-Patent Document 9), substituted azole derivatives (Patent Document 1), such as 6-(2-chloro-4-methylphenyl)-3-(4-cyclopropyl-5-(3-neopentylcyclo butyl)isoxazol-3-yl)-5-oxohexanoic acid, N-(2-chloro-2'-(trifluoromethoxy)-[1,1'-biphenyl]-4-yl)-2-(4-(meth ylsulfonyl)phenyl)acetamide (Patent Document 2), isoindoline derivatives, such as (S)-1-isopropyl-N-((1-(methylsulfonyl)piperidin-4-yl)methyl)-2-((tr ans-4-(trifluoromethyl)cyclohexyl)methyl)isoindoline-5-carboxamid e (Patent Document 3), and biaryl derivatives, such as 1-acetyl-N-(2-chloro-2'-(trifluoromethoxy)-[1,1'-biphenyl]-4-yl)pip eridine-2-carboxamide (Patent Document 4), have been reported previously.

As the compound having a cyclic amine structure, such as 2-substituted 5,6,7,8-tetrahydro-1,6-naphthyridine, (2-((1-cyclobutylpiperidin-4-yl)oxy)-7,8-dihydro-1,6-naphthyridin-6(5H)-yl)(4-methoxyphenyl)methanone, etc. has been reported as a histamine H3 receptor antagonist (Patent Document 5), and as the compound having a cyclic amine structure, such as 7-substituted 2H-benzo[b][1,4]oxazin-3(4H)-one, methyl 2-benzyl-3-((4-(4-carbamimidoylbenzyl)-3-oxo-3,4-dihydro-2H-be nzo[b][1,4]oxazin-7-yl)amino)-3-oxopropanoate, etc. has been reported as a platelet aggregation inhibitor and a thrombin inhibitor and/or a blood coagulation factor Xa inhibitor (Patent Document 6), but the effects of these compounds on RORγ have been neither disclosed nor suggested.

Prior Art Document

[Patent Document]

[Patent Document 1] JP 2012-236822 A
[Patent Document 2] WO 2013/029338
[Patent Document 3] US 2016/0122318
[Patent Document 4] WO 2017/131156
[Patent Document 5] WO 2010/026113
[Patent Document 6] WO 2005/051934

[Non-Patent Document]

[Non-Patent Document 1] Chen et al., International Immunopharmacology, 2011, Vol. 11, p.536-542
[Non-Patent Document 2] Hofmann et al., Current Opinion in Allergy and Clinical Immunology, 2016, Vol. 16, p.451-457
[Non-Patent Document 3] Ivanov et al., Cell, 2006, Vol. 126, p.1121-1133
[Non-Patent Document 4] Jetten, Nuclear Receptor Signaling, 2009, Vol. 7, e003
[Non-Patent Document 5] Hamzaoui et al., Medical Science Monitor, 2011, Vol. 17, p.CR227-234
[Non-Patent Document 6] Ma et al., Journal of the European Academy of Dermatology and Venereology, 2014, Vol. 28, p.1079-1086
[Non-Patent Document 7] Leppkes et al., Gastroenterology, 2009, Vol. 136, p.257-267
[Non-Patent Document 8] Jin et al., Molecular Endocrinology, 2010, Vol. 24, p.923-929
[Non-Patent Document 9] Solt et al., Nature, 2011, Vol. 472, p.491-494
[Non-Patent Document 10] Zhao et al., British Journal of Dermatology, 2009, Vol. 161, p.1301-1306
[Non-Patent Document 11] Jetten et al., The Journal of Immunology, 2007, Vol. 178, p.3208-3218

## SUMMARY OF THE INVENTION

However, for the actual treatment of autoimmune diseases and allergic diseases, steroids or immunosuppressive agents acting on the whole immune system are used as internal medicines, and due to concerns about serious side effects, such as infection, currently there are many clinical cases in which administration must be discontinued before sufficient drug efficacy is obtained. Therefore, it is desired to develop a new medicament targeted to a molecule playing an important role in the mechanism of the onset and progress of autoimmune diseases and allergic diseases.
Therefore, an object of the present invention is to provide a novel compound which has RORγ antagonist activity and shows a therapeutic effect or a preventive effect on autoimmune diseases, such as psoriasis, or allergic diseases, such as allergic dermatitis.
The present inventors have intensively studied so as to solve the abovementioned problems and found a novel cyclic amine derivative having RORγ antagonist activity, thereby completing the present invention.
That is, the present invention provides a cyclic amine derivative represented by the following formula (I):

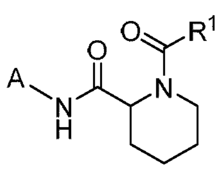

(I)

wherein

$R^1$ represents an alkyl group having 1 to 3 carbon atoms;
A represents a group represented by the following general formula (II-1), (II-2), or (II-3):

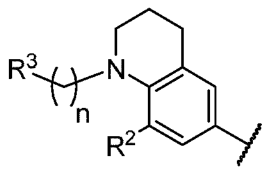

(II-1)

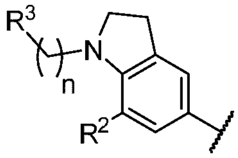

(II-2)

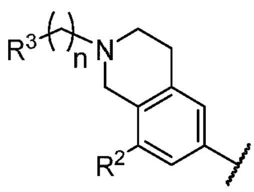

(II-3)

$R^2$ represents a hydrogen atom or a halogen atom;
$R^3$ represents an aryl group or a cycloalkyl group having 4 to 6 carbon atoms, wherein any 1 or 2 hydrogen atoms of the aryl or cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl or cycloalkyl group may be each independently substituted with a halogen atom;
n represents 1 or 2;
a wavy line represents the point of attachment to the general formula (I),
or a pharmacologically acceptable salt thereof.

In the cyclic amine derivative represented by the abovementioned general formula (I), it is preferable that $R^2$ is a hydrogen atom, a fluorine atom, or a chlorine atom, and $R^3$ is an aryl group or a cycloalkyl group having 4 to 6 carbon atoms, wherein any 1 or 2 hydrogen atoms of the aryl or cycloalkyl group represented by $R^3$ may be each independently substituted with a methyl group or a methoxy group, and wherein any 1 to 3 hydrogen atoms of the methyl or methoxy group which can be a substituent of the aryl or cycloalkyl group may be each independently substituted with a fluorine atom or a chlorine atom.

In this case, higher RORγ antagonist activity can be expected.

In the cyclic amine derivative represented by the abovementioned general formula (I), it is more preferable that:

$R^2$ is a fluorine atom or a chlorine atom;
$R^3$ is a phenyl group or a cyclohexyl group, wherein any 1 or 2 hydrogen atoms of the phenyl or cyclohexyl group represented by $R^3$ may be each independently substituted with a methyl group or a methoxy group, and wherein any 1 to 3 hydrogen atoms of the methyl or methoxy group which can be a substituent of the phenyl or cyclohexyl group may be each independently substituted with a fluorine atom or a chlorine atom; and
n is 1.

In this case, higher RORγ antagonist activity can be expected, and furthermore, an excellent therapeutic effect or preventive effect in autoimmune diseases, such as psoriasis, or allergic diseases, such as allergic dermatitis, can be expected.

In the cyclic amine derivative represented by the abovementioned general formula (I), it is still more preferable that:

$R^1$ is a methyl group;
A is a group represented by the following general formula (II-1) or (II-2):

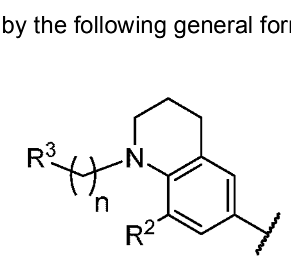

(II-1)

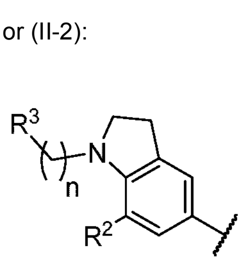

(II-2)

$R^2$ is a chlorine atom, $R^3$ is a phenyl group or a cyclohexyl group, wherein any 1 hydrogen atom of the phenyl or cyclohexyl group represented by $R^3$ may be each independently substituted with a trifluoromethyl group or a trifluoromethoxy group;

n is 1; and
a wavy line represents the point of attachment to the general formula (I).

In this case, higher RORγ antagonist activity can be expected, and furthermore, an excellent therapeutic effect or preventive effect in autoimmune diseases, such as psoriasis, or allergic diseases, such as allergic dermatitis, can be expected.

The present invention also provides a medicament and an RORγ antagonist, each of which contains the cyclic amine derivative represented by the abovementioned general formula (I) or a pharmacologically acceptable salt thereof as an active ingredient.

The abovementioned medicament is preferably a therapeutic agent or preventive agent for an autoimmune disease or an allergic disease, more preferably a therapeutic agent or preventive agent for psoriasis or alopecia areata as the abovementioned therapeutic agent or preventive agent for an autoimmune disease, more preferably a therapeutic agent or preventive agent for allergic dermatitis as the abovementioned therapeutic agent or preventive agent for an allergic disease, and more preferably a therapeutic agent or preventive agent for contact dermatitis or atopic dermatitis as the abovementioned therapeutic agent or preventive agent for allergic dermatitis.

Since the cyclic amine derivative or a pharmacologically acceptable salt thereof according to the present invention has RORγ antagonist activity, it can effectively suppress the function of RORγ and can be used as a therapeutic agent or preventive agent for autoimmune diseases or allergic diseases.

## BRIEF DESCRIPTION OF THE DRAWINGS

FIG. 1 is a graph showing the suppressive effect of the compound of Example 1 on the increase of ear thickness in an imiquimod-induced mouse psoriasis model.
FIG. 2 is a graph showing the suppressive effect of the compound of Example 4 on the increase of ear thickness in an imiquimod-induced mouse psoriasis model.
FIG. 3 is a graph showing the suppressive effect of the compound of Example 9 on the increase of ear thickness in an imiquimod-induced mouse psoriasis model.
FIG. 4 is a graph showing the suppressive effect of the compound of Example 1 on the ear swelling rate in a dinitrofluorobenzene-induced mouse allergic dermatitis model.
FIG. 5 is a graph showing the suppressive effect of the compound of Example 4 on the ear swelling rate in a dinitrofluorobenzene-induced mouse allergic dermatitis model.
FIG. 6 is a graph showing the suppressive effect of the compound of Example 9 on the ear swelling rate in a dinitrofluorobenzene-induced mouse allergic dermatitis model.
FIG. 7 is a graph showing the suppressive effect of the compound of Example 1 on the increase in ear thickness in an oxazolone-induced mouse atopic dermatitis model.
FIG. 8 is a graph showing the suppressive effect of the compound of Example 4 on the increase in ear thickness in an oxazolone-induced mouse atopic dermatitis model.
FIG. 9 is a graph showing the suppressive effect of the compound of Example 9 on the increase in ear thickness in an oxazolone-induced mouse atopic dermatitis model.
FIG. 10 is a graph showing the suppressive effect of the compound of Example 4 on the increase in the hair loss score in a mouse alopecia areata model.

## DETAILED DESCRIPTION OF THE INVENTION

The cyclic amine derivative according to the present invention is characterized by being represented by the following general formula (I):

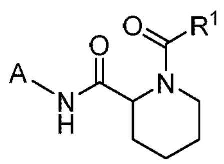

(I)

wherein

$R^1$ represents an alkyl group having 1 to 3 carbon atoms;
A represents a group represented by the following general formula (II-1), (II-2), or (II-3):

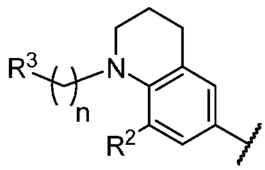

(II-1)

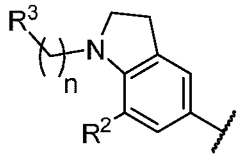

(II-2)

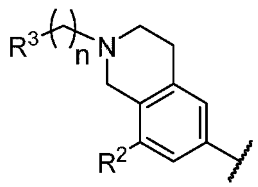

(II-3)

$R^2$ represents a hydrogen atom or a halogen atom;
$R^3$ represents an aryl group or a cycloalkyl group having 4 to 6 carbon atoms, wherein any 1 or 2 hydrogen atoms of the aryl or cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl or cycloalkyl group may be each independently substituted with a halogen atom;
n represents 1 or 2;
a wavy line represents the point of attachment to the general formula (I).

The following terms used herein are defined as follows, unless otherwise specified.

The term "halogen atom" means a fluorine atom, a chlorine atom, a bromine atom, or an iodine atom.

The term "alkyl group having 1 to 3 carbon atoms" means a methyl group, an ethyl group, a propyl group, or an isopropyl group.

The term "any 1 to 3 hydrogen atoms of an alkyl group having 1 to 3 carbon atoms may be each independently substituted with a halogen atom" means an alkyl group having 1 to 3 carbon atoms as defined above, any 1 to 3 hydrogen atoms of which may be each independently substituted with a halogen atom as defined above, in other words, is synonymous with an alkyl group having 1 to 3 carbon atoms, wherein any 1 to 3 hydrogen atoms of the alkyl group may be each independently substituted with a halogen atom as defined above, and examples thereof include a methyl group, an ethyl group, a propyl group, an isopropyl group, a fluoromethyl group, a difluoromethyl group, a trifluoromethyl, a 2-fluoroethyl group, a trifluoroethyl group, a trichloromethyl group, or a trichloroethyl group.

The term "any 1 to 3 hydrogen atoms of a methyl group may be each independently substituted with a fluorine atom or a chlorine atom" means a methyl group, any 1 to 3 hydrogen atoms of which may be each independently substituted with a fluorine atom or a chlorine atom, in other words, is synonymous with a methyl group, wherein any 1 to 3 hydrogen atoms of the methyl group may be each independently substituted with a fluorine atom or a chlorine atom, and examples thereof include a methyl group, a fluoromethyl group, a difluoromethyl group, a trifluoromethyl group, or a trichloromethyl group.

The term "alkyloxy group having 1 to 3 carbon atoms" means a methoxy group, an ethoxy group, a propyloxy group, or an isopropyloxy group.

The term "any 1 to 3 hydrogen atoms of an alkyloxy group having 1 to 3 carbon atoms may be each independently substituted with a halogen atom" means an alkyloxy group having 1 to 3 carbon atoms as defined above, any 1 to 3 hydrogen atoms of which may be each independently substituted with a halogen atom as defined above, in other words, is synonymous with an alkyloxy group having 1 to 3 carbon atoms, wherein any 1 to 3 hydrogen atoms of the alkyloxy group may be each independently substituted with a halogen atom as defined above, and examples thereof include a methoxy group, an ethoxy group, a propyloxy group, an isopropyloxy group, a fluoromethoxy group, a difluoromethoxy group, a trifluoromethoxy group, a 2-fluoroethoxy group, a trifluoroethoxy group, a trichloromethoxy group, or a trichloroethoxy group.

The term "any 1 to 3 hydrogen atoms of a methoxy group may be each independently substituted with a fluorine atom or a chlorine atom" means a methoxy group, any 1 to 3 hydrogen atoms of which may be each independently substituted with a fluorine atom or a chlorine atom, in other words, is synonymous with a methoxy group, wherein any 1 to 3 hydrogen atoms of the methoxy group may be each independently substituted with a fluorine atom or a chlorine atom, and means a methoxy group, a fluoromethoxy group, a difluoromethoxy group, a trifluoromethoxy group, or a trichloromethoxy group.

The term "aryl group" means an aromatic hydrocarbon group, and examples thereof include a phenyl group, a 1-naphthyl group, or a 2-naphthyl group.

The term "cycloalkyl group having 4 to 6 carbon atoms" means a cyclobutyl group, a cyclopentyl group, or a

cyclohexyl group.

The term "aryl group, wherein any 1 or 2 hydrogen atoms of the aryl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl group may be each independently substituted with a halogen atom" means an aryl group as defined above, any 1 or 2 hydrogen atoms of which may be each independently substituted with: an alkyl group having 1 to 3 carbon atoms as defined above, wherein any 1 to 3 hydrogen atoms of the alkyl group may be each independently substituted with a halogen atom as defined above; or an alkyloxy group having 1 to 3 carbon atoms as defined above, wherein any 1 to 3 hydrogen atoms of the alkyloxy group may be each independently substituted with a halogen atom as defined above, and examples thereof include a phenyl group, a 1-naphthyl group, a 2-naphthyl group, a tolyl group, a dimethylphenyl group, an ethylphenyl group, an ethylmethylphenyl group, a propylphenyl group, a methylpropylphenyl group, an isopropylphenyl group, an isopropylmethylphenyl group, a (fluoromethyl)phenyl group, a (difluoromethyl)phenyl group, a (trifluoromethyl)phenyl group, a methyl(trifluoromethyl)phenyl group, an ethyl(trifluoromethyl)phenyl group, a propyl(trifluoromethyl)phenyl group, an isopropyl(trifluoromethyl)phenyl group, a (2-fluoroethyl)phenyl group, a (trifluoroethyl)phenyl group, a (trichloromethyl)phenyl group, a (trichloroethyl)phenyl group, a methoxyphenyl group, a methoxy(methyl)phenyl group, a methoxy(trifluoromethyl)phenyl group, an ethoxyphenyl group, an ethoxy(methyl)phenyl group, an ethoxy(trifluoromethyl)phenyl group, a propyloxyphenyl group, a methyl(propyloxy)phenyl group, a trifluoromethyl(propyloxy)phenyl group, an isopropyloxyphenyl group, an isopropyloxy(methyl)phenyl group, an isopropyloxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, a (trifluoroethoxy)phenyl group, or a (trichloromethoxy)phenyl group.

The term "aryl group, wherein any 1 or 2 hydrogen atoms of the aryl group represented by $R^3$ may be each independently substituted with a methyl group or a methoxy group, and wherein any 1 to 3 hydrogen atoms of the methyl or methoxy group which can be a substituent of the aryl group may be each independently substituted with a fluorine atom or a chlorine atom" means an aryl group as defined above, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a phenyl group, a 1-naphthyl group, a 2-naphthyl group, a tolyl group, a dimethylphenyl group, a (fluoromethyl)phenyl group, a (difluoromethyl)phenyl group, a (trifluoromethyl)phenyl group, a methyl(trifluoromethyl)phenyl group, a (trichloromethyl)phenyl group, a methoxyphenyl group, a methoxy(methyl)phenyl group, a methoxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, or a (trichloromethoxy)phenyl group.

The term "phenyl group, wherein any 1 or 2 hydrogen atoms of the phenyl group represented by $R^3$ may be each independently substituted with a methyl group or a methoxy group, and wherein any 1 to 3 hydrogen atoms of the methyl or methoxy group which can be a substituent of the phenyl group may be each independently substituted with a fluorine atom or a chlorine atom" means a phenyl group, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a phenyl group, a tolyl group, a dimethylphenyl group, a (fluoromethyl)phenyl group, a (difluoromethyl)phenyl group, a (trifluoromethyl)phenyl group, a methyl(trifluoromethyl)phenyl group, a (trichloromethyl)phenyl group, a methoxyphenyl group, a methoxy(methyl)phenyl group, a methoxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, or a (trichloromethoxy)phenyl group.

The term "phenyl group, wherein any 1 hydrogen atom of the phenyl group represented by $R^3$ may be each independently substituted with a trifluoromethyl group or a trifluoromethoxy group" means a phenyl group, a (trifluoromethyl)phenyl group, or a (trifluoromethoxy)phenyl group.

The term "cycloalkyl group having 4 to 6 carbon atoms, wherein any 1 or 2 hydrogen atoms of the cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the cycloalkyl group may be each independently substituted with a halogen atom" means a cycloalkyl group having 4 to 6 carbon atoms as defined above, any 1 or 2 hydrogen atoms of which may be each independently substituted with: an alkyl group having 1 to 3 carbon atoms as defined above, wherein any 1 to 3 hydrogen atoms of the alkyl group may be each independently substituted with a halogen atom as defined above; or an alkyloxy group having 1 to 3 carbon atoms as defined above, wherein any 1 to 3 hydrogen atoms of the

alkyloxy group may be each independently substituted with a halogen atom as defined above, and examples thereof include a cyclobutyl group, a cyclopentyl group, a cyclohexyl group, a methylcyclobutyl group, a dimethylcyclobutyl group, a (trifluoromethyl)cyclobutyl group, a methyl(trifluoromethyl)cyclobutyl group, a methoxycyclobutyl group, a methoxy(methyl)cyclobutyl group, a methoxy(trifluoromethyl)cyclobutyl group, a (trifluoromethoxy)cyclobutyl group, a methyl(trifluoromethoxy)cyclobutyl group, a trifluoromethoxy(trifluoromethyl)cyclobutyl group, a methoxy(trifluoromethoxy)cyclobutyl group, a methylcyclopentyl group, a dimethylcyclopentyl group, a (trifluoromethyl)cyclopentyl group, a methyl(trifluoromethyl)cyclopentyl group, a methoxycyclopentyl group, a methoxy(methyl)cyclopentyl group, a methoxy(trifluoromethyl)cyclopentyl group, a (trifluoromethoxy)cyclopentyl group, a methyl(trifluoromethoxy)cyclopentyl group, a trifluoromethoxy(trifluoromethyl)cyclopentyl group, a methoxy(trifluoromethoxy)cyclopentyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "cycloalkyl group having 4 to 6 carbon atoms, wherein any 1 or 2 hydrogen atoms of the cycloalkyl group represented by $R^3$ may be each independently substituted with a methyl group or a methoxy group, and wherein any 1 to 3 hydrogen atoms of the methyl or methoxy group which can be a substituent of the cycloalkyl group may be each independently substituted with a fluorine atom or a chlorine atom" means a cycloalkyl group having 4 to 6 carbon atoms as defined above, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a cyclobutyl group, a cyclopentyl group, a cyclohexyl group, a methylcyclobutyl group, a dimethylcyclobutyl group, a (trifluoromethyl)cyclobutyl group, a methyl(trifluoromethyl)cyclobutyl group, a methoxycyclobutyl group, a methoxy(methyl)cyclobutyl group, a methoxy(trifluoromethyl)cyclobutyl group, a (trifluoromethoxy)cyclobutyl group, a methyl(trifluoromethoxy)cyclobutyl group, a trifluoromethoxy(trifluoromethyl)cyclobutyl group, a methoxy(trifluoromethoxy)cyclobutyl group, a methylcyclopentyl group, a dimethylcyclopentyl group, a (trifluoromethyl)cyclopentyl group, a methyl(trifluoromethyl)cyclopentyl group, a methoxycyclopentyl group, a methoxy(methyl)cyclopentyl group, a methoxy(trifluoromethyl)cyclopentyl group, a (trifluoromethoxy)cyclopentyl group, a methyl(trifluoromethoxy)cyclopentyl group, a trifluoromethoxy(trifluoromethyl)cyclopentyl group, a methoxy(trifluoromethoxy)cyclopentyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "cyclohexyl group, wherein any 1 or 2 hydrogen atoms of the cyclohexyl group represented by $R^3$ may be each independently substituted with a methyl group or a methoxy group, and wherein any 1 to 3 hydrogen atoms of the methyl or methoxy group which can be a substituent of the cyclohexyl group may be each independently substituted with a fluorine atom or a chlorine atom" means a cyclohexyl group, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a cyclohexyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "cyclohexyl group, wherein any 1 hydrogen atom of the cyclohexyl group represented by $R^3$ may be each independently substituted with a trifluoromethyl group or a trifluoromethoxy group" means a cyclohexyl group, a (trifluoromethyl)cyclohexyl group, or a (trifluoromethoxy)cyclohexyl group.

The term "aryl group or cycloalkyl group having 4 to 6 carbon atoms, wherein any 1 or 2 hydrogen atoms of the aryl or cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl or cycloalkyl group may be each independently substituted with a halogen atom" means an aryl group as defined above or a cycloalkyl group having 4 to 6 carbon atoms as defined above, any 1 or 2 hydrogen atoms of which may be each independently substituted with: an alkyl group having 1 to 3 carbon atoms as defined above, wherein any 1 to 3 hydrogen atoms of the alkyl group may be each independently substituted with a halogen atom as defined above; or an alkyloxy group having 1 to 3 carbon atoms as defined above, wherein any 1 to 3 hydrogen atoms of the alkyloxy group may be each independently substituted with a halogen atom as defined above, and examples thereof include a phenyl group, a 1-naphthyl group, a 2-naphthyl

group, a tolyl group, a dimethylphenyl group, an ethylphenyl group, an ethylmethylphenyl group, a propylphenyl group, a methylpropylphenyl group, an isopropylphenyl group, an isopropylmethylphenyl group, a (fluoromethyl)phenyl group, a (difluoromethyl)phenyl group, a (trifluoromethyl)phenyl group, a methyl(trifluoromethyl)phenyl group, an ethyl(trifluoromethyl)phenyl group, a propyl(trifluoromethyl)phenyl group, an isopropyl(trifluoromethyl)phenyl group, a (2-fluoroethyl)phenyl group, a (trifluoroethyl)phenyl group, a (trichloromethyl)phenyl group, a (trichloroethyl)phenyl group, a methoxyphenyl group, a methoxy(methyl)phenyl group, a methoxy(trifluoromethyl)phenyl group, an ethoxyphenyl group, an ethoxy(methyl)phenyl group, an ethoxy(trifluoromethyl)phenyl group, a propyloxyphenyl group, a methyl(propyloxy)phenyl group, a trifluoromethyl(propyloxy)phenyl group, an isopropyloxyphenyl group, an isopropyloxy(methyl)phenyl group, an isopropyloxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, a (trifluoroethoxy)phenyl group, a (trichloromethoxy)phenyl group, a cyclobutyl group, a cyclopentyl group, a cyclohexyl group, a methylcyclobutyl group, a dimethylcyclobutyl group, a (trifluoromethyl)cyclobutyl group, a methyl(trifluoromethyl)cyclobutyl group, a methoxycyclobutyl group, a methoxy(methyl)cyclobutyl group, a methoxy(trifluoromethyl)cyclobutyl group, a (trifluoromethoxy)cyclobutyl group, a methyl(trifluoromethoxy)cyclobutyl group, a trifluoromethoxy(trifluoromethyl)cyclobutyl group, a methoxy(trifluoromethoxy)cyclobutyl group, a methylcyclopentyl group, a dimethylcyclopentyl group, a (trifluoromethyl)cyclopentyl group, a methyl(trifluoromethyl)cyclopentyl group, a methoxycyclopentyl group, a methoxy(methyl)cyclopentyl group, a methoxy(trifluoromethyl)cyclopentyl group, a (trifluoromethoxy)cyclopentyl group, a methyl(trifluoromethoxy)cyclopentyl group, a trifluoromethoxy(trifluoromethyl)cyclopentyl group, a methoxy(trifluoromethoxy)cyclopentyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "aryl group or cycloalkyl group having 4 to 6 carbon atoms, wherein any 1 or 2 hydrogen atoms of the aryl or cycloalkyl group represented by $R^3$ may be each independently substituted with a methyl group or a methoxy group, and wherein any 1 to 3 hydrogen atoms of the methyl or methoxy group which can be a substituent of the aryl or cycloalkyl group may be each independently substituted with a fluorine atom or a chlorine atom" means an aryl group as defined above or a cycloalkyl group having 4 to 6 carbon atoms as defined above, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a phenyl group, a 1-naphthyl group, a 2-naphthyl group, a tolyl group, a dimethylphenyl group, a (fluoromethyl)phenyl group, a (difluoromethyl)phenyl group, a (trifluoromethyl)phenyl group, a methyl(trifluoromethyl)phenyl group, a (trichloromethyl)phenyl group, a methoxyphenyl group, a methoxy(methyl)phenyl group, a methoxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, a (trichloromethoxy)phenyl group, a cyclobutyl group, a cyclopentyl group, a cyclohexyl group, a methylcyclobutyl group, a dimethylcyclobutyl group, a (trifluoromethyl)cyclobutyl group, a methyl(trifluoromethyl)cyclobutyl group, a methoxycyclobutyl group, a methoxy(methyl)cyclobutyl group, a methoxy(trifluoromethyl)cyclobutyl group, a (trifluoromethoxy)cyclobutyl group, a methyl(trifluoromethoxy)cyclobutyl group, a trifluoromethoxy(trifluoromethyl)cyclobutyl group, a methoxy(trifluoromethoxy)cyclobutyl group, a methylcyclopentyl group, a dimethylcyclopentyl group, a (trifluoromethyl)cyclopentyl group, a methyl(trifluoromethyl)cyclopentyl group, a methoxycyclopentyl group, a methoxy(methyl)cyclopentyl group, a methoxy(trifluoromethyl)cyclopentyl group, a (trifluoromethoxy)cyclopentyl group, a methyl(trifluoromethoxy)cyclopentyl group, a trifluoromethoxy(trifluoromethyl)cyclopentyl group, a methoxy(trifluoromethoxy)cyclopentyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "phenyl group or cyclohexyl group, wherein any 1 or 2 hydrogen atoms of the phenyl or cyclohexyl group represented by $R^3$ may be each independently substituted with a methyl group or a methoxy group, and wherein any 1 to 3 hydrogen atoms of the methyl or methoxy group which can be a substituent of the phenyl or cyclohexyl group may be each independently substituted with a fluorine atom or a chlorine atom" means a phenyl group or a cyclohexyl group, any 1 or 2 hydrogen atoms of which may be each independently substituted with: a methyl group as defined above, wherein any 1 to 3 hydrogen atoms of the methyl group may be each independently substituted with a fluorine atom or a chlorine atom; or a methoxy group as defined above, wherein any 1 to 3 hydrogen atoms of the methoxy group may be each independently substituted with a fluorine atom or a chlorine atom, and examples thereof include a phenyl group, a tolyl group, a dimethylphenyl group, a (fluoromethyl)phenyl group, a (difluoromethyl)phenyl group, a (trifluoromethyl)phenyl group, a methyl(trifluoromethyl)phenyl group, a (trichloromethyl)phenyl group, a methoxyphenyl group, a methoxy(methyl)phenyl group, a methoxy(trifluoromethyl)phenyl group, a (fluoromethoxy)phenyl group, a (difluoromethoxy)phenyl group, a (trifluoromethoxy)phenyl group, a methyl(trifluoromethoxy)phenyl group, a trifluoromethoxy(trifluoromethyl)phenyl group, a methoxy(trifluoromethoxy)phenyl group, a (trichloromethoxy)phenyl group, a cyclohexyl group, a methylcyclohexyl group, a dimethylcyclohexyl group, a (trifluoromethyl)cyclohexyl group, a methyl(trifluoromethyl)cyclohexyl group, a methoxycyclohexyl group, a methoxy(methyl)cyclohexyl group, a methoxy(trifluoromethyl)cyclohexyl group, a (trifluoromethoxy)cyclohexyl group, a methyl(trifluoromethoxy)cyclohexyl group, a trifluoromethoxy(trifluoromethyl)cyclohexyl group, or a methoxy(trifluoromethoxy)cyclohexyl group.

The term "phenyl group or cyclohexyl group, wherein any 1 hydrogen atom of the phenyl or cyclohexyl represented by $R^3$ may be each independently substituted with a trifluoromethyl group or a trifluoromethoxy group" means a phenyl group, a (trifluoromethyl)phenyl group, a (trifluoromethoxy)phenyl group, a cyclohexyl group, a (trifluoromethyl)cyclohexyl group, or a (trifluoromethoxy)cyclohexyl group.

Regarding the abovementioned cyclic amine derivative, in the abovementioned general formula (I), $R^1$ is preferably a methyl group.

A is preferably a group represented by the abovementioned general formula (II-1) or (II-2).

$R^2$ is preferably a hydrogen atom, a fluorine atom, or a chlorine atom, more preferably a fluorine atom or a chlorine atom, and still more preferably a chlorine atom.

$R^3$ is preferably an aryl group or a cycloalkyl group having 4 to 6 carbon atoms, wherein any 1 or 2 hydrogen atoms of the aryl or cycloalkyl group represented by $R^3$ may be each independently substituted with a methyl group or a methoxy group, and wherein any 1 to 3 hydrogen atoms of the methyl or methoxy group which can be a substituent of the aryl or cycloalkyl group may be each independently substituted with a fluorine atom or a chlorine atom, more preferably a phenyl group or a cyclohexyl group, wherein any 1 or 2 hydrogen atoms of the phenyl or cyclohexyl group represented by $R^3$ may be each independently substituted with a methyl group or a methoxy group, and wherein any 1 to 3 hydrogen atoms of the methyl or methoxy group which can be a substituent of the phenyl or cyclohexyl group may be each independently substituted with a fluorine atom or a chlorine atom, and still more preferably a phenyl group or a cyclohexyl group, wherein any 1 hydrogen atom of the phenyl or cyclohexyl group represented by $R^3$ may be each independently substituted with a trifluoromethyl group or a trifluoromethoxy group.

Here, when a substituent(s) exist(s) in the abovementioned cycloalkyl group, for example, a cyclohexyl group, specific examples thereof include a cis-4-(trifluoromethyl)cyclohexyl group, a trans-4-(trifluoromethyl)cyclohexyl group, a cis-4-(trifluoromethoxy)cyclohexyl group, a trans-4-(trifluoromethoxy)cyclohexyl group or the like.

n is preferably 1.

The cyclic amine derivative represented by the abovementioned general formula (I) preferably has a configuration represented by the following general formula (I-a). That is, regarding the cyclic amine derivative represented by the abovementioned general formula (I), the configuration of the carbon atom at position 2 of the piperidinyl group is preferably an R-configuration in the abovementioned general formula (I).

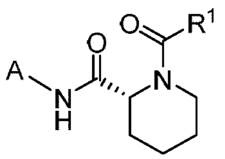

(I-a)

In the cyclic amine derivative represented by the abovementioned general formula (I), it is possible to select any embodiments for the abovementioned preferable $R^1$, the abovementioned preferable $R^2$, the abovementioned preferable $R^3$, the abovementioned preferable n, the abovementioned preferable general formula (I), and the abovementioned preferable A, and to combine them. For example, the following combinations are exemplified, but combinations are not limited thereto.

In the cyclic amine derivative represented by the abovementioned general formula (I), it is preferable that:

$R^1$ is an alkyl group having 1 to 3 carbon atoms;
$R^2$ is a hydrogen atom or a halogen atom;
$R^3$ is an aryl group or a cycloalkyl group having 4 to 6 carbon atoms, wherein any 1 or 2 hydrogen atoms of the aryl or cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl or cycloalkyl group may be each independently substituted with a halogen atom;
n is 1 or 2;
a wavy line is the point of attachment to the general formula (I-a);

the general formula (I) is the following general formula (I-a); and
A is a group represented by the following general formula (II-1), (II-2), or (II-3).

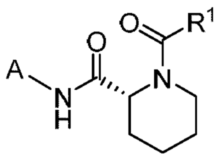

(I-a)

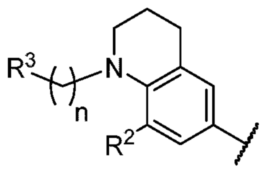

(II-1)

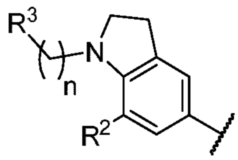

(II-2)

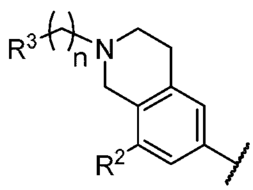

(II-3)

In another embodiment of the cyclic amine derivative represented by the abovementioned general formula (I), it is more preferable that:

$R^1$ is a methyl group;
$R^2$ is a chlorine atom;
$R^3$ is a phenyl group or a cyclohexyl group, wherein any 1 hydrogen atom of the phenyl or cyclohexyl group represented by $R^3$ may be each independently substituted with a trifluoromethyl group or a trifluoromethoxy group;
n is 1;
the general formula (I) is the abovementioned general formula (I-a); and
A is a group represented by the abovementioned general formula (II-1) or (II-2).

Specific examples of preferred compound of the cyclic amine derivative represented by the abovementioned general formula (I) are shown in Table 1, but the present invention is not limited thereto.

[Table 1]

| Structural formula | Structural formula |
|---|---|
| 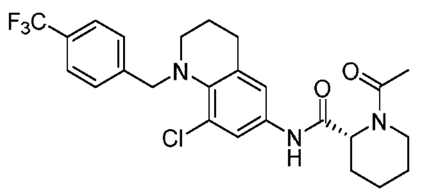 | 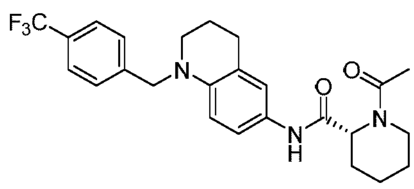 |
| 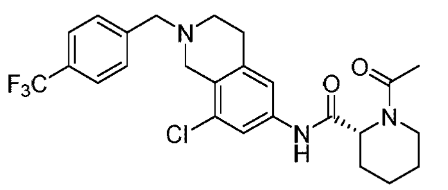 | 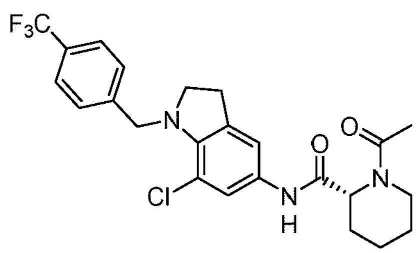 |

(continued)

| Structural formula | Structural formula |
|---|---|
| 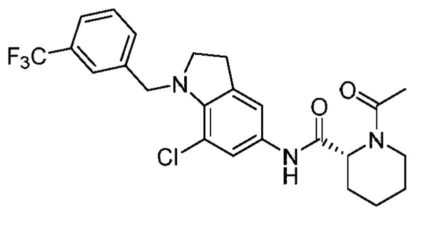 | 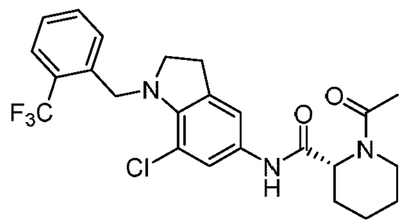 |
| 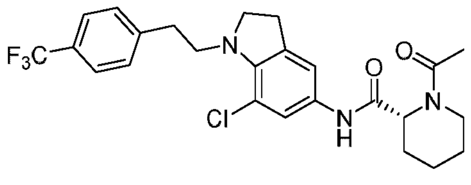 | 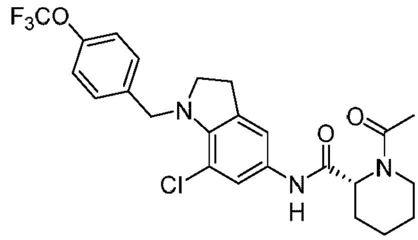 |
| 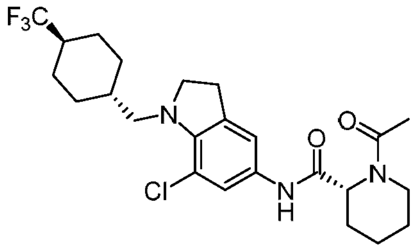 | 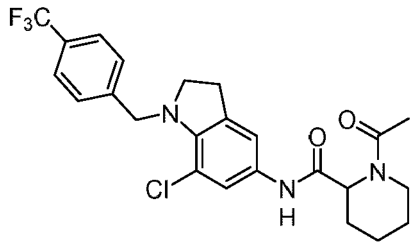 |

The compounds mentioned in Table 1 also include stereoisomers thereof and solvates thereof, and pharmacologically acceptable salts thereof and mixtures thereof.

The cyclic amine derivative represented by the abovementioned general formula (I) might include stereoisomers, and include not only a single stereoisomer but also mixtures of stereoisomers, such as racemates and diastereomer mixtures (for example, mixtures of enantiomers).

The term "stereoisomer" means a compound which has the same chemical structure but has a different configuration in three-dimensional space, and examples thereof include a conformational isomer, a rotamer, a tautomer, an enantiomer, a diastereomer or the like.

The cyclic amine derivative represented by the abovementioned general formula (I) may be labeled with one or more isotopes, and examples of the labeled isotope include $^{2}H$, $^{3}H$, $^{13}C$, $^{14}C$, $^{15}N$, $^{15}O$, $^{18}O$, and/or $^{125}I$.

Examples of the "pharmacologically acceptable salt" of the cyclic amine derivative represented by the abovementioned general formula (I) include a salt with an inorganic acid, or a salt with an organic acid. Examples of the salt with an inorganic acid include a hydrochloride, a sulfate, a nitrate, a hydrobromide, a hydroiodide, or a phosphate, and examples of the salt with an organic acid include an oxalate, a malonate, a citrate, a fumarate, a lactate, a malate, a succinate, a tartrate, an acetate, a trifluoroacetate, a maleate, a gluconate, a benzoate, an ascorbate, a glutarate, a mandelate, a phthalate, a methanesulfonate, an ethanesulfonate, a benzenesulfonate, a p-toluenesulfonate, a camphorsulfonate, an aspartate, a glutamate, or a cinnamate.

The cyclic amine derivative represented by the abovementioned general formula (I) or a pharmacologically acceptable salt thereof may be an anhydride or a solvate, such as a hydrate. Here, the solvate is preferably a pharmacologically acceptable solvate. The pharmacologically acceptable solvate may be either a hydrate or a non-hydrate and is preferably a hydrate. Examples of the solvent constituting the solvate include alcohol-based solvents, such as methanol, ethanol, or n-propanol, N,N-dimethylformamide (hereinafter abbreviated to DMF), dimethylsulfoxide (hereinafter abbreviated to DMSO), or water.

The cyclic amine derivative represented by the abovementioned general formula (I) or a pharmacologically acceptable salt thereof can be a solvate, such as a hydrate, by a known method. Examples of the known method include a method of treating the cyclic amine derivative represented by the abovementioned general formula (I) or a pharmacologically acceptable salt thereof with water, other solvents (for example, alcohol-based solvents, such as methanol, ethanol, or n-propanol, DMF, DMSO), or a mixed solvent thereof.

The cyclic amine derivative represented by the abovementioned general formula (I) (hereinafter referred to as a cyclic amine derivative (I)) can be produced by an appropriate method based on features derived from a basic skeleton and types of substituents thereof. A starting material and a reagent used in the production of these compounds can generally be commercially available or produced by known methods.

The cyclic amine derivative (I) and the intermediate and starting material to be used in the production thereof can be isolated and purified by known means. Examples of known means for isolation and purification include solvent extraction, reprecipitation, recrystallization, or chromatography.

When the cyclic amine derivative (I) contains a stereoisomer, each enantiomer or diastereomer can be obtained as a single optically active substance by a known method. Examples of the known method include crystallization, enzymatic resolution, or chiral chromatography.

Crystallization can be performed according to a known method (for example, Brittain, H.G., "Polymorphism in Pharmaceutical Solids, Second Edition", CRC Press, LLC) or a method analogous thereto. Seed crystals may be used as appropriate.

Examples of the solvent used for crystallization of the cyclic amine derivative (I) or a pharmacologically acceptable salt thereof include ether-based solvents, such as tetrahydrofuran (hereinafter abbreviated to THF), 1,4-dioxane, diethyl ether, tert-butyl methyl ether, or anisole, alcohol-based solvents, such as methanol, ethanol, 2-methoxyethanol, 2-ethoxyethanol, n-propanol, 2-propanol, 2-methyl-1-propanol, n-butanol, 2-butanol, 3-methyl-1-butanol, n-pentanol, or ethylene glycol, aromatic hydrocarbon solvents, such as toluene, xylene, cumene, or tetralin, aprotic polar solvents, such as DMF, N,N-dimethylacetamide, formamide, N-methylpyrrolidone, DMSO, or sulfolane, nitrile-based solvents, such as acetonitrile or propionitrile, ester-based solvents, such as methyl acetate, ethyl acetate, propyl acetate, isopropyl acetate, butyl acetate, isobutyl acetate, or ethyl formate, ketone-based solvents, such as acetone, methyl ethyl ketone, methyl butyl ketone, or methyl isobutyl ketone, halogen-based solvents, such as dichloromethane, chloroform, 1,2-dichloroethene, 1,1,2-trichloroethene, or chlorobenzene, hydrocarbon solvents, such as hexane, pentane, heptane, cyclohexane, or methylcyclohexane, nitro-based solvents, such as nitromethane, pyridine-based solvents, such as pyridine, carboxylic acid-based solvents, such as acetic acid or formic acid, water or a mixed solvent thereof, or a mixed solvent of a solvent thereof and a solvent containing the cyclic amine derivative (I) and a base or an acid which forms the abovementioned pharmacologically acceptable salt.

In each of the reactions of the production methods mentioned below, when the starting compound has an amino group or a carboxyl group, a protective group may be introduced into these groups, and after the reaction, the protective group can be deprotected as appropriate to obtain the target compound.

Examples of the protective group of the amino group include an alkylcarbonyl group having 2 to 6 carbon atoms (e.g., an acetyl group), a benzoyl group, an alkyloxycarbonyl group having 2 to 8 carbon atoms (e.g., a tert-butoxycarbonyl group or a benzyloxy carbonyl group), an aralkyl group having 7 to 10 carbon atoms (e.g., a benzyl group), or a phthaloyl group.

Examples of the protective group of the carboxyl group include an alkyl group having 1 to 6 carbon atoms (e.g., a methyl group, an ethyl group, or a tert-butyl group) or an aralkyl group having 7 to 10 carbon atoms (e.g., a benzyl group).

The deprotection of the protective group varies depending on the kind of the protective group, but deprotection can be performed according to a known method (for example, Greene, TW, "Greene's Protective Groups in Organic Synthesis", Wiley-Interscience) or a method analogous thereto.

As shown in, for example, Scheme 1, the cyclic amine derivative (I) can be obtained by a deprotection reaction (first step) of a tert-butoxycarbonyl group of an N-tert-butoxycarbonylpipecolic acid amide derivative (III) in the presence of an acid, followed by a condensation reaction (second step-1) of the pipecolic acid amide derivative (IV) obtained in the first step with an organic acid chloride derivative (V) in the presence of a base. The cyclic amine derivative (I) can also be obtained by a condensation reaction (second step-2) of the pipecolic acid amide derivative (IV) with an organic acid anhydride derivative (VI). An optically active substance of the cyclic amine derivative (I) can be obtained by, for example, using an optically active substance of the N-tert-butoxycarbonylpipecolic acid amide derivative (III).

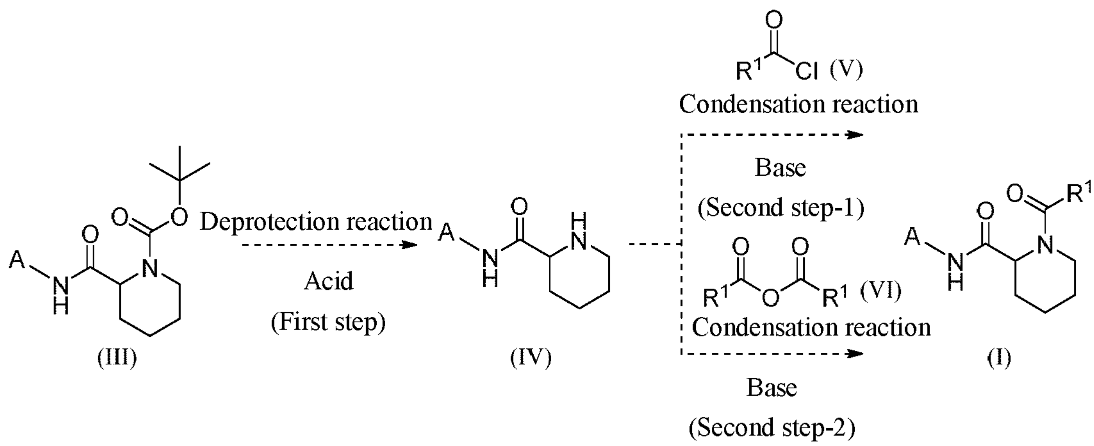


Scheme 1

In Scheme 1, A and $R^1$ are as defined above.

(First step)

Examples of the acid used in the deprotection reaction include an acid, such as hydrochloric acid, trifluoroacetic acid, or hydrofluoric acid, and hydrochloric acid or trifluoroacetic acid is preferable.

The amount of the acid used in the deprotection reaction is preferably 0.5 to 100 equivalents, and more preferably 1 to 30 equivalents, based on the N-tert-butoxycarbonylpipecolic acid amide derivative (III).

The reaction solvent in the deprotection reaction is appropriately selected according to the type of the reagent to be used, but is not particularly limited as long as it does not inhibit the reaction, and examples thereof include ether-based solvents, such as diethyl ether, THF, dimethoxyethane, or 1,4-dioxane, ester-based solvents, such as ethyl acetate or propyl acetate, halogen-based solvents, such as dichloromethane, chloroform, or 1,2-dichloroethane, alcohol-based solvents, such as methanol or ethanol, or mixed solvents thereof, and ester-based solvents, such as ethyl acetate or propyl acetate, or halogen-based solvents, such as dichloromethane, chloroform, or 1,2-dichloroethane, are preferable.

The reaction temperature of the deprotection reaction is preferably -78°C to 200°C, and more preferably -20°C to 100°C.

The reaction time of the deprotection reaction is appropriately selected according to the conditions, such as the reaction temperature, and the reaction time is preferably 1 to 50 hours.

The concentration of the N-tert-butoxycarbonylpipecolic acid amide derivative (III) used in the deprotection reaction at the start of the reaction is preferably 1 mmol/L to 1 mol/L.

The N-tert-butoxycarbonylpipecolic acid amide derivative (III) used in the deprotection reaction can be produced by, for example, the method mentioned in Scheme 2 or Scheme 3 below.

(Second step-1, second step-2)

The amount of the organic acid chloride derivative (V) or organic acid anhydride derivative (VI) used in the condensation reaction is preferably 0.5 to 10 equivalents, and more preferably 1 to 3 equivalents, based on the pipecolic acid amide derivative (IV).

Examples of the base used in the condensation reaction include an organic base, such as triethylamine or diisopropylethylamine, an inorganic base, such as sodium hydrogen carbonate or potassium carbonate, a hydrogenated metal compound, such as sodium hydride, potassium hydride, or calcium hydride, an alkyl lithium, such as methyl lithium or butyl lithium, lithium amide, such as lithium hexamethyldisilazide or lithium diisopropylamide, or a mixture thereof, and an organic base, such as triethylamine or diisopropylethylamine, is preferable.

The amount of the base used in the condensation reaction is preferably 0.5 to 10 equivalents, and more preferably 1 to 5 equivalents, based on the pipecolic acid amide derivative (IV).

The reaction solvent used in the condensation reaction is appropriately selected according to the type of the reagent to be used or the like, but is not particularly limited as long as it does not inhibit the reaction, and examples thereof include ether-based solvents, such as THF, 1,4-dioxane, ethylene glycol dimethyl ether, or dimethoxyethane, halogen-based solvents, such as dichloromethane, chloroform, or 1,2-dichloroethane, aprotic polar solvents, such as DMF or DMSO, or nitrile-based solvents, such as acetonitrile or propionitrile, and halogen-based solvents, such as dichloromethane, chloroform, or 1,2-dichloroethane, are preferable.

The reaction temperature of the condensation reaction is preferably -78°C to 200°C, and more preferably -20°C to 100°C.

The reaction time of the condensation reaction is appropriately selected according to the conditions, such as the reaction temperature, and the reaction time is preferably 0.5 to 30 hours.

The concentration of the pipecolic acid amide derivative (IV) used in the condensation reaction at the start of the reaction is preferably 1 mmol/L to 1 mol/L.

The pipecolic acid amide derivative (IV) used in the condensation reaction may be a free form or a salt, such as a hydrochloride.

The organic acid chloride derivative (V) and the organic acid anhydride derivative (VI) used in the condensation reaction can be purchased or produced by a known method or a method analogous thereto.

Among the N-tert-butoxycarbonylpipecolic acid amide derivative (III) shown in Scheme 1, an N-tert-butoxycarbonylpipecolic acid amide derivative (III-a), A of which is a group represented by the abovementioned general formula (II-1) or (II-2), can be obtained by, for example, as shown in Scheme 2, a reductive amination reaction (first step-1) of a secondary amine derivative (VII-a) with an aldehyde derivative (VIII) in the presence of a reducing agent, or an alkylation reaction (first step-2) of the secondary amine derivative (VII-a) with a halogenated alkyl derivative (IX) in the presence of a base, followed by a halogenation reaction (second step) of the tertiary amine derivative (X) obtained in the first step in the presence of a halogenating agent, followed by a reduction reaction (fourth step) of the nitrophenyl derivative (XI) obtained in the second step or the third step-1 or the third step-2 mentioned below in the presence of a metal and an acid, followed by a condensation reaction (fifth step) of the aniline derivative (XII) obtained in the fourth step with a pipecolic acid derivative (XIII) in the presence of a condensing agent and a base. The nitrophenyl derivative (XI) can also be obtained by a reductive amination reaction (third step-1) of a secondary amine derivative (VII-b) with the aldehyde derivative (VIII), or an alkylation reaction (third step-2) of the secondary amine derivative (VII-b) with the halogenated alkyl derivative (IX) in the presence of a base. When $R^2$ is a hydrogen atom, the N-tert-butoxycarbonylpipecolic acid amide derivative can be obtained by subjecting the tertiary amine derivative (X) obtained in the first step not to the above halogenation reaction (second step) but to the above reduction reaction (fourth step) and the above condensation reaction (fifth step). An optically active substance of the N-tert-butoxycarbonylpipecolic acid amide derivative (III-a) can be obtained by, for example, using an optically active substance of the pipecolic acid derivative (XIII).

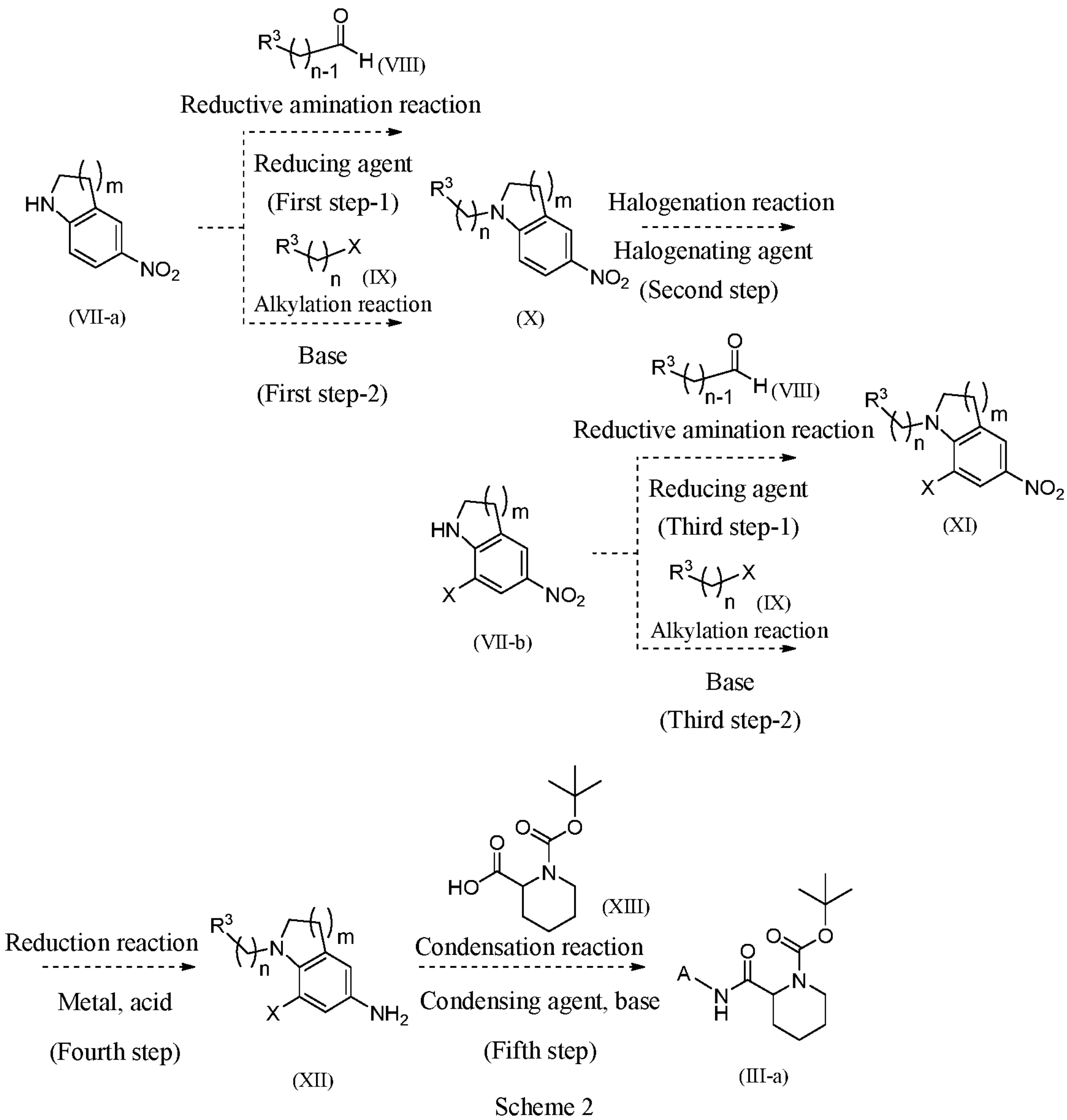


Scheme 2

In Scheme 2, m represents 1 or 2, X represents a halogen atom, A represents a group represented by the abovementioned general formula (II-1) or (II-2), and $R^2$, $R^3$, and n are as defined above.

(First step-1)

The amount of the aldehyde derivative (VIII) used in the reductive amination reaction is preferably 0.5 to 10 equivalents, and more preferably 1 to 3 equivalents, based on the secondary amine derivative (VII-a).

Examples of the reducing agent used in the reductive amination reaction include sodium borohydride, sodium cyanoborohydride, or sodium triacetoxyborohydride, and sodium triacetoxyborohydride is preferable.

The amount of the reducing agent used in the reductive amination reaction is preferably 0.5 to 10 equivalents, and more preferably 1 to 3 equivalents, based on the secondary amine derivative (VII-a).

The reaction solvent used in the reductive amination reaction is appropriately selected according to the type of the reagent to be used, but is not particularly limited as long as it does not inhibit the reaction, and examples thereof include alcohol-based solvents, such as methanol or ethanol, ether-based solvents, such as diethyl ether, THF, dimethoxyethane, or 1,4-dioxane, halogen-based solvents, such as dichloromethane, chloroform, or 1,2-dichloroethane, or mixed solvents thereof, and halogen-based solvents, such as dichloromethane, chloroform, or 1,2-dichloroethane, are preferable.

The reaction temperature of the reductive amination reaction is preferably -78°C to 200°C, and more preferably -20°C to 100°C.

The reaction time of the reductive amination reaction is appropriately selected according to the conditions, such as the reaction temperature, and is preferably 1 to 30 hours.

The concentration of the secondary amine derivative (VII-a) used in the reductive amination reaction at the start of the reaction is preferably 1 mmol/L to 1 mol/L.

The secondary amine derivative (VII-a) used in the reductive amination reaction may be a free form or a salt, such as a hydrochloride.

The secondary amine derivative (VII-a) and the aldehyde derivative (VIII) used in the reductive amination reaction can be purchased or produced by a known method or a method analogous thereto.

(First step-2)

The amount of the halogenated alkyl derivative (IX) used in the alkylation reaction is preferably 0.5 to 10 equivalents, and more preferably 1 to 3 equivalents, based on the secondary amine derivative (VII-a).

Examples of the base used in the alkylation reaction include an organic base, such as triethylamine or diisopropylethylamine, an inorganic base, such as sodium hydrogen carbonate or potassium carbonate, a hydrogenated metal compound, such as sodium hydride, potassium hydride, or calcium hydride, an alkyl lithium, such as methyl lithium or butyl lithium, a lithium amide, such as lithium hexamethyldisilazide or lithium diisopropylamide, or a mixture thereof, and an inorganic base, such as sodium hydrogen carbonate or potassium carbonate, is preferable.

The amount of the base used in the alkylation reaction is preferably 0.5 to 10 equivalents, and more preferably 1 to 3 equivalents, based on the secondary amine derivative (VII-a).

The reaction solvent used in the alkylation reaction is appropriately selected according to the type of the reagent to be used, but is not particularly limited as long as it does not inhibit the reaction, and examples thereof include ether-based solvents, such as THF, 1,4-dioxane, ethylene glycol dimethyl ether, or dimethoxyethane, halogen-based solvents, such as dichloromethane, chloroform, or 1,2-dichloroethane, aprotic polar solvents, such as DMF or DMSO, or nitrile-based solvents, such as acetonitrile or propionitrile, and aprotic polar solvents, such as DMF or DMSO, are preferable.

The reaction temperature of the alkylation reaction is preferably -78°C to 200°C, and more preferably -20°C to 100°C.

The reaction time of the alkylation reaction is appropriately selected according to the conditions, such as the reaction temperature, and is preferably 1 to 30 hours.

The concentration of the secondary amine derivative (VII-a) used in the alkylation reaction at the start of the reaction is preferably 1 mmol/L to 1 mol/L.

The secondary amine derivative (VII-a) used in the alkylation reaction may be a free form or a salt, such as a hydrochloride.

The secondary amine derivative (VII-a) and the halogenated alkyl derivative (IX) used in the alkylation reaction can be purchased or produced by a known method or a method analogous thereto.

(Second step)

Examples of the halogenating agent used in the halogenation reaction include an N-halogenated succinimide derivative of N-chlorosuccinimide (hereinafter abbreviated to NCS), N-bromosuccinimide (hereinafter abbreviated to NBS), or N-iodosuccinimide (hereinafter abbreviated to NIS), an N-halogenated hydantoin derivative of 1,3-dichloro-5,5-dimethylhydantoin, 1,3-dibromo-5,5-dimethylhydantoin, or 1,3-diiodo-5,5-dimethylhydantoin, or a single halogen of chlorine, bromine, or iodine, and an N-halogenated succinimide derivative of NCS, NBS, or NIS is preferable.

The amount of the halogenating agent used in the halogenation reaction is preferably 0.5 to 10 equivalents, and more preferably 1 to 3 equivalents, based on the tertiary amine derivative (X).

The reaction solvent used in the halogenation reaction is appropriately selected according to the type of the reagent to be used, but is not particularly limited as long as it does not inhibit the reaction, and examples thereof include ether-based solvents, such as THF, 1,4-dioxane, ethylene glycol dimethyl ether, or dimethoxyethane, halogen-based solvents, such as dichloromethane, chloroform, or 1,2-dichloroethane, aprotic polar solvents, such as DMF or DMSO, or nitrile-based solvents, such as acetonitrile or propionitrile, and aprotic polar solvents, such as DMF or DMSO, are preferable.

The reaction temperature of the halogenation reaction is preferably -78°C to 200°C, and more preferably -20°C to 100°C.

The reaction time of the halogenation reaction is appropriately selected according to the conditions, such as the reaction temperature, and is preferably 1 to 30 hours.

The concentration of the tertiary amine derivative (X) used in the halogenation reaction at the start of the reaction is preferably 1 mmol/L to 1 mol/L.

The tertiary amine derivative (X) used in the halogenation reaction may be a free form or a salt, such as a

hydrochloride.

(Third step-1)

The amount of the aldehyde derivative (VIII), the reducing agent, the amount of the reducing agent, the reaction solvent, the reaction temperature, the reaction time, and the concentration at the start of the reaction in the reductive amination reaction are the same as in first step-1.

The secondary amine derivative (VII-b) used in the reductive amination reaction may be a free form or a salt, such as a hydrochloride.

The secondary amine derivative (VII-b) and the aldehyde derivative (VIII) used in the reductive amination reaction can be purchased or produced by a known method or a method analogous thereto.

(Third step-2)

The amount of the halogenated alkyl derivative (IX), the base, the amount of the base, the reaction solvent, the reaction temperature, the reaction time, and the concentration at the start of the reaction in the alkylation reaction is the same as in first step-2.

The secondary amine derivative (VII-b) used in the alkylation reaction may be a free form or a salt, such as a hydrochloride.

The secondary amine derivative (VII-b) and the halogenated alkyl derivative (IX) used in the alkylation reaction can be purchased or produced by a known method or a method analogous thereto.

(Fourth step)

Examples of the metal used in the reduction reaction include an iron powder or tin(II) chloride, and an iron powder is preferable.

The amount of the metal used in the reduction reaction is preferably 0.5 to 50 equivalents, and more preferably 1 to 10 equivalents, based on the nitrophenyl derivative (XI).

Examples of the acid used in the reduction reaction include acetic acid, hydrochloric acid, or an aqueous ammonium chloride solution, and acetic acid or an aqueous ammonium chloride solution is preferable.

The amount of the acid used in the reduction reaction is preferably 0.5 to 50 equivalents, and more preferably 1 to 10 equivalents, based on the nitrophenyl derivative (XI).

The reaction solvent used in the reduction reaction is appropriately selected according to the type of the reagent to be used or the like, but is not particularly limited as long as it does not inhibit the reaction, and examples thereof include alcohol-based solvents, such as methanol or ethanol, ether-based solvents, such as diethyl ether, THF, dimethoxyethane, or 1,4-dioxane, water or mixed solvents thereof, and a mixed solvent of alcohol-based solvents, such as methanol or ethanol, ether-based solvents, such as diethyl ether, THF, dimethoxyethane, or 1,4-dioxane, and water is preferable.

The reaction temperature of the reduction reaction is preferably 0 to 200°C, and more preferably 50 to 150°C.

The reaction time of the reduction reaction is appropriately selected according to the conditions, such as the reaction temperature, and is preferably 1 to 30 hours.

The concentration of the nitrophenyl derivative (XI) used in the reduction reaction at the start of the reaction is preferably 1 mmol/L to 1 mol/L.

The nitrophenyl derivative (XI) used in the reduction reaction may be a free form or a salt, such as a hydrochloride.

(Fifth step)

The amount of the pipecolic acid derivative (XIII) used in the condensation reaction is preferably 0.1 to 10 equivalents, and more preferably 0.5 to 3 equivalents, based on the aniline derivative (XII).

Examples of the condensing agent used in the condensation reaction include N,N'-dicyclohexylcarbodiimide, N-ethyl-N'-3-dimethylaminopropylcarbodiimide hydrochloride (hereinafter abbreviated to EDC·HCl), N,N'-carbodiimidazole, {{[(1-cyano-2-ethoxy-2-oxoethylidene)amino]oxy}-4-morpholinom ethylene}dimethylammonium hexafluorophosphate (hereinafter abbreviated to COMU), O-(7-azabenzotriazol-1-yl)-1,1,3,3-tetramethyluronium hexafluorophosphate (hereinafter abbreviated to HATU), or O-(benzotriazol-1-yl)-N,N,N',N'-tetramethyluronium hexafluorophosphate (hereinafter abbreviated to HBTU), and HATU or HBTU is preferable.

The amount of the condensing agent used in the condensation reaction is preferably 0.5 to 10 equivalents, and more preferably 1 to 3 equivalents, based on the aniline derivative (XII).

Examples of the base used in the condensation reaction include an organic base, such as triethylamine or

diisopropylethylamine, an inorganic base, such as sodium hydrogen carbonate or potassium carbonate, a hydrogenated metal compound, such as sodium hydride, potassium hydride, or calcium hydride, an alkyl lithium, such as methyl lithium or butyl lithium, lithium amide, such as lithium hexamethyldisilazide or lithium diisopropylamide, or a mixture thereof, and an organic base, such as triethylamine or diisopropylethylamine, is preferable.

The amount of the base used in the condensation reaction is preferably 0.5 to 10 equivalents, and more preferably 1 to 5 equivalents, based on the aniline derivative (XII).

The reaction solvent used in the condensation reaction is appropriately selected according to the type of the reagent to be used or the like, but is not particularly limited as long as it does not inhibit the reaction, and examples thereof include ether-based solvents, such as THF, 1,4-dioxane, ethylene glycol dimethyl ether, or dimethoxyethane, halogen-based solvents, such as dichloromethane, chloroform, or 1,2-dichloroethane, aprotic polar solvents, such as DMF or DMSO, or nitrile-based solvents, such as acetonitrile or propionitrile, and halogen-based solvents, such as dichloromethane, chloroform, or 1,2-dichloroethane, or aprotic polar solvents, such as DMF or DMSO, are preferable.

The reaction temperature of the condensation reaction is preferably 0 to 200°C, and more preferably 20 to 100°C.

The reaction time of the condensation reaction is appropriately selected according to the conditions, such as the reaction temperature, and the reaction time is preferably 1 to 30 hours.

The concentration of the aniline derivative (XII) used in the condensing reaction at the start of the reaction is preferably 1 mmol/L to 1 mol/L.

The aniline derivative (XII) used in the condensation reaction may be a free form or a salt, such as a hydrochloride.

The pipecolic acid derivative (XIII) used in the condensation reaction can be purchased or produced by a known method or a method analogous thereto.

Among the N-tert-butoxycarbonylpipecolic acid amide derivative (III) shown in Scheme 1, an N-tert-butoxycarbonylpipecolic acid amide derivative (III-b), A of which is a group represented by the abovementioned general formula (II-3), can be obtained by, for example, as shown in Scheme 3, a bromination reaction (first step) of a methylphenyl derivative (XIV) in the presence of NBS and a radical initiator, followed by a cyanation reaction (second step) of the benzyl bromide derivative (XV) obtained in the first step in the presence of a cyanating agent, followed by a reduction-cyclization reaction (third step) of the benzyl cyanide derivative (XVI) obtained in the second step in the presence of a reducing agent, followed by a reduction reaction (fourth step) of the lactam derivative (XVII) obtained in the third step in the presence of a reducing agent, followed by an alkylation reaction (fifth step) of the secondary amine derivative (XVIII) obtained in the fourth step with a halogenated alkyl derivative (IX) in the presence of a base, followed by a coupling reaction (sixth step) of the halogenated aryl derivative (XIX) obtained in the fifth step with a primary pipecolic acid amide derivative (XX) in the presence of a metal catalyst, a ligand, and a base. An optically active substance of the N-tert-butoxycarbonylpipecolic acid amide derivative (III-b) can be obtained by, for example, using an optically active substance of the primary pipecolic acid amide derivative (XX).

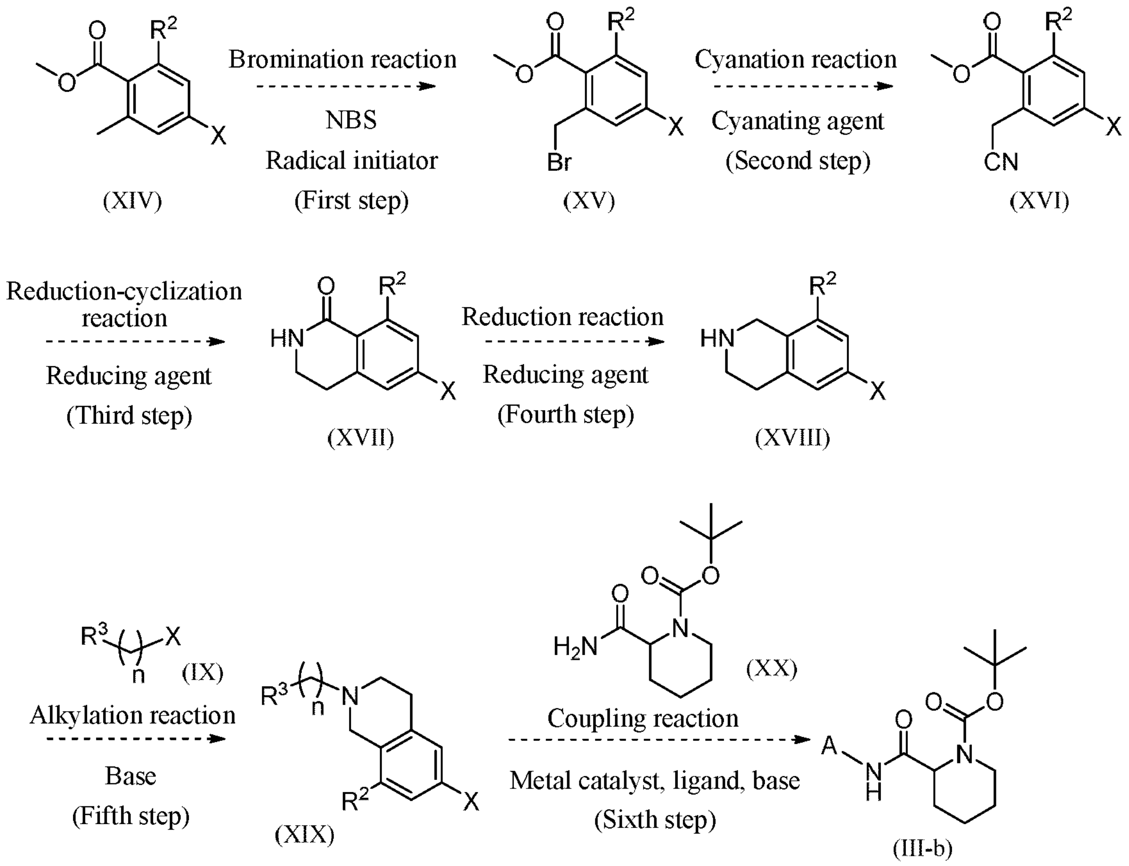


Scheme 3

In Scheme 3, A represents a group represented by the abovementioned general formula (II-3), and $R^2$, $R^3$, n, and X are as defined above.

(First step)

The amount of NBS used in the bromination reaction is preferably 0.5 to 10 equivalents, and more preferably 1 to 3 equivalents, based on the methylphenyl derivative (XIV).

Examples of the radical initiator used in the bromination reaction include 2,2'-azobis(isobutyronitrile) or benzoyl peroxide.

The amount of the radical initiator used in the bromination reaction is preferably 0.01 to 5 equivalents, and more preferably 0.05 to 0.5 equivalents, based on the methylphenyl derivative (XIV).

The reaction solvent used in the bromination reaction is appropriately selected according to the type of the reagent to be used or the like, but is not particularly limited as long as it does not inhibit the reaction, and examples thereof include halogen-based solvents, such as dichloromethane or carbon tetrachloride, or nitrile-based solvents, such as acetonitrile or propionitrile, and halogen-based solvents, such as dichloromethane or carbon tetrachloride, are preferable.

The reaction temperature of the bromination reaction is preferably -78°C to 200°C, and more preferably -20°C to 160°C.

The reaction time of the bromination reaction is appropriately selected according to the conditions, such as the reaction temperature, and is preferably 1 to 30 hours.

The concentration of the methylphenyl derivative (XIV) used in the bromination reaction at the start of the reaction is preferably 1 mmol/L to 1 mol/L.

The methylphenyl derivative (XIV) used in the bromination reaction can be purchased or produced by a known method or a method analogous thereto.

(Second step)

Examples of the cyanating agent used in the cyanation reaction include sodium cyanide or potassium cyanide.

The amount of the cyanating agent used in the cyanation reaction is preferably 0.5 to 10 equivalents, and more preferably 1 to 3 equivalents, based on the benzyl bromide derivative (XV).

The reaction solvent used in the cyanation reaction is appropriately selected according to the type of the reagent to be used or the like, but is not particularly limited as long as it does not inhibit the reaction, and examples thereof include alcohol-based solvents, such as methanol or ethanol, ether-based solvents, such as THF, dimethoxyethane, or 1,4-dioxane, halogen-based solvents, such as dichloromethane, chloroform, or 1,2-dichloroethane, aprotic polar solvents, such as DMF or DMSO, or nitrile-based solvents, such as acetonitrile or propionitrile, water, or mixed solvents thereof, and a mixed solvent of alcohol-based solvents, such as methanol or ethanol, and water is preferable.

The reaction temperature of the cyanation reaction is preferably -20°C to 100°C, and more preferably 0 to 50°C.

The reaction time of the cyanation reaction is appropriately selected according to the conditions, such as the reaction temperature, and is preferably 1 to 30 hours.

The concentration of the benzyl bromide derivative (XV) used in the cyanation reaction at the start of the reaction is preferably 1 mmol/L to 1 mol/L.

(Third step)

Examples of the reducing agent used in the reduction-cyclization reaction include metal hydrides, such as lithium aluminum hydride, aluminum hydride, sodium bis(2-methoxyethoxy)aluminum hydride, a borane-THF complex, sodium borohydride/nickel(II) chloride, or sodium borohydride/cobalt(II) chloride, and sodium borohydride/cobalt(II) chloride is preferable. Here, cobalt(II) chloride may be a hydrate (for example, cobalt(II) chloride hexahydrate).

The amount of the reducing agent used in the reduction-cyclization reaction is preferably 0.5 to 10 equivalents, and more preferably 1 to 3 equivalents, based on the benzyl cyanide derivative (XVI).

The reaction solvent used in the reduction-cyclization reaction is appropriately selected according to the type of the reagent to be used, but is not particularly limited as long as it does not inhibit the reaction, and examples thereof include alcohol-based solvents, such as methanol or ethanol, ether-based solvents, such as diethyl ether, THF, dimethoxyethane, or 1,4-dioxane, or mixed solvents thereof, and a mixed solvent of alcohol-based solvents, such as methanol or ethanol, and ether-based solvents, such as diethyl ether, THF, dimethoxyethane, or 1,4-dioxane, is preferable.

The reaction temperature of the reduction-cyclization reaction is preferably -20°C to 100°C, and more preferably 0 to 50°C.

The reaction time of the reduction-cyclization reaction is appropriately selected according to the conditions, such as the reaction temperature, and is preferably 1 to 30 hours.

The concentration of the benzyl cyanide derivative (XVI) used in the reduction-cyclization reaction at the start of the reaction is preferably 1 mmol/L to 1 mol/L.

(Fourth step)

Examples of the reducing agent used in the reduction reaction include metal hydrides, such as lithium aluminum hydride, aluminum hydride, sodium bis(2-methoxyethoxy)aluminum hydride, or a borane-THF complex, and a borane-THF complex is preferable.

The amount of the reducing agent used in the reduction reaction is preferably 0.5 to 10 equivalents, and more preferably 1 to 3 equivalents, based on the lactam derivative (XVII).

The reaction solvent used in the reduction reaction is appropriately selected according to the type of the reagent to be used, but is not particularly limited as long as it does not inhibit the reaction, and examples thereof include ether-based solvents, such as diethyl ether, THF, dimethoxyethane, or 1,4-dioxane.

The reaction temperature of the reduction reaction is preferably -20°C to 200°C, and more preferably 0 to 100°C.

The reaction time of the reduction reaction is appropriately selected according to the conditions, such as the reaction temperature, and is preferably 1 to 30 hours.

The concentration of the lactam derivative (XVII) used in the reduction reaction at the start of the reaction is preferably 1 mmol/L to 1 mol/L.

(Fifth step)

The amount of the halogenated alkyl derivative (IX) used in the alkylation reaction is preferably 0.5 to 10 equivalents, and more preferably 1 to 3 equivalents, based on the secondary amine derivative (XVIII).

Examples of the base used in the alkylation reaction include an organic base, such as triethylamine or diisopropylethylamine, an inorganic base, such as sodium hydrogen carbonate or potassium carbonate, a hydrogenated metal compound, such as sodium hydride, potassium hydride, or calcium hydride, an alkyl lithium, such as methyl lithium or butyl lithium, a lithium amide, such as lithium hexamethyldisilazide or lithium diisopropylamide, or a mixture thereof,

and an inorganic base, such as sodium hydrogen carbonate or potassium carbonate, is preferable.

The amount of the base used in the alkylation reaction is preferably 0.5 to 10 equivalents, and more preferably 1 to 3 equivalents, based on the secondary amine derivative (XVIII).

The reaction solvent used in the alkylation reaction is appropriately selected according to the type of the reagent to be used, but is not particularly limited as long as it does not inhibit the reaction, and examples thereof include ether-based solvents, such as THF, 1,4-dioxane, ethylene glycol dimethyl ether, or dimethoxyethane, halogen-based solvents, such as dichloromethane, chloroform, or 1,2-dichloroethane, aprotic polar solvents, such as DMF or DMSO, or nitrile-based solvents, such as acetonitrile or propionitrile, and aprotic polar solvents, such as DMF or DMSO, are preferable.

The reaction temperature of the alkylation reaction is preferably -78°C to 200°C, and more preferably -20°C to 100°C.

The reaction time of the alkylation reaction is appropriately selected according to the conditions, such as the reaction temperature, and is preferably 1 to 30 hours.

The concentration of the secondary amine derivative (XVIII) used in the alkylation reaction at the start of the reaction is preferably 1 mmol/L to 1 mol/L.

The secondary amine derivative (XVIII) used in the alkylation reaction may be a free form or a salt, such as a hydrochloride.

The halogenated alkyl derivative (IX) used in the alkylation reaction can be purchased or produced by a known method or a method analogous thereto.

(Sixth step)

The amount of the primary pipecolic acid amide derivative (XX) used in the coupling reaction is preferably 0.5 to 10 equivalents, and more preferably 1 to 3 equivalents, based on the halogenated aryl derivative (XIX).

Examples of the metal catalyst used in the coupling reaction include 1,1'-bis(diphenylphosphino)ferrocene dichloropalladium(II) dichloromethane adduct, palladium(II) acetate, tris(dibenzylideneacetone)dipalladium(0), or tetrakis(triphenylphosphine)palladium(0), and tris(dibenzylideneacetone)dipalladium(0) is preferable.

The amount of the metal catalyst used in the coupling reaction is preferably 0.01 to 5 equivalents, and more preferably 0.05 to 0.5 equivalents, based on the halogenated aryl derivative (XIX).

Examples of the ligand used in the coupling reaction include tri-tert-butylphosphine, 1,1'-bis(diphenylphosphino)ferrocene, 4,5-bis(diphenylphosphino)-9,9-dimethylxanthene, or 2,2'-bis(diphenylphosphino)-1,1'-binaphthyl, and 4,5-bis(diphenylphosphino)-9,9-dimethylxanthene is preferable.

The amount of the ligand used in the coupling reaction is preferably 0.01 to 5 equivalents, and more preferably 0.05 to 0.5 equivalents, based on the halogenated aryl derivative (XIX).

Examples of the base used in the coupling reaction include an organic base, such as triethylamine or diisopropylethylamine, an inorganic base, such as potassium carbonate or cesium carbonate, a lithium amide, such as lithium hexamethyldisilazide or lithium diisopropylamide, or a metal alkoxide, such as sodium tert-butoxide, potassium tert-butoxide, or a mixture thereof, and an inorganic base, such as potassium carbonate or cesium carbonate, is preferable.

The amount of the base used in the coupling reaction is preferably 0.5 to 10 equivalents, and more preferably 1 to 5 equivalents, based on the halogenated aryl derivative (XIX).

The reaction solvent used in the coupling reaction is appropriately selected according to the type of reagent to be used or the like, but is not particularly limited as long as it does not inhibit the reaction, and examples thereof include ether-based solvents, such as THF, 1,4-dioxane, ethylene glycol dimethyl ether, or dimethoxyethane, nitrile-based solvents, such as acetonitrile or propionitrile, aromatic hydrocarbon solvents, such as benzene or toluene, aprotic polar solvents, such as DMF or DMSO, water, or a mixture thereof, and ether-based solvents, such as THF, 1,4-dioxane, ethylene glycol dimethyl ether, or dimethoxyethane, are preferable.

The reaction temperature of the coupling reaction is preferably 0 to 200°C, and more preferably from 50 to 150°C.

The reaction time of the coupling reaction is appropriately selected according to the conditions, such as the reaction temperature, and is preferably 1 to 60 hours.

The concentration of the halogenated aryl derivative (XIX) used in the coupling reaction at the start of the reaction is preferably 1 mmol/L to 1 mol/L.

The primary pipecolic acid amide derivative (XX) used in the coupling reaction can be purchased or produced by a known method or a method analogous thereto.

The medicament, the RORγ antagonist, and the therapeutic agent or preventive agent for an autoimmune disease or allergic disease according to the present invention are characterized by containing the cyclic amine derivative (I) or a pharmacologically acceptable salt thereof as an active ingredient. The autoimmune disease mentioned above is preferably psoriasis or alopecia areata, and the allergic disease mentioned above is preferably allergic dermatitis, and more preferably contact dermatitis or atopic dermatitis.

"RORγ antagonist" means a compound having an effect to suppress the function of RORγ, thereby eliminating

or attenuating the activity thereof.

"Autoimmune disease" is a general term for diseases in which excessive immune responses attack an individual's own normal cells and tissues, resulting in symptoms, and examples thereof include multiple sclerosis, psoriasis, rheumatoid arthritis, systemic lupus erythematosus, inflammatory bowel disease, ankylosing spondylitis, uveitis, polymyalgia rheumatica, scleroderma, vasculitis, pemphigus, pemphigoid, or dermatomyositis. In addition, the autoimmune disease herein includes acne, vitiligo, or alopecia areata.

"Allergic disease" is a disease derived from excessive immune responses to specific antigens, and examples thereof include allergic dermatitis, contact dermatitis, atopic dermatitis, allergic rhinitis (pollinosis), allergic conjunctivitis, allergic gastroenteritis, bronchial asthma, childhood asthma, or food allergy.

"Psoriasis" is an inflammatory disease of the skin associated with invasion and activation of immune cells and resultant acanthosis. Typically, a symptom called desquamation in which white scales thickly adhere to red rashes in various parts of the whole body and then peel off occurs. Examples of psoriasis include plaque psoriasis, pustular psoriasis, psoriasis arthropathica, guttate psoriasis, and erythrodermic psoriasis.

"Alopecia areata" is a disease which develops as a result of temporary impairment of hair matrix cells due to some kind of cause; which lacks prodromal symptoms or subjective symptoms; and in which well-defined patches of hair loss appear suddenly. According to the severity, alopecia areata is classified into ophiasis in which hair loss occurs in the hairlines in the occipital to temporal regions of the head, alopecia totalis in which the entire scalp hair is lost due to fusion of patches of hair loss, and alopecia universalis in which not only the scalp hair but also the hair of the whole body are lost.

"Allergic dermatitis" is a general term for skin diseases caused by allergic reactions and is characterized by chronic itching and rashes on the face, neck, elbow and/or knee. Examples of allergic dermatitis include contact dermatitis, atopic dermatitis, and the like.

"Contact dermatitis" is an eczematous inflammatory disease that develops when an exogenous antigen is brought into contact with the skin, and examples thereof include allergic contact dermatitis, photocontact dermatitis, systemic contact dermatitis, or contact urticaria. Examples of the antigen include metal allergens (cobalt, nickel, etc.), plant allergens (poison oak, primrose, etc.), or food allergens (mango, ginkgo nut, etc.).

"Atopic dermatitis" is a skin disease in which many patients have atopic predisposition. It is characterized by symmetric systemic eczema that repeats exacerbation and remission. Examples thereof include diffuse neurodermatitis, atopic eczema, atopic neurodermatitis, Besnier prurigo, acute infantile eczema, flexural eczema, pediatric eczema in extremities, pediatric atopic eczema, pediatric dry eczema, pediatric eczema, adult atopic dermatitis, endogenic eczema, infantile dermatitis, or chronic infantile eczema.

The cyclic amine derivative (I) or a pharmacologically acceptable salt thereof is characterized by suppressing the function of ROR$\gamma$ by inhibiting the binding between ROR$\gamma$ and a coactivator. Since it is known that ROR$\gamma$ is involved in various diseases and that improvement in the pathological state or remission of the symptoms can be expected by suppression of the function of ROR$\gamma$, the cyclic amine derivative (I) or a pharmacologically acceptable salt thereof can be used as a medicament for diseases in which improvement in the pathological state or remission of the symptoms can be expected by suppression of the function of ROR$\gamma$, particularly as a therapeutic agent or preventive agent for autoimmune diseases or allergic diseases. The therapeutic agent or preventive agent for autoimmune diseases mentioned above can be preferably used as a therapeutic agent or preventive agent for multiple sclerosis, psoriasis, rheumatoid arthritis, systemic lupus erythematosus, inflammatory bowel disease, ankylosing spondylitis, uveitis, polymyalgia rheumatica, scleroderma, vasculitis, pemphigus, pemphigoid, dermatomyositis, acne, vitiligo, or alopecia areata, and more preferably used as a therapeutic agent or preventive agent for psoriasis or alopecia areata. The therapeutic agent or preventive agent for allergic diseases mentioned above can be preferably used as a therapeutic agent or preventive agent for allergic dermatitis, atopic dermatitis, allergic rhinitis (pollinosis), allergic conjunctivitis, allergic gastroenteritis, bronchial asthma, childhood asthma, or food allergy, and more preferably used as a therapeutic agent or preventive agent for contact dermatitis or atopic dermatitis.

It is possible to evaluate that the cyclic amine derivative (I) or a pharmacologically acceptable salt thereof has ROR$\gamma$ antagonist activity that inhibits the binding between ROR$\gamma$ and a coactivator, using an in vitro study. Examples of the in vitro study include a method of evaluating the binding between ROR$\gamma$ and an agonist (e.g., cholesterol) (WO 2012/158784, WO 2013/018695) and a method of evaluating the binding between a ligand-binding domain of ROR$\gamma$ and a coactivator (WO 2012/064744, WO 2013/018695). The inhibitory effect on the transcription activity of ROR$\gamma$ can be evaluated using various reporter gene assays (WO 2012/158784, WO 2012/064744, WO 2013/018695).

The fact that the cyclic amine derivative (I) or a pharmacologically acceptable salt thereof suppresses the function of ROR$\gamma$ can be evaluated using lymphocytic cells derived from various organs, such as spleen or peripheral blood, with IL-17 production or Th17 cell differentiation as an index. Examples of the method using IL-17 production as an index include a method of measuring IL-17 production by IL-23 stimulation using mouse splenocytes (The Journal of Biological Chemistry, 2003, Vol. 278, No. 3, p.1910-1914). Examples of the method using Th17 cell differentiation as an index include a method of measuring the IL-17 production amount or the proportion of IL-17-positive cells, etc. by

stimulating CD4-positive naive T cells derived from mouse splenocytes or human PBMC with various cytokines (e.g., IL-1β, IL-6, IL-23, and/or TGF-β) and various antibodies (e.g., anti-CD3 antibody, anti-CD28 antibody, anti-IL-4 antibody, anti-IFN-y antibody, and/or anti-IL-2 antibody) to be differentiated into Th17 (WO 2012/158784, WO 2013/018695).

The fact that the cyclic amine derivative (I) or a pharmacologically acceptable salt thereof is effective for treatment or prevention of autoimmune diseases can be evaluated using a disease model. Examples of the disease model include an experimental autoimmune encephalomyelitis model (Journal of Neuroscience Research, 2006, Vol. 84, p.1225-1234), an imiquimod-induced psoriasis model (Journal of Immunology, 2009, Vol. 182, p.5836-5845), a collagen arthritis model (Annual Review of Immunology, 1984, Vol. 2, p.199-218), a spontaneous model of systemic lupus erythematosus (Nature, 2000, Vol. 404, p.995-999), a TNBS-induced colitis model (European Journal of Pharmacology, 2001, Vol. 431, p.103-110), an ankylosing spondylitis model (Arthritis Research & Therapy, 2012, Vol. 14, p.253-265), an experimental autoimmune uveitis model (Journal of Immunology, 2006, Vol. 36, p.3071-3081), a scleroderma model (Journal of Investigative Dermatology, 1999, Vol. 112, p.456-462), a vasculitis model (The Journal of Clinical Investigation, 2002, Vol. 110, p.955-963), a pemphigus model (The Journal of Clinical Investigation, 2000, Vol. 105, p.625-631), a pemphigoid model (Experimental Dermatology, 2012, Vol. 21, p.901-905), a dermatomyositis model (American Journal of Pathology, 1985, Vol. 120, p.323-325), a spontaneous model of acne (European Journal of Dermatology, 2005, Vol. 15, p.459-464), a vitiligo model (Pigment Cell & Melanoma Research, 2014, Vol. 27, p.1075-1085), or an alopecia areata model (Journal of Investigative Dermatology, 2015, Vol. 135, p.2530-2532). The experimental autoimmune encephalomyelitis model is generally used as a multiple sclerosis model. The imiquimod-induced psoriasis model is generally used as a psoriasis model.

The alopecia areata model is a disease model in which lymphocytes of a donor mouse with spontaneous hair loss are transplanted into a recipient mouse to induce systemic hair loss (Journal of Investigative Dermatology, 2015, Vol. 135, p.2530-2532). Due to the similarity of its symptoms and pathological findings to those of humans, the model has been widely used for studying the drug efficacy of a therapeutic agent or a prophylactic agent for alopecia areata. For example, in this disease model, a suppressive effect on hair loss symptoms has been observed by administration of ruxolitinib, which is a Janus kinase (JAK) inhibitor (Nature Medicine, 2014, no.20, p.1043-1049). Furthermore, by administration of ruxolitinib to patients with alopecia areata, a hair growth effect has been observed in 75% of patients in the treatment group (Journal of Clinical Investigation Insight, 2016, no.22, e89790). These results also showed that the drug efficacy evaluation using this disease model is useful for studying a therapeutic agent or a preventive agent for alopecia areata.

The fact that the cyclic amine derivative (I) or a pharmacologically acceptable salt thereof is effective for treatment or prevention of allergic diseases can be evaluated using a disease model. Examples of the disease model include a dinitrofluorobenzene (hereinafter abbreviated to DNFB)-induced allergic dermatitis model (Pharmacological Reports, 2013, Vol. 65, p.1237-1246), an oxazolone-induced atopic dermatitis model (Journal of Investigative Dermatology, 2014, Vol. 134, p.2122-2130), an ovalbumin-induced allergic rhinitis model (Journal of Animal Science, 2010, Vol. 81, p.699-705), an IgE-induced allergic conjunctivitis model (British Journal of Ophthalmology, 2012, Vol. 96, p.1332-1336), an allergic gastroenteritis model (Gastroenterology, 1997, Vol. 113, p.1560-1569), an ovalbumin-induced asthma model (American Journal of Respiratory and Critical Care Medicine, 1997, Vol. 156, p.766-775), or an ovalbumin-induced food allergy model (Clinical & Experimental Allergy, 2005, Vol. 35, p.461-466). The DNFB-induced allergic dermatitis model is generally used as an allergic dermatitis model, particularly as a contact dermatitis model. The oxazolone-induced atopic dermatitis model is generally used as an atopic dermatitis model.

The efficacy of the cyclic amine derivative (I) or a pharmacologically acceptable salt thereof for treatment or prevention of autoimmune diseases or allergic diseases can be evaluated using the in vitro study mentioned above, for example, with decrease in the amount of binding between a ligand-binding domain of RORγ and a coactivator, or decrease in the IL-17 production amount, which is an index of the function of RORγ, as an index. The efficacy for treatment or prevention of multiple sclerosis can be evaluated using the experimental autoimmune encephalomyelitis model mentioned above, for example, with decrease in the neurological symptom score, which is a characteristic index of multiple sclerosis, as an index. The efficacy for treatment or prevention of psoriasis can be evaluated using the imiquimod-induced psoriasis model mentioned above, for example, with decrease in the thickness of the skin, such as auricle, which increases with progression of symptoms of the psoriasis model, as an index. The efficacy for treatment or prevention of alopecia areata can be evaluated using the alopecia areata model mentioned above, for example, with decrease in the hair loss score, which is a characteristic index of alopecia areata, as an index. The efficacy for treatment or prevention of allergic dermatitis, particularly contact dermatitis, can be evaluated using the DNFB-induced allergic dermatitis model mentioned above, for example, with decrease in the thickness of the skin, such as auricle, which increases with progression of dermatitis symptoms, as an index. The efficacy for treatment or prevention of atopic dermatitis can be evaluated using the oxazolone-induced atopic dermatitis model mentioned above, for example, with decrease in the thickness of the skin, such as auricle, which increases with progression of dermatitis symptoms, as an index.

The cyclic amine derivative (I) or a pharmacologically acceptable salt thereof can be used as a useful medicament (particularly, a therapeutic agent or preventive agent for an autoimmune disease or an allergic disease) when

administered to a mammal (for example, mouse, rat, hamster, rabbit, dog, cat, monkey, bovine, sheep, or human), particularly human. When the cyclic amine derivative (I) or a pharmacologically acceptable salt thereof is clinically used as a medicament, the cyclic amine derivative (I) or a pharmacologically acceptable salt thereof directly or in combination with a pharmaceutically acceptable carrier can be orally or parenterally administered. The medicament mentioned above can be appropriately mixed with additives, such as binders, excipients, lubricants, disintegrants, sweetening agents, stabilizers, flavoring agents, perfumes, colorants, fluidizers, preservatives, buffers, solubilizers, emulsifiers, surfactants, suspending agents, diluents, or isotonizing agents as appropriate. Examples of the pharmaceutically acceptable carrier include these additives. The medicament mentioned above can be manufactured by a usual method using these drug carriers appropriately. Examples of the dosage form of the medicament mentioned above include oral preparations, such as tablets, capsules, granules, powders, or syrups; parenteral preparations, such as inhalants, injections, suppositories, or liquids; or ointments, creams, or patches for topical administration. Furthermore, known long-acting preparations may be used.

Examples of the binder include syrup, gelatin, gum arabic, sorbitol, polyvinyl chloride, or tragacanth.

Examples of the excipient include sugar, lactose, cornstarch, calcium phosphate, sorbitol, or glycine.

Examples of the lubricant include magnesium stearate, calcium stearate, polyethylene glycol, talc, or silica.

Examples of the disintegrant include starch or calcium carbonate.

Examples of the sweetening agent include glucose, fructose, invert sugar, sorbitol, xylitol, glycerin, or simple syrup.

The medicament mentioned above preferably contains 0.00001 to 90% by weight, and more preferably 0.01 to 70% by weight of the cyclic amine derivative (I) or a pharmacologically acceptable salt thereof. The dose is appropriately selected according to the symptoms, age, and body weight of the patient, and the administration method. As an amount of effective ingredients for an adult, 0.1 μg to 1 g per day for injections, 1 μg to 10 g per day for oral preparations, and 1 μg to 10 g per day for patches are preferable, and each can be administered once or several times in divided doses.

To supplement or enhance the therapeutic or preventive effect or to reduce the dosage, the medicament may be used in mixture or combination with other medicaments in suitable amounts.

The present invention will be described in more detail by way of the following Reference Examples and Examples, but the present invention is not limited thereto.

## EXAM PLES

Commercially available compounds were used for the compounds used in the synthesis of the compounds of Reference Examples and Examples, no mention being made on the synthesis method thereof. "Room temperature" in the following Reference Examples and Examples usually indicates the temperature in a range of about 10°C to about 35°C. Percentage (%) is mol/mol% for yield, % by volume for solvents used in column chromatography and high-performance liquid chromatography, and % by weight for others unless otherwise specified. The name of the solvent shown in the NMR data indicates the solvent used for the measurement. 400 MHz NMR spectra were measured using JNM-AL 400 nuclear magnetic resonance spectrometer (JEOL Ltd.) or JNM-ECS 400 nuclear magnetic resonance spectrometer (JEOL Ltd.). Chemical shifts were reported by δ (unit: ppm) with tetramethylsilane as a standard, signals were indicated by s (singlet), d (doublet), t (triplet), q (quartet), quint (quintet), sept (septet), m (multiplet), br (broad), dd (double doublet), dt (double triplet), ddd (double double doublet), dq (double quartet), td (triplet doublet), and tt (triplet triplet). Mentioned is not made when protons of a hydroxyl group, an amino group or the like have a very gentle peak. ESI-MS spectra were measured using Agilent Technologies 1200 Series, G6130A (Agilent Technologies). Silica gel 60 (Merck) was used as silica gel, amine silica gel DM 1020 (Fuji Silysia Chemical Ltd.) was used as amine silica gel, and YFLC W-prep2XY (Yamazen Corporation) was used as chromatography.

(Reference Example 1) Synthesis of 8-chloro-6-nitro-1-(4-(trifluoromethyl)benzyl)-1,2,3,4-tetrahydroqu inoline:

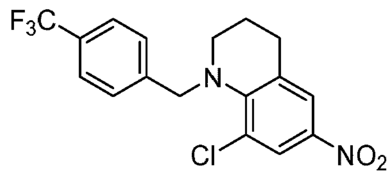

6-Nitro-1,2,3,4-tetrahydroquinoline (1.50 g, 8.42 mmol) was dissolved in DMF (10.0 mL), and potassium carbonate (1.40 g, 10.1 mmol) and 1-(bromomethyl)-4-(trifluoromethyl)benzene (2.21 g, 9.26 mmol) were added at room

temperature. After stirring the reaction mixture at the same temperature for 16 hours, distilled water was added to the reaction mixture and the aqueous layer was extracted with diethyl ether. The organic layer was washed with brine, dried over anhydrous sodium sulfate and filtered, and then the filtrate was concentrated under reduced pressure. The obtained crude product was used for the subsequent reaction without further purification.

The above crude product was dissolved in DMF (2.0 mL) and NCS (0.271 g, 2.03 mmol) was added at room temperature. After stirring at the same temperature for 16 hours, an aqueous sodium thiosulfate solution was added to the reaction mixture, and the aqueous layer was extracted with diethyl ether. The organic layer was washed with brine, dried over anhydrous sodium sulfate and filtered, and then the filtrate was concentrated under reduced pressure. The obtained residue was purified by silica gel column chromatography (n-hexane/ethyl acetate = 95/5 to 80/20) to obtain the title compound (hereinafter referred to as the compound of Reference Example 1) (0.488 g, 1.32 mmol, 16%) as a yellow solid. $^1$H-NMR (400MHz, $CDCl_3$) δ: 3.11-3.15(m, 2H), 3.64-3.68(m, 2H), 5.05(s, 2H), 7.41(d, J=8.2Hz, 2H), 7.63(d, J=8.2Hz, 2H), 7.81-7.82(m, 1H), 8.05(d, J=2.3Hz, 1H).

(Reference Example 2) Synthesis of 8-chloro-1-(4-(trifluoromethyl)benzyl)-1,2,3,4-tetrahydroquinoline-6-amine:

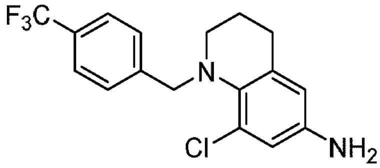

The compound of Reference Example 1 (0.488 g, 1.32 mmol) was dissolved in a mixed soluton of ethanol (5.0 mL) and distilled water (2.0 mL), and ammonium chloride (0.704 g, 13.2 mmol) and iron powder (0.514 g, 9.21 mmol) were added at room temperature. After stirring the reaction mixture at 80°C for 4 hours, an aqueous saturated sodium hydrogen carbonate solution was added to the reaction mixture, followed by filtration. To the filtrate, distilled water was added and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 85/15 to 70/30) to obtain the title compound (hereinafter referred to as the compound of Reference Example 2) (0.402 g, 1.18 mmol, 73%) as a yellow oil. $^1$H-NMR (400MHz, DMSO-$D_6$) δ: 1.73-1.79(m, 2H), 2.75(t, J=6.6Hz, 2H), 2.80-2.83(m, 2H), 4.19(s, 2H), 6.72(d, J=2.3Hz, 1H), 6.94(d, J=2.3Hz, 1H), 7.75(s, 4H).
ESI-MS:m/z = 342$(M+H)^+$.

(Reference Example 3) Synthesis of tert-butyl (R)-2-((8-chloro-1-(4-(trifluoromethyl)benzyl)-1,2,3,4-tetrahydroq uinolin-6-yl)carbamoyl)piperidine-1-carboxylate:

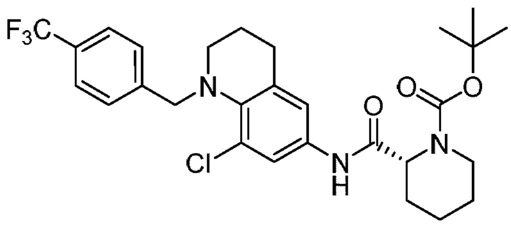

The compound of Reference Example 2 (0.0300 g, 0.0880 mmol) and (R)-1-(tert-butoxycarbonyl)piperidine-2-carboxylic acid (0.0242 g, 0.106 mmol) were dissolved in DMF (1.0 mL), and HATU (0.0402 g, 0.106 mmol) and diisopropylethylamine (0.0231 mL, 0.132 mmol) were added at 0°C. After stirring the reaction mixture at room temperature for 3 hours, distilled water was added to the reaction mixture and the aqueous layer was extracted with diethyl ether. The organic layer was washed with an aqueous saturated sodium hydrogen carbonate solution and brine, dried over anhydrous sodium sulfate and filtered, and then the filtrate was concentrated under reduced pressure. The obtained residue was purified by silica gel column chromatography (n-hexane/ethyl acetate = 95/5 to 85/15) to obtain the title compound (hereinafter referred to as the compound of Reference Example 3) (0.0324 g, 0.0587 mmol, 67%) as a white

amorphous.
$^1$H-NMR (400MHz, $CDCl_3$) δ: 1.42-1.71(m, 5H), 1.52(s, 9H), 1.77-1.83(m, 2H), 2.32-2.35(m, 1H), 2.80-2.86(m, 3H), 2.91-2.93(m, 2H), 4.07(brs, 1H), 4.24(s, 2H), 4.85-4.85(m, 1H), 7.22(d, J=2.3Hz, 1H), 7.39(d, J=2.3Hz, 1H), 7.61(d, J=8.2Hz, 2H), 7.69(d, J=8.2Hz, 2H), 8.08(brs, 1H).
ESI-MS:m/z = 552(M+H)$^+$.

(Example 1) Synthesis of (R)-1-acetyl-N-(8-chloro-1-(4-(trifluoromethyl)benzyl)-1,2,3,4-tetr ahydroquinolin-6-yl)piperidine-2-carboxamide:

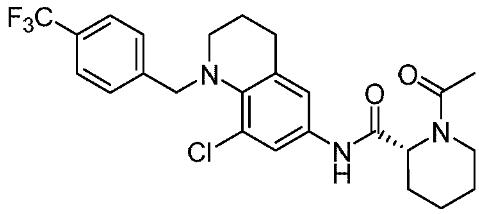

The compound of Reference Example 3 (0.0300 g, 0.0543 mmol) was dissolved in dichloromethane (1.0 mL) and trifluoroacetic acid (0.250 mL, 3.26 mmol) was added at 0°C. After stirring the reaction mixture at room temperature for 2 hours, an aqueous saturated sodium hydrogen carbonate solution was added to the reaction mixture and the aqueous layer was extracted with ethyl acetate. The organic layer was washed with brine, dried over anhydrous sodium sulfate and filtered, and then the filtrate was concentrated under reduced pressure. The obtained crude product was used for the subsequent reaction without purification.
The above crude product was dissolved in dichloromethane, and triethylamine (0.0114 mL, 0.0815 mmol) and acetyl chloride (0.00464 mL, 0.0652 mmol) were added at 0°C. After stirring at the same temperature for 1 hour, methanol was added to the reaction mixture, followed by concentration under reduced pressure. The obtained residue was purified by silica gel column chromatography (n-hexane/ethyl acetate = 60/40 to 30/70) and recrystallized from ethyl acetate/n-hexane to obtain the title compound (hereinafter referred to as the compound of Example 1) (0.0236 g, 0.0478 mmol, 88%) as a white crystal.
$^1$H-NMR (400MHz, $CDCl_3$) δ: 1.46-1.60(m, 2H), 1.72-1.82(m, 4H), 1.90-2.01(m, 1H), 2.21(s, 3H), 2.26-2.29(m, 1H), 2.80(t, J=6.6Hz, 2H), 2.90-2.93(m, 2H), 3.15-3.18(m, 1H), 3.75-3.78(m, 1H), 4.23(s, 2H), 5.25-5.26(m, 1H), 7.18(d, J=2.3Hz, 1H), 7.44(d, J=2.3Hz, 1H), 7.61(d, J=8.2Hz, 2H), 7.68(d, J=8.2Hz, 2H), 8.27(s, 1H).
ESI-MS:m/z = 494(M+H)$^+$.

(Reference Example 4) Synthesis of tert-butyl (1-(4-(trifluoromethyl)benzyl)-1,2,3,4-tetrahydroquinolin-6-yl)carb amate:

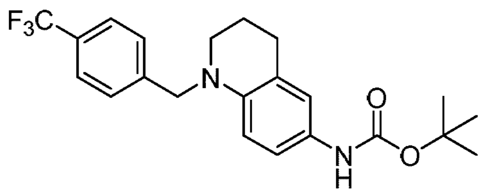

tert-Butyl (1,2,3,4-tetrahydroquinolin-6-yl)carbamate (0.150 g, 0.604 mmol) was dissolved in DMF (3.0 mL), and potassium carbonate (0.125 g, 0.906 mmol) and 1-(bromomethyl)-4-(trifluoromethyl)benzene (0.159 g, 0.664 mmol) were added at room temperature. After stirring the reaction mixture at the same temperature for 20 hours, distilled water was added to the reaction mixture and the aqueous layer was extracted with ethyl acetate. The organic layer was washed with brine, dried over anhydrous sodium sulfate and filtered, and then the filtrate was concentrated under reduced pressure. The residue was purified by silica gel column chromatography (n-hexane/ethyl acetate = 90/10 to 75/25) to obtain the title compound (hereinafter referred to as the compound of Reference Example 4) (0.246 g, 0.604 mmol, quantitative) as a white solid.
$^1$H-NMR (400MHz, $CDCl_3$) δ: 1.49(s, 9H), 1.98-2.04(m, 2H), 2.81(t, J=6.4Hz, 2H), 3.33(t, J=5.7Hz, 2H), 4.48(s, 2H), 6.17(brs, 1H), 6.33(d, J=9.1Hz, 1H), 6.82(dd, J=8.7, 2.7Hz, 1H), 7.11(brs, 1H), 7.36(d, J=7.8Hz, 2H), 7.56(d, J=8.2Hz, 2H).

ESI-MS:m/z = 407(M+H)$^+$.

(Reference Example 5) Synthesis of 1-(4-(trifluoromethyl)benzyl)-1,2,3,4-tetrahydroquinoline-6-amine:

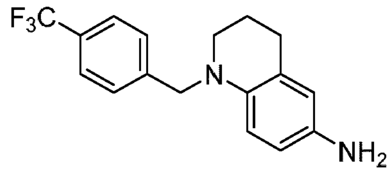

The compound of Reference Example 4 (0.240 g, 0.590 mmol) was dissolved in dichloromethane (3.0 mL) and trifluoroacetic acid (0.227 mL, 2.95 mmol) was added at 0°C. After stirring the reaction mixture at room temperature for 4.5 hours, an aqueous saturated sodium hydrogen carbonate solution was added to the reaction mixture, and the aqueous layer was extracted with ethyl acetate. The organic layer was washed with brine, dried over anhydrous sodium sulfate and filtered, and then the filtrate was concentrated under reduced pressure to obtain the title compound (hereinafter referred to as the compound of Reference Example 5) (0.169 g, 0.552 mmol, 93%) as a reddish brown solid.
$^1$H-NMR (400MHz, $CDCl_3$) δ: 1.97-2.03(m, 2H), 2.76(t, J=6.4Hz, 2H), 3.26(t, J=5.7Hz, 2H), 4.42(s, 2H), 6.30(d, J=8.2Hz, 1H), 6.40(dd, J=8.7, 2.7Hz, 1H), 6.46(d, J=2.7Hz, 1H), 7.40(d, J=7.8Hz, 2H), 7.56(d, J=8.2Hz, 2H).
ESI-MS:m/z = 307(M+H)$^+$.

(Reference Example 6) Synthesis of tert-butyl (R)-2-((1-(4-(trifluoromethyl)benzyl)-1,2,3,4-tetrahydroquinolin-6-yl)carbamoyl)piperidine-1-carboxylate:

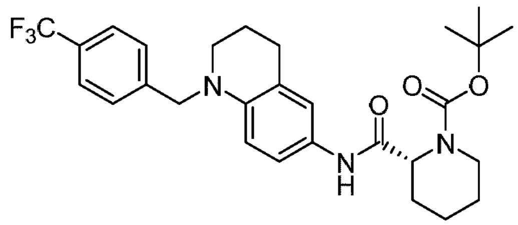

According to the same procedure as in Reference Example 3, except that the compound of Reference Example 5 was used in place of the compound of Reference Example 2, the title compound (hereinafter referred to as the compound of Reference Example 6) (0.0720 g, 0.139 mmol, 85%) was obtained as a colorless oil.
$^1$H-NMR (400MHz, $CDCl_3$) δ: 1.46-1.67(m,15H), 1.99-2.05(m, 2H), 2.31-2.38(m, 1H), 2.78-2.84(m, 3H), 3.35(t, J=5.7Hz, 2H), 4.05(brs, 1H), 4.50(s, 2H), 4.84(s, 1H), 6.34(d, J=8.6Hz, 1H), 6.95(dd, J=8.4, 2.0Hz, 1H), 7.27(s, 1H), 7.36(d, J=8.2Hz, 2H), 7.56(d, J=7.7Hz, 2H).
ESI-MS:m/z = 518(M+H)$^+$.

(Example 2) Synthesis of (R)-1-acetyl-N-(1-(4-(trifluoromethyl)benzyl)-1,2,3,4-tetrahydroqui nolin-6-yl)piperidine-2-carboxamide:

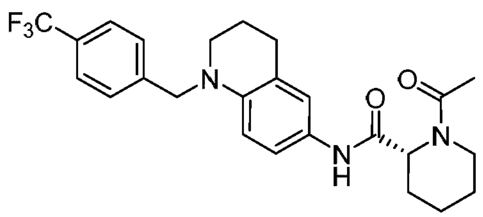

According to the same procedure as in Example 1, except that the compound of Reference Example 6 was used in place of the compound of Reference Example 3, the title compound (hereinafter referred to as the compound of Example 2) (0.0263 g, 0.0572 mmol, 43%) was obtained as a white solid.
$^1$H-NMR (400MHz, $CDCl_3$) δ: 1.44-1.58(m, 2H), 1.68-1.75(m, 2H), 1.89-2.05(m, 3H), 2.18(s, 3H), 2.27(d, J=13.7Hz, 1H), 2.80(t, J=6.4Hz, 2H), 3.16(td, J=13.3, 2.7Hz, 1H), 3,34(t, J=5.7Hz, 2H), 3.73(d, J=12.8Hz, 1H), 4.50(d, J=8.7Hz, 2H), 5.26(d, J=5.5Hz, 1H), 6.33(d, J=8.7Hz, 1H), 7.00(dd, J=8.7, 2.7Hz, 1H), 7.22(d, J=2.7Hz, 1H), 7.35(d, J=7.8Hz, 2H), 7.55(d, J=8.2Hz, 2H), 7.94(s, 1H). ESI-MS:m/z = 460(M+H)$^+$.

(Reference Example 7) Synthesis of methyl 4-bromo-2-chloro-6-methylbenzoate:

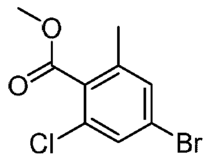

4-Bromo-2-methylbenzoic acid (2.02 g, 9.40 mmol), palladium(0) acetate (0.211 g, 0.940 mmol) and NCS (1.50 g, 11.2 mmol) were dissolved in DMF (20 mL), followed by stirring at 110°C for 3 hours. To the reaction mixture, an aqueous 1 M sodium hydroxide solution was added and the aqueous layer was washed with ethyl acetate. To the aqueous layer, 1 M hydrochloric acid was added, followed by extraction with ethyl acetate. The organic layer was washed with brine, dried over anhydrous sodium sulfate and filtered, and then the filtrate was concentrated under reduced pressure. The obtained crude product was used for the subsequent reaction without further purification.

The above crude product was dissolved in a mixed solution of diethyl ether (8.0 mL) and methanol (4.0 mL), and a trimethylsilyldiazomethane-hexane solution (2.0 M, 2.33 mL, 4.67 mmol) was added at room temperature. After stirring the reaction mixture at the same temperature for 0.5 hour, acetic acid was added to the reaction mixture, followed by concentration under reduced pressure. The obtained residue was purified by silica gel column chromatography (n-hexane/chloroform = 85/15 to 70/30) to obtain the title compound (hereinafter referred to as the compound of Reference Example 7) (1.02 g, 3.88 mmol, 41%) as a colorless oil. $^1$H-NMR (400MHz, $CDCl_3$) δ: 2.31(s, 3H), 3.94(s, 3H), 7.29(d, J=1.8Hz, 1H), 7.42(d, J=1.8Hz, 1H).

(Reference Example 8) Synthesis of methyl 4-bromo-2-(bromomethyl)-6-chlorobenzoate:

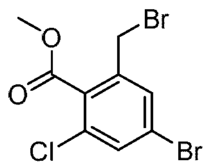

The compound of Reference Example 7 (1.18 g, 4.48 mmol) was dissolved in carbon tetrachloride (22 mL), and NBS (0.956 g, 5.37 mmol) and benzoyl peroxide (0.0541 g, 0.224 mmol) were added at room temperature. After stirring the reaction mixture at 80°C for 19 hours, the reaction mixture was filtered and the filtrate was concentrated under reduced pressure. The obtained residue was purified by silica gel column chromatography (n-hexane/chloroform=90/10 to 75/25) to obtain the title compound (hereinafter referred to as the compound of Reference Example 8) (0.865 g, 2.53 mmol, 56%) as a white solid.
$^1$H-NMR (400MHz, $CDCl_3$) δ: 3.99(s, 3H), 4.44(s, 2H), 7.49(d, J=1.8Hz, 1H), 7.55(d, J=1.8Hz, 1H).

(Reference Example 9) Synthesis of methyl 4-bromo-2-chloro-6-(cyanomethyl)benzoate:

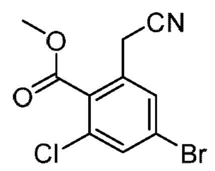

The compound of Reference Example 8 (0.100 g, 0.292 mmol) was dissolved in a mixed solution of ethanol (1.5 mL) and distilled water (0.150 mL), and sodium cyanide (0.0157 g, 0.321 mmol) was added at 0°C. After stirring the reaction mixture at room temperature for 24 hours, distilled water was added to the reaction mixture, and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 95/5 to 85/15) to obtain the title compound (hereinafter referred to as the compound of Reference Example 9) (0.0507 g, 0.176 mmol, 60%) as a white solid.
$^1$H-NMR (400MHz, $CDCl_3$) $\delta$: 3.79(s, 2H), 3.99(s, 3H), 7.59(d, J=1.8Hz, 1H), 7.61(d, J=1.8Hz, 1H).

(Reference Example 10) Synthesis of 6-bromo-8-chloro-3,4-dihydroisoquinolin-1(2H)-one:

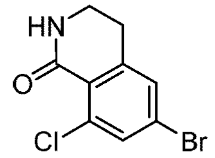

The compound of Reference Example 9 (0.0500 g, 0.173 mmol) was dissolved in a mixed solution of methanol (1.0 mL) and THF (1.0 mL), and cobalt(II) chloride hexahydrate (0.0825 g, 0.347 mmol) and sodium borohydride (0.0393 g, 1.04 mmol) were added at 0°C. After stirring the reaction mixture at the same temperature for 0.5 hour, an aqueous potassium sodium tartrate solution was added to the reaction mixture at 0°C. After stirring at room temperature for 1 hour, the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (ethyl acetate/methanol=99/1 to 97/3) to obtain the title compound (hereinafter referred to as the compound of Reference Example 10) (0.0250 g, 0.0960 mmol, 55%) as a white solid.
$^1$H-NMR (400MHz, $CDCl_3$) $\delta$: 2.95-2.98(m, 2H), 3.47-3.51(m, 2H), 6.09(brs, 1H), 7.31(d, J=1.8Hz, 1H), 7.56(d, J=1.8Hz, 1H).
ESI-MS:m/z = 261$(M+H)^+$.

(Reference Example 11) Synthesis of 6-bromo-8-chloro-1,2,3,4-tetrahydroisoquinoline:

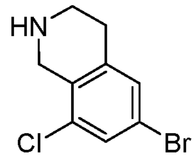

The compound of Reference Example 10 (0.0580 g, 0.223 mmol) was dissolved in THF (1.0 mL), and a solution of borane-THF complex in THF (0.95 M, 0.469 mL, 0.223 mmol) was added at 0°C. After stirring the reaction mixture at 60°C for 8 hours, methanol and 1 M hydrochloric acid were added to the reaction mixture at room temperature. After stirring the reaction mixture at 60°C for 3 hours, an aqueous 1 M sodium hydroxide solution was added to the reaction mixture and the aqueous layer was extracted with ethyl acetate. The organic layer was washed with brine, dried over anhydrous sodium sulfate and filtered, and then the filtrate was concentrated under reduced pressure to obtain the title compound (hereinafter referred to as the compound of Reference Example 11) (0.0325 g, 0.132 mmol, 59%) as a colorless oil.
$^1$H-NMR (400MHz, $CDCl_3$) $\delta$: 2.77(t, J=5.9Hz, 2H), 3.08(t, J=5.9Hz, 2H), 3.95(s, 2H), 7.17(brs, 1H), 7.34(d, J=1.8Hz, 1H).

ESI-MS:m/z = 248(M+H)+.

(Reference Example 12) Synthesis of 6-bromo-8-chloro-2-(4-(trifluoromethyl)benzyl)-1,2,3,4-tetrahydroi soquinoline:

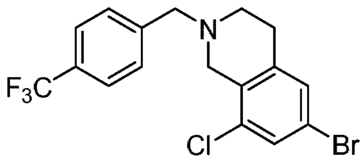

The compound of Reference Example 11 (0.0300 g, 0.122 mmol) was dissolved in DMF (1.0 mL), and potassium carbonate (0.0219 g, 0.146 mmol) and 1-(bromomethyl)-4-(trifluoromethyl)benzene (0.0349 g, 0.146 mmol) were added at room temperature. After stirring the reaction mixture at the same temperature for 4 hours, distilled water was added to the reaction mixture and the aqueous layer was extracted with diethyl ether. The organic layer was washed with brine, dried over anhydrous sodium sulfate and filtered, and then the filtrate was concentrated under reduced pressure. The obtained residue was purified by silica gel column chromatography (n-hexane/ethyl acetate = 95/5 to 85/15) to obtain the title compound (hereinafter referred to as the compound of Reference Example 12) (0.0397 g, 0.0981 mmol, 81%) as a colorless oil.
$^1$H-NMR (400MHz, $CDCl_3$) δ: 2.67(t, J=5.7Hz, 2H), 2.87(t, J=5.7Hz, 2H), 3.62(s, 2H), 3.78(s, 2H), 7.19(d, J=1.8Hz, 1H), 7.34(d, J=1.8Hz, 1H), 7.50(d, J=8.2Hz, 2H), 7.60(d, J=8.2Hz, 2H). ESI-MS:m/z = 248(M+H)+.

(Reference Example 13) Synthesis of tert-butyl (R)-2-carbamoylpiperidine-1-carboxylate:

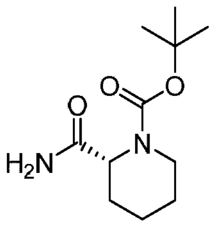

(R)-1-(tert-Butoxycarbonyl)piperidine-2-carboxylic acid (0.500 g, 2.18 mmol) and ammonium chloride (0.700 g, 13.1 mmol) were suspended in DMF (10 mL), and HATU (1.24 g, 3.27 mmol) and diisopropylethylamine (1.14 mL, 6.54 mmol) were added at 0°C. After stirring the reaction mixture at room temperature for 15 hours, distilled water was added to the reaction mixture and the aqueous layer was extracted with diethyl ether. The organic layer was washed with an aqueous saturated sodium hydrogen carbonate solution and brine, dried over anhydrous sodium sulfate and filtered, and then the filtrate was concentrated under reduced pressure. The obtained residue was purified by silica gel column chromatography (n-hexane/ethyl acetate = 60/40 to 30/70) to obtain the title compound (hereinafter referred to as the compound of Reference Example 13) (0.467 g, 2.05 mmol, 94%) as a white solid.
$^1$H-NMR (400MHz, $CDCl_3$) δ: 1.42-1.68(m, 5H), 1.48(s, 9H), 2.28-2.30(m, 1H), 2.79-2.85(m, 1H), 4.05(brs, 1H), 4.78(brs, 1H), 5.44(brs, 1H), 6.03(brs, 1H).
ESI-MS:m/z = 251(M+Na)+.

(Reference Example 14) Synthesis of tert-butyl (R)-2-((8-chloro-2-(4-(trifluoromethyl)benzyl)-1,2,3,4-tetrahydrois oquinolin-6-yl)carbamoyl)piperidine-1-carboxylate:

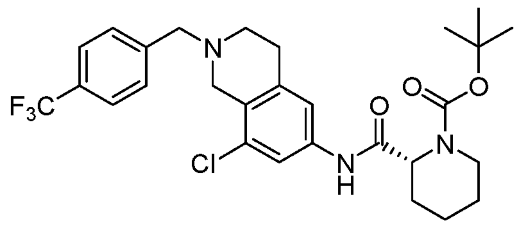

The compound of Reference Example 12 (0.0390 g, 0.0964 mmol), the compound of Reference Example 13 (0.0264 g, 0.116 mmol), tris(dibenzylidineacetone)dipalladium(0) (0.00177 g, 0.00193 mmol), 4,5-bis(diphenylphosphino)-9,9-dimethylxanthene (0.00335 g, 0.00578 mmol) and cesium carbonate (0.0440 g, 0.135 mmol) were suspeded in 1,4-dioxane (1.0 mL), followed by stirring at 90°C for 24 hours. The reaction mixture was filtered and the filtrate was concentrated under reduced pressure. The obtained residue was purified by silica gel column chromatography (n-hexane/ethyl acetate = 90/10 to 75/25) to obtain the title compound (hereinafter referred to as the compound of Reference Example 14 (0.0304 g, 0.0551 mmol, 57%) as a white amorphous.
$^1$H-NMR (400MHz, $CDCl_3$) δ: 1.42-1.69(m, 5H), 1.51(s, 9H), 2.30-2.33(m, 1H), 2.66(t, J=5.9Hz, 2H), 2.78-2.87(m, 3H), 3.64(s, 2H), 3.77(s, 2H), 4.05(brs, 1H), 4.83-4.84(m, 1H), 7.22(d, J = 1.8Hz, 1H), 7.40(d, J=1.8Hz, 1H), 7.51(d, J=8.2Hz, 2H), 7.60(d, J=8.2Hz, 2H).
ESI-MS:m/z = 552(M+H)$^+$.

(Example 3) Synthesis of (R)-1-acetyl-N-(8-chloro-2-(4-(trifluoromethyl)benzyl)-1,2,3,4-tetr ahydroisoquinolin-6-yl)piperidine-2-carboxamide:

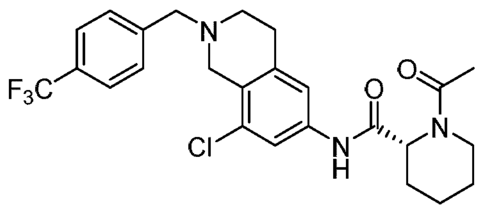

According to the same procedure as in Example 1, except that the compound of Reference Example 14 was used in place of the compound of Reference Example 3, the title compound (hereinafter referred to as the compound of Example 3) (0.0115 g, 0.0233 mmol, 78%) was obtained as a white amorphous.
$^1$H-NMR (400MHz, $CDCl_3$) δ: 1.45-1.60(m, 2H), 1.71-1.77(m, 2H), 1.89-2.01(m, 1H), 2.21(s, 3H), 2.25-2.28(m, 1H), 2.65(t, J=5.7Hz, 2H), 2.84(t, J=5.7Hz, 2H), 3.12-3.19(m, 1H), 3.64(s, 2H), 3.74-3.77(m, 1H), 3.77(s, 2H), 5.24-5.25(m, 1H), 7.19(d, J=1.8Hz, 1H), 7.44(d, J=1.8Hz, 1H), 7.50(d, J=8.2Hz, 2H), 7.60(d, J=8.2Hz, 2H), 8.39(s, 1H).
ESI-MS:m/z = 494(M+H)$^+$.

(Reference Example 15) Synthesis of 7-chloro-5-nitro-1-(4-(trifluoromethyl)benzyl)indol ine:

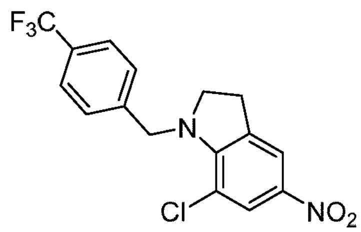

5-Nitroindoline (0.100 g, 0.609 mmol) was dissolved in DMF (3.0 mL), and potassium carbonate (0.126 g, 0.914 mmol) and 1-(bromomethyl)-4-(trifluoromethyl)benzene (0.160 g, 0.670 mmol) were added at room temperature. After stirring the reaction mixture at the same temperature for 16 hours, distilled water was added to the reaction mixture and

the aqueous layer was extracted with diethyl ether. The organic layer was washed with brine, dried over anhydrous sodium sulfate and filtered, and then the filtrate was concentrated under reduced pressure. The obtained crude product was used for the subsequent reaction without further purification.

The above crude product was dissolved in DMF (3.0 mL), and NCS (0.0975 g, 0.730 mmol) was added at room temperature. After stirring the reaction mixture at the same temperature for 2 hours, an aqueous sodium thiosulfate solution was added to the reaction mixture and the aqueous layer was extracted with diethyl ether. The organic layer was washed with brine, dried over anhydrous sodium sulfate and filtered, and then the filtrate was concentrated under reduced pressure. The obtained residue was purified by silica gel column chromatography (n-hexane/ethyl acetate = 95/5 to 80/20) to obtain the title compound (hereinafter referred to as the compound of Reference Example 15) (0.109 g, 0.306 mmol, 50%) as a reddish brown solid.

$^1$H-NMR (400MHz, $CDCl_3$) δ: 3.11-3.15(m, 2H), 3.64-3.68(m, 2H), 5.05(s, 2H), 7.41(d, J=8.2Hz, 2H), 7.63(d, J=8.2Hz, 2H), 7.81-7.82(m, 1H), 8.05(d, J=2.3Hz, 1H).

ESI-MS:m/z = 357(M+H)$^+$.

(Reference Example 16) Synthesis of 7-chloro-1-(4-(trifluoromethyl)benzyl)indoline-5-amine:

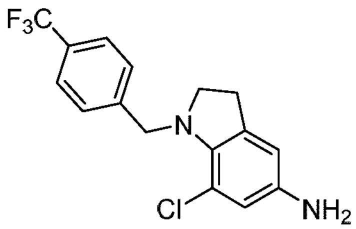

The compound of Reference Example 15 (0.105 g, 0.294 mmol) was dissolved in a mixed solution of THF (1.0 mL), ethanol (1.0 mL) and distilled water (1.0 mL), and acetic acid (0.0843 g, 1.47 mmol) and iron powder (0.0822 g, 1.47 mmol) were added at room temperature. After stirring the reaction mixture at 50°C for 1 hour, an aqueous saturated sodium hydrogen carbonate solution was added to the reaction mixture, followed by filtration. To the filtrate, distilled water was added and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 85/15 to 70/30) to obtain the title compound (hereinafter referred to as the compound of Reference Example 16) (0.0697 g, 0.213 mmol, 73%) as a brown oil.

$^1$H-NMR (400MHz, $CDCl_3$) δ: 2.88(t, J=8.6Hz, 2H), 3.29(t, J=8.6Hz, 2H), 3.88(brs, 2H), 4.61(s, 2H), 6.45-6.45(m, 1H), 6.48(d, J=1.8Hz, 1H), 7.49(d, J=8.2Hz, 2H), 7.58(d, J=8.2Hz, 2H).

ESI-MS:m/z = 327(M+H)$^+$.

(Reference Example 17) Synthesis of tert-butyl (R)-2-((7-chloro-1-(4-(trifluoromethyl)benzyl)indolin-5-yl)carbamo yl)piperidine-1-carboxylate:

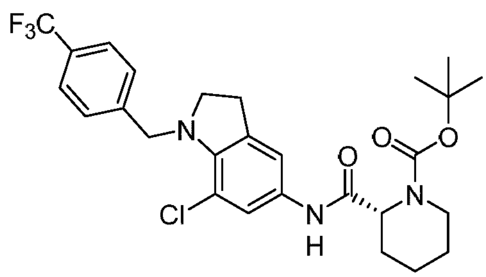

The compound of Reference Example 16 (0.0620 g, 0.190 mmol) and (R)-1-(tert-butoxycarbonyl)piperidine-2-carboxylic acid (0.0522 g, 0.228 mmol) were dissolved in DMF (1.0 mL), and HATU (0.0869 g, 0.228 mmol) and diisopropylethylamine (0.0497 mL, 0.285 mmol) were added at 0°C. After stirring the reaction mixture at room temperature

for 20 hours, distilled water was added to the reaction mixture and the aqueous layer was extracted with diethyl ether. The organic layer was washed with an aqueous saturated sodium hydrogen carbonate solution and brine, dried over anhydrous sodium sulfate and filtered, and then the filtrate was concentrated under reduced pressure. The obtained residue was purified by silica gel column chromatography (n-hexane/ethyl acetate = 95/5 to 85/15) to obtain the title compound (hereinafter referred to as the compound of Reference Example 17) (0.0831 g, 0.154 mmol, 81%) as a white amorphous.

$^1$H-NMR (400MHz, $CDCl_3$) $\delta$: 1.46-1.69(m, 5H), 1.52(s, 9H), 2.32-2.35(m, 1H), 2.79-2.86(m, 1H), 2.95-3.00(m, 2H), 3.35-3.40(m, 2H), 4.06(brs, 1H), 4.77(s, 2H), 4.84(brs, 1H), 7.19(d, J=1.8Hz, 1H), 7.22(d, J=1.8Hz, 1H), 7.46(d, J=8.2Hz, 2H), 7.58(d, J=8.2Hz, 2H).

ESI-MS:m/z = 538(M+H)$^+$.

(Example 4) Synthesis of (R)-1-acetyl-N-(7-chloro-1-(4-(trifluoromethyl)benzyl)indolin-5-yl) piperidine-2-carboxamide:

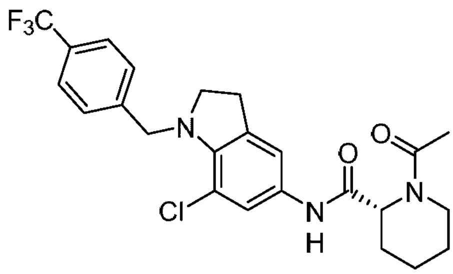

(Production method 1)

The compound of Reference Example 17 (0.0800 g, 0.149 mmol) was dissolved in dichloromethane (1.0 mL) and trifluoroacetic acid (0.250 mL, 3.24 mmol) was added at 0°C. After stirring the reaction mixture at room temperature for 2 hours, an aqueous saturated sodium hydrogen carbonate solution was added to the reaction mixture and the aqueous layer was extracted with ethyl acetate. The organic layer was washed with brine, dried over anhydrous sodium sulfate and filtered, and then the filtrate was concentrated under reduced pressure. The obtained crude product was used for the subsequent reaction without further purification.

The above crude product was dissolved in dichloromethane, and triethylamine (0.0286 mL, 0.206 mmol) and acetyl chloride (0.0117 mL, 0.164 mmol) were added at 0°C. After stirring the reaction mixture at the same temperature for 1 hour, methanol was added to the reaction mixture, followed by concentration under reduced pressure. The obtained residue was purified by silica gel column chromatography (n-hexane/ethyl acetate = 50/50 to 20/80) to obtain the title compound (hereinafter referred to as the compound of Example 4) (0.0568 g, 0.118 mmol, 86%) as a white amorphous.

(Production method 2)

The compound of Reference Example 17 (3.54 g, 6.58 mmol) was dissolved in ethyl acetate (10 mL) and a hydrogen chloride-ethyl acetate solution (4.0 M, 6.58 mL, 26.3 mmol) was added at 0°C. After stirring the reaction mixture at room temperature for 15 hours, an aqueous 2 M potassium carbonate solution was added to the reaction mixture and the aqueous layer was extracted with ethyl acetate. The organic layer was washed with brine, dried over anhydrous sodium sulfate and filtered, and then the filtrate was concentrated under reduced pressure. The obtained crude product was used for the subsequent reaction without further purification.

The above crude product was dissolved in dichloromethane (17 mL), and triethylamine (1.38 mL, 9.87 mmol) and acetic anhydride (0.745 mL, 7.89 mmol) were added at 0°C. After stirring the reaction mixture at room temperature for 20 hours, an aqueous saturated sodium hydrogen carbonate solution was added to the reaction mixture, and the aqueous layer was extracted with chloroform. The organic layer was washed with brine, dried over anhydrous sodium sulfate and filtered, and then the filtrate was concentrated under reduced pressure. The obtained residue was purified by silica gel column chromatography (n-hexane/ethyl acetate = 50/50 to 20/80). The obtained white amorphous of the compound of Example 4 was dissolved in a mixed solution of ethyl acetate (9.0 mL) and n-hexane (13.5 mL), and a seed crystal of the compound of Example 4 obtained by the following method was added at room temperature. After stirring at the same temperature for 30 minutes, n-hexane (9.0 mL) was added to this mixed solution. After stirring at the same temperature for 1 hour, the precipitated solid was collected by filtration to obtain the title compound (compound of Example 4) (2.42 g, 5.04 mmol, 77%) as a white crystal. As a result of analysis using a chiral column, the retention

time of the obtained compound of Example 4 was 36.8 minutes, and the optical purity at that time was 99.9%ee. The analysis conditions using the chiral column are as follows.
Measurement equipment; High-performance liquid chromatograph LC-2010CHT, manufactured by Shimadzu Corporation
Column; CHIRALCEL OZ-3R 0.46 cmφ × 15 cm, particle size of 3 μm, manufactured by Daicel Chemical Industries Ltd.
Column temperature; 40°C
Mobile phase; (Solution A) aqueous 20 mM potassium dihydrogen phosphate solution, (Solution B) acetonitrile
Composition of mobile phase; Solution A:Solution B = 55:45 (liquid was fed for 50 minutes)
Flow rate; 1.0 mL/minute
Detection; UV (210 nm)
$^1$H-NMR (400MHz, $CDCl_3$) δ: 1.46-1.59(m, 2H), 1.70-1.77(m, 2H), 1.89-2.01(m, 1H), 2.20(s, 3H), 2.26-2.29(m, 1H), 2.96(t, J=8.8Hz, 2H), 3.13-3.21(m, 1H), 3.34-3.39(m, 2H), 3.74-3.78(m, 1H), 4.75(s, 2H), 5.24-5.26(m, 1H), 7.20(brs, 1H), 7.22(brs, 1H), 7.46(d, J=8.2Hz, 2H), 7.58(d, J=8.2Hz, 2H), 8.17(s, 1H).
ESI-MS:m/z = 480$(M+H)^+$.

The seed crystal of the compound of Example 4 was obtained by the following method.

The white amorphous (8 mg) of the compound of Example 4 was suspended in ethyl acetate/n-heptane = 1:4 (v/v, 40 μl) and shaken at room temperature for 7 days, thereby crystallizing the compound of Example 4. After removing the solvent and air-drying, a seed crystal of the compound of Example 4 was obtained.

(Reference Example 18) Synthesis of 7-chloro-5-nitro-1-(3-(trifluoromethyl)benzyl)indoline:

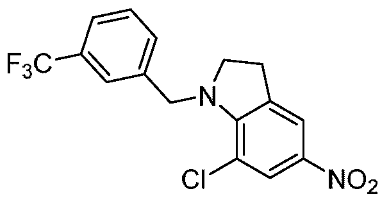

5-Nitroindoline (0.100 g, 0.609 mmol) and 3-(trifluoromethyl)benzaldehyde (0.0800 mL, 0.0609 mmol) was dissolved in dichloromethane (3.0 mL), and acetic acid (0.0174 mL, 0.305 mmol) and sodium triacetoxyborohydride (0.194 g, 0.914 mmol) were added at room temperature. After stirring the reaction mixture at the same temperature for 16 hours, an aqueous saturated sodium hydrogen carbonate solution was added to the reaction mixture and the aqueous layer was extracted with ethyl acetate. The organic layer was washed with brine, dried over anhydrous sodium sulfate and filtered, and then the filtrate was concentrated under reduced pressure. The obtained crude product was used for the subsequent reaction without further purification.

The above crude product was dissolved in DMF (3.0 mL) and NCS (0.0975 g, 0.730 mmol) was added at room temperature. After stirring at the same temperature for 2 hours, an aqueous sodium thiosulfate solution was added to the reaction mixture and the aqueous layer was extracted with diethyl ether. The organic layer was washed with brine, dried over anhydrous sodium sulfate and filtered, and then the filtrate was concentrated under reduced pressure. The obtained residue was purified by silica gel column chromatography (n-hexane/ethyl acetate = 95/5 to 80/20) to obtain the title compound (hereinafter referred to as the compound of Reference Example 18) (0.158 g, 0.443 mmol, 73%) as a dark brown solid.
$^1$H-NMR (400MHz, $CDCl_3$) δ: 3.12(t, J=8.9Hz, 2H), 3.65(t, J=8.9Hz, 2H), 5.04(s, 2H), 7.49-7.50(m, 2H), 7.55-7.59(m, 2H), 7.82(d, J=2.1Hz, 1H), 8.06(d, J=2.1Hz, 1H).
ESI-MS:m/z = 357$(M+H)^+$.

(Reference Example 19) Synthesis of 7-chloro-1-(3-(trifluoromethyl)benzyl)indoline-5-amine:

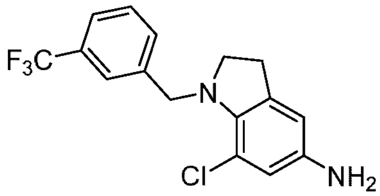

According to the same procedure as in Reference Example 16, except that the compound of Reference Example 18 was used in place of the compound of Reference Example 15, the title compound (hereinafter referred to as the compound of Reference Example 19 (0.130 g, 0.398 mmol, 93%) was obtained as a dark brown oil.
$^1$H-NMR (400MHz, $CDCl_3$) δ: 2.88(t, J=8.5Hz, 2H), 3.28(t, J=8.5Hz, 2H), 3.41(s, 2H), 4.59(s, 2H), 6.45(d, J=2.3Hz, 1H), 6.48(d, J=2.3Hz, 1H), 7.42-7.45(m, 1H), 7.52(d, J=7.8Hz, 1H), 7.58(d, J=7.8Hz, 1H), 7.64(s, 1H).
ESI-MS:m/z = 327(M+H)$^+$.

(Reference Example 20) Synthesis of tert-butyl (R)-2-((7-chloro-1-(3-(trifluoromethyl)benzyl)indolin-5-yl)carbamo yl)piperidine-1-carboxylate:

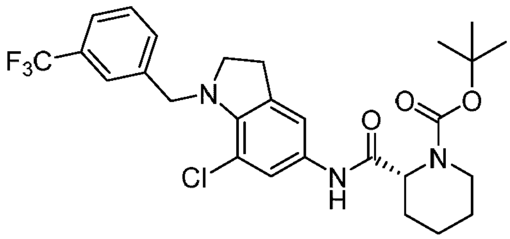

According to the same procedure as in Reference Example 17, except that the compound of Reference Example 19 was used in place of the compound of Reference Example 16, the title compound (hereinafter referred to as the compound of Reference Example 20) (0.181 g, 0.336 mmol, 87%) was obtained as a pale yellow solid.
$^1$H-NMR (400MHz, $CDCl_3$) δ: 1.42-1.69(m, 5H), 1.52(s, 9H), 2.32-2.35(m, 1H), 2.79-2.86(m, 1H), 2.98(t, J=8.5Hz, 2H), 3.36(t, J=8.5Hz, 2H), 4.07(brs, 1H), 4.75(s, 2H), 4.84-4.85(m, 1H), 7.19(d, J=1.8Hz, 1H), 7.22(d, J=1.8Hz, 1H), 7.43-7.46(m, 1H), 7.52-7.57(m, 2H), 7.61(s, 1H).
ESI-MS:m/z = 538(M+H)$^+$.

(Example 5) Synthesis of (R)-1-acetyl-N-(7-chloro-1-(3-(trifluoromethyl) benzyl)indolin-5-yl) piperidine-2-carboxamide:

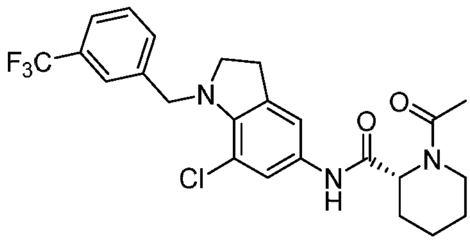

According to the same procedure as in Example 4, except that the compound of Reference Example 20 was used in place of the compound of Reference Example 17, the title compound (hereinafter referred to as the compound of Example 5) (0.0807 g, 0.168 mmol, 90%) was obtained as a white solid.
$^1$H-NMR (400MHz, $CDCl_3$) δ: 1.46-1.57(m, 2H), 1.70-1.77(m, 2H), 1.89-2.00(m, 1H), 2.20(s, 3H),2.26-2.29(m, 1H), 2.96(t, J=8.7Hz, 2H), 3.18(td, J=13.3, 2.7Hz, 1H), 3.35(t, J=8.7Hz, 2H), 3.74-3.77(m, 1H), 4.74(s, 2H), 5.24-5.26(m, 1H), 7.20(d, J=2.1Hz, 1H), 7.22(d, J=2.1Hz, 1H), 7.42-7.46(m, 1H), 7.51-7.56(m, 2H), 7.61(s, 1H), 8.18(s, 1H).
ESI-MS:m/z = 480(M+H)$^+$.

(Reference Example 21) Synthesis of 7-chloro-5-nitro-1-(2-(trifluoromethyl)benzyl)indoline:

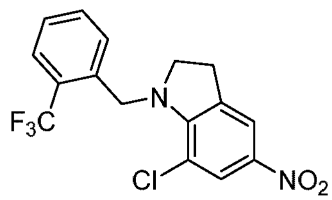

According to the same procedure as in Reference Example 18, except that 2-(trifluoromethyl)benzaldehyde was used in place of 3-(trifluoromethyl)benzaldehyde, the title compound (hereinafter referred to as the compound of Reference Example 21) (0.140 g, 0.392 mmol, 65%) was obtained as a dark brown solid.
$^1$H-NMR (400MHz, $CDCl_3$) δ: 3.17(t, J=9.1Hz, 2H), 3.72(t, J=9.1Hz, 2H), 5.19(s, 2H), 7.39-7.47(m, 2H), 7.53-7.57(m, 1H), 7.70(d, J=7.8Hz, 1H), 7.82-7.83(m, 1H), 8.03(d, J=2.3Hz, 1H).
ESI-MS:m/z = 357$(M+H)^+$.

(Reference Example 22) Synthesis of 7-chloro-1-(2-(trifluoromethyl)benzyl)indoline-5-amine:

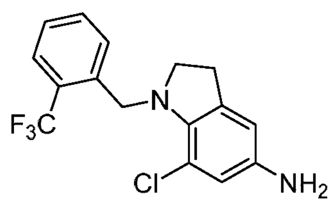

According to the same procedure as in Reference Example 16, except that the compound of Reference Example 21 was used in place of the compound of Reference Example 15, the title compound (hereinafter referred to as the compound of Reference Example 22) (0.139 g, 0.537 mmol, 52%) was obtained as a dark brown oil.
$^1$H-NMR (400MHz, $CDCl_3$) δ: 2.97(t, J=8.5Hz, 2H), 3.34(t, J=8.5Hz, 2H), 3.39(s, 2H), 4.75(s, 2H), 6.46-6.47(m, 2H), 7.34(dd, J=7.8, 7.5Hz, 1H), 7.53(dd, J=7.8, 7.5Hz, 1H), 7.64(d, J=7.8Hz, 1H), 7.90(d, J=7.8Hz, 1H).
ESI-MS:m/z = 327$(M+H)^+$.

(Reference Example 23) Synthesis of tert-butyl (R)-2-((7-chloro-1-(2-(trifluoromethyl)benzyl)indolin-5-yl)carbamo yl)piperidine-1-carboxylate:

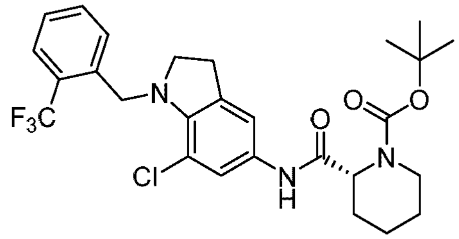

According to the same procedure as in Reference Example 17, except that the compound of Reference Example 22 was used in place of the compound of Reference Example 16, the title compound (hereinafter referred to as the compound of Reference Example 23) (0.164 g, 0.305 mmol, 87%) was obtained as a pale yellow solid.
$^1$H-NMR (400MHz, $CDCl_3$) δ: 1.42-1.68(m, 5H), 1.51(s, 9H), 2.32-2.35(m, 1H), 2.80-2.86(m, 1H), 3.04(t, J=8.9Hz, 2H), 3.45(t, J=8.9Hz, 2H), 4.07(brs, 1H), 4.84(brs, 1H), 4.92(s, 2H), 7.13(d, J=2.1Hz, 1H), 7.24(d, J=2.1Hz, 1H), 7.35(dd, J=7.8, 7.5Hz, 1H), 7.52(dd, J=7.8, 7.5Hz, 1H), 7.65(d, J=7.8Hz, 1H), 7.74(d, J=7.8Hz, 1H).

ESI-MS:m/z = 538(M+H)+.

(Example 6) Synthesis of (R)-1-acetyl-N-(7-chloro-1-(2-(trifluoromethyl)benzyl)indolin-5-yl) piperidine-2-carboxamide:

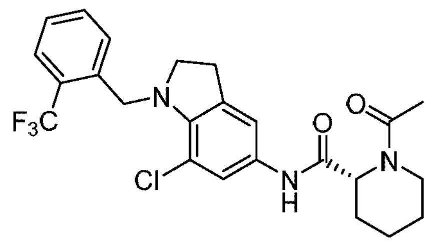

According to the same procedure as in Example 4, except that the compound of Reference Example 23 was used in place of the compound of Reference Example 17, the title compound (hereinafter referred to as the compound of Example 6) (0.0815 g, 0.170 mmol, 91%) was obtained as a white solid.
$^1$H-NMR (400MHz, $CDCl_3$) δ: 1.46-1.58(m, 2H), 1.70-1.77(m, 2H), 1.89-1.99(m, 1H), 2.20(s, 3H), 2.26-2.29(m, 1H), 3.02(t, J=8.7Hz, 2H), 3.15-3.22(m, 1H), 3.43(t, J=8.7Hz, 2H), 3.74-3.77(m, 1H), 4.91(s, 2H), 5.25-5.26(m, 1H), 7.17(d, J=2.1Hz, 1H), 7.22(d, J=2.1Hz, 1H), 7.34(t, J=7.5Hz, 1H), 7.52(t, J=7.5Hz, 1H), 7.65(d, J=7.8Hz, 1H), 7.73(d, J=7.8Hz, 1H), 8.16(s, 1H).
ESI-MS:m/z = 480(M+H)+.

(Reference Example 24) Synthesis of 1-(5-nitroindolin-1-yl)-2-(4-(trifluoromethyl)phenyl)ethan-1-one:

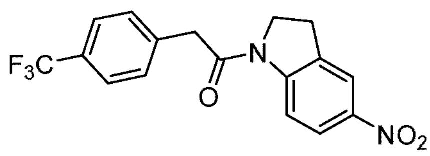

5-Nitroindoline (0.200 g, 1.22 mmol) and 2-(4-(trifluoromethyl)phenyl)acetic acid (0.249 g, 1.22 mmol) were dissolved in DMF (3.0 mL), and HATU (0.510 g, 1.34 mmol) and diisopropylethylamine (0.319 mL, 1.83 mmol) were added at 0°C. After stirring the reaction mixture at room temperature for 24 hours, distilled water was added to the reaction mixture, the aqueous layer was extracted with ethyl acetate. The organic layer was washed with brine, dried over anhydrous sodium sulfate and filtered, and then the filtrate was concentrated under reduced pressure. The obtained residue was purified by aminosilica gel column chromatography (chloroform) and reprecipitated from ethyl acetate/diethyl ether to obtain the title compound (hereinafter referred to as the compound of Reference Example 24) (0.282 g, 0.805 mmol, 66%) as a yellow solid.
$^1$H-NMR (400MHz, $CDCl_3$) δ: 3.31(t, J=8.5Hz, 2H), 3.91(s, 2H), 4.25(t, J=8.7Hz, 2H), 7.43(d, J=8.2Hz, 2H), 7.64(d, J=8.2Hz, 2H), 8.06(d, J=2.3Hz, 1H), 8.14(dd, J=8.9, 2.5Hz, 1H), 8.33(d, J=8.7Hz, 1H).
ESI-MS:m/z = 349(M-H)-.

(Reference Example 25) Synthesis of 5-nitro-1-(4-(trifluoromethyl)phenethyl)indoline:

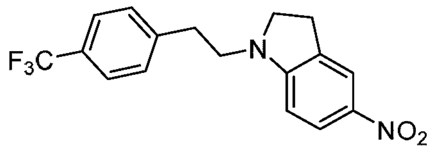

The compound of Reference Example 24 (0.280 g, 0.799 mmol) was dissolved in THF (2.0 mL) and a solution of borane-THF complex in THF (0.90 M, 1.78 mL, 1.60 mmol) was added at 0°C. After stirring the reaction mixture at

room temperature for 2.5 hours, distilled water was added to the reaction mixture and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 80/20 to 67/33) to obtain the title compound (hereinafter referred to as the compound of Reference Example 25) (0.187 g, 0.556 mmol, 70%) as a colorless solid.
$^1$H-NMR (400MHz, $CDCl_3$) δ: 2.97(t, J=7.5Hz, 2H), 3.06(t, J=8.2Hz, 2H), 3.51(t, J=7.3Hz, 2H), 3.61(t, J=8.7Hz, 2H), 6.22(d, J=8.7Hz, 1H), 7.34(d, J=7.8Hz, 2H), 7.58(d, J=7.8Hz, 2H), 7.89(d, J=2.3Hz, 1H), 8.04(dd, J=9.1, 2.3Hz, 1H).
ESI-MS:m/z = 337(M+H)$^+$.

(Reference Example 26) Synthesis of 7-chloro-5-nitro-1-(4-(trifluoromethyl)phenethyl)indoline:

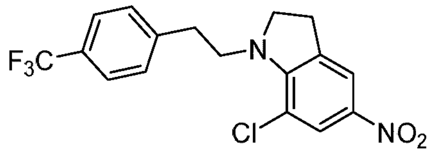

The compound of Reference Example 25 (0.186 g, 0.553 mmol) was dissolved in DMF (2.8 mL) and NCS (0.0890 g, 0.664 mmol) was added at room temperature. After stirring the reaction mixture at the same temperature for 3 hours, an aqueous sodium thiosulfate solution was added to the reaction mixture and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 75/25 to 60/40) to obtain the title compound (hereinafter referred to as the compound of Reference Example 26) (0.150 g, 0.405 mmol, 73%) as a yellow solid.
$^1$H-NMR (400MHz, $CDCl_3$) δ: 3.03(t, J=7.8Hz, 2H), 3.06(t, J=9.6Hz, 2H), 3.66(t, J=9.1Hz, 2H), 3.93(t, J=7.8Hz, 2H), 7.35(d, J=8.2Hz, 2H), 7.58(d, J=7.8Hz, 2H), 7.76-7.77(m, 1H), 8.04(dd, J=9.1, 2.3Hz, 1H).

(Reference Example 27) Synthesis of 7-chloro-1-(4-(trifluoromethyl)phenethyl)indoline-5-amine:

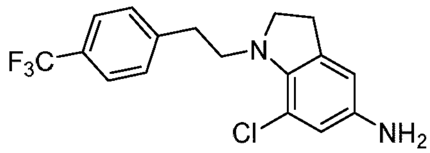

According to the same procedure as in Reference Example 16, except that the compound of Reference Example 26 was used in place of the compound of Reference Example 15, the title compound (hereinafter referred to as the compound of Reference Example 27) (0.119 g, 0.349 mmol, 86%) was obtained as a brown solid.
$^1$H-NMR (400MHz, $CDCl_3$) δ: 2.87-2.95(m, 4H), 3.38(brs, 2H), 3.43(t, J=8.5Hz, 2H), 3.60-3.64(m, 2H), 6.44-6.45(m, 2H), 7.35(d, J=7.8Hz, 2H), 7.54(d, J=8.2Hz, 2H).
ESI-MS:m/z = 341(M+H)$^+$.

(Reference Example 28) Synthesis of (R)-1-acetylpiperidine-2-carboxylic acid:

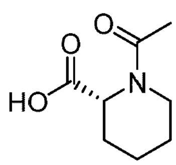

(R)-1-(tert-Butoxycarbonyl)piperidine-2-carboxylic acid (0.500 g, 2.18 mmol) was dissolved in DMF (2.2 mL),

and cesium carbonate (0.782 g, 2.40 mmol) and (bromomethyl)benzene (0.259 mL, 2.18 mmol) were added at 0°C. After stirring the reaction mixture at room temperature for 20 hours, distilled water was added to the reaction mixture and the aqueous layer was extracted with diethyl ether. The organic layer was washed with brine, dried over anhydrous sodium sulfate and filtered, and then the filtrate was concentrated under reduced pressure. The obtained crude product was used for the subsequent reaction without further purification.

The above crude product was dissolved in dichloromethane (9.6 mL) and trifluoroacetic acid (1.47 mL, 19.1 mmol) was added at 0°C. After stirring the reaction mixture at room temperature for 1.5 hours, an aqueous saturated sodium hydrogen carbonate solution was added to the reaction mixture and the aqueous layer was extracted with dichloromethane. The organic layer was washed with brine, dried over anhydrous sodium sulfate and filtered, and then the filtrate was concentrated under reduced pressure. The obtained crude product was used for the subsequent reaction without further purifictaion.

The above crude product was dissolved in dichloromethane (9.6 mL), and triethylamine (0.534 mL, 3.83 mmol) and acetic anhydride (0.199 mL, 2.11 mmol) were added at 0°C. After stirring the reaction mixture at room temperature for 4 hours, an aqueous saturated sodium hydrogen carbonate solution was added to the reaction mixture and the aqueous layer was extracted with dichloromethane. The organic layer was washed with brine, dried over anhydrous sodium sulfate and filtered, and then the filtrate was concentrated under reduced pressure. The obtained residue was purified by silica gel column chromatography (n-hexane/ethyl acetate = 50/50 to 25/75) and then used for the subsequent reaction.

The above product was dissolved in methanol (9.5 mL) and palladium-carbon (5% by weight, 0.0404 g) was added at room temperature. After stirring the reaction mixture at the same temperature for 3.5 hours under hydrogen atmosphere, the reaction mixture was filtered and the filtrate was concentrated under reduced pressure to obtain the title compound (hereinafter referred to as the compound of Reference Example 28) (0.318 g, 1.86 mmol, 85%) as a white solid.

$^1$H-NMR (400MHz, $CDCl_3$) δ: 1.40-1.55(m, 2H), 1.60-1.75(m, 3H), 2.10(s, 0.5H), 2.16(s, 2.5H), 2.27-2.35(m, 0.8H), 2.69(t, J = 13.0Hz, 0.2H), 3.28(td, J=12.9, 3.0Hz, 1H), 3.73(d, J=13.3Hz, 1H), 4.55(d, J=12.8Hz, 0.2H), 5.35(d, J=4.6Hz, 0.8H).

ESI-MS:m/z = 170(M-H)$^-$.

(Example 7) Synthesis of (R)-1-acetyl-N-(7-chloro-1-(4-(trifluoromethyl)phenethyl)indolin-5-yl)piperidine-2-carboxamide:

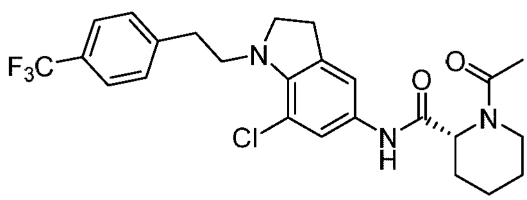

The compound of Reference Example 27 (0.0300 g, 0.0880 mmol) and the compound of Reference Example 28 (0.0181 g, 0.106 mmol) were dissolved in DMF (0.30 mL), and HATU (0.0402 g, 0.106 mmol) and diisopropylethylamine (0.0231 mL, 0.132 mmol) were added at 0°C. After stirring the reaction mixture at room temperature for 5 hours, distilled water was added to the reaction mixture and the aqueous layer was extracted with ethyl acetate. The organic layer was washed with an aqueous saturated sodium hydrogen carbonate solution and brine, dried over anhydrous sodium sulfate and filtered, and then the filtrate was concentrated under reduced pressure. The obtained residue was purified by silica gel column chromatography (n-hexane/ethyl acetate = 20/80 to 0/100) to obtain the title compound (hereinafter referred to as the compound of Example 7) (0.0199 g, 0.0403 mmol, 46%) as a white solid.

$^1$H-NMR (400MHz, $CDCl_3$) δ: 1.48-1.59(m, 2H), 1.74(t, J=13.7Hz, 2H), 1.90-1.98(m, 1H), 2.20(s, 3H), 2.27(d, J=13.3Hz, 1H), 2.90(t, J=7.8Hz, 2H), 2.96(t, J=8.7Hz, 2H), 3.16(td, J=13.3, 2.7Hz, 1H), 3.48(t, J=8.7Hz, 2H), 3.69-3.77(m, 3H), 5.25(d, J=5.0Hz, 1H), 7.17(d, J=2.3Hz, 1H), 7.20(d, J=2.3Hz, 1H), 7.34(d, J=8.2Hz, 2H), 7.54(d, J=7.8Hz, 2H), 8.13(brs, 1H).

ESI-MS:m/z = 494(M+H)$^+$.

(Reference Example 29) Synthesis of 7-chloro-5-nitro-1-(4-(trifluoromethoxy)benzyl)indoline:

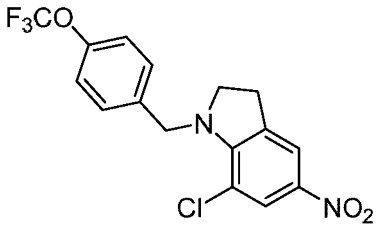

According to the same procedure as in Reference Example 15, except that 1-(bromomethyl)-4-(trifluoromethoxy)benzene was used in place of 1-(bromomethyl)-4-(trifluoromethyl)benzene, the title compound (hereinafter referred to as the compound of Reference Example 29) (0.124 g, 0.333 mmol, 55%) was obtained as a yellow solid.
$^1$H-NMR (400MHz, $CDCl_3$) δ: 3.08-3.13(m, 2H), 3.62-3.66(m, 2H), 4.99(s, 2H), 7.21(d, J=8.7Hz, 2H), 7.32(d, J=8.7Hz, 2H), 7.80-7.81(m, 1H), 8.05(d, J=2.3Hz, 1H).
ESI-MS:m/z = 373(M+H)+.

(Reference Example 30) Synthesis of 7-chloro-1-(4-(trifluoromethoxy)benzyl)indoline-5-amine:

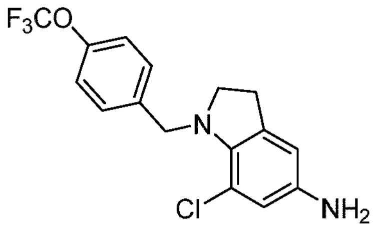

According to the same procedure as in Reference Example 16, except that the compound of Reference Example 29 was used in place of the compound of Reference Example 15, the title compound (hereinafter referred to as the compound of Reference Example 30) (0.105 g, 0.306 mmol, 95%) was obtained as a dark brown oil. $^1$H-NMR (400MHz, $CDCl_3$) δ: 2.86(t, J=8.5Hz, 2H), 3.27(t, J=8.5Hz, 2H), 3.41(brs, 2H), 4.55(s, 2H), 6.44-6.44(m, 1H), 6.47-6.48(m, 1H), 7.16(d, J=8.2Hz, 2H), 7.39(d, J=8.2Hz, 2H).
ESI-MS:m/z = 343$(M+H)^+$.

(Reference Example 31) Synthesis of tert-butyl (R)-2-((7-chloro-1-(4-(trifluoromethoxy)benzyl)indolin-5-yl)carbam oyl)piperidine-1-carboxylate:

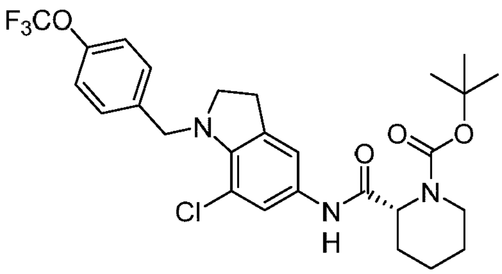

According to the same procedure as in Reference Example 3, except that the compound of Reference Example 30 was used in place of the compound of Reference Example 2, the title compound (hereinafter referred to as the compound of Reference Example 31) (0.136 g, 0.245 mmol, 84%) was obtained as a white solid.
$^1$H-NMR (400MHz, $CDCl_3$) δ: 1.44-1.47(m, 1H), 1.51(s, 9H), 1.55-1.68(m, 4H), 2.32-2.35(m, 1H), 2.79-2.86(m, 1H), 2.96(t, J=8.7Hz, 2H), 3.35(t, J=8.7Hz, 2H), 4.07(brs, 1H), 4.71(s, 2H), 4.84-4.85(m, 1H), 7.16-7.20(m, 4H), 7.37(d, J=8.7Hz, 2H).
ESI-MS:m/z = 554$(M+H)^+$.

(Example 8) Synthesis of (R)-1-acetyl-N-(7-chloro-1-(4-(trifluoromethoxy)benzyl)indolin-5-yl )piperidine-2-carboxamide:

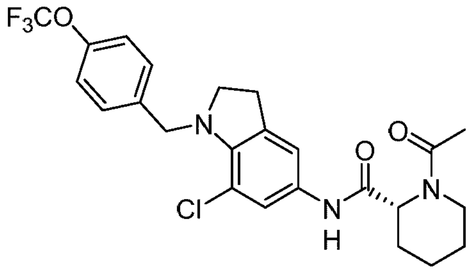

According to the same procedure as in Example 1, except that the compound of Reference Example 31 was used in place of the compound of Reference Example 3, the title compound (hereinafter referred to as the compound of Example 8) (0.0658 g, 0.133 mmol, 82%) was obtained as a white solid.
$^1$H-NMR (400MHz, $CDCl_3$) δ: 1.48-1.57(m, 2H), 1.70-1.77(m, 2H), 1.89-2.00(m, 1H), 2.20(s, 3H), 2.25-2.29(m, 1H), 2.94(t, J=8.7Hz, 2H), 3.17(td, J=13.3, 2.7Hz, 1H), 3.34(t, J=8.7Hz, 2H), 3.74-3.77(m, 1H), 4.70(s, 2H), 5.24-5.25(m, 1H), 7.17(d, J=8.7Hz, 2H), 7.18(d, J=1.8Hz, 1H), 7.22(d, J=1.8Hz, 1H), 7.36(d, J=8.7Hz, 2H), 8.16(s, 1H).
ESI-MS:m/z = 496(M+H)$^+$.

(Reference Example 32) Synthesis of trans-4-(trifluoromethyl)cyclohexyl)methanol:

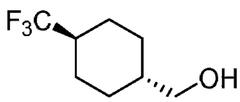

trans-4-(Trifluoromethyl)cyclohexane-1-carboxylic acid (0.400 g, 2.04 mmol) was dissolved in THF (5.1 mL) and a solution of borane-THF complex in THF (0.90 M, 2.72 mL, 2.45 mmol) was added at 0°C. After stirring the reaction mixture at room temperature for 2 hours, distilled water was added to the reaction mixture, the aqueous layer was extracted with ethyl acetate. The organic layer was washed with brine, dried over anhydrous sodium sulfate and filtered, and then the filtrate was concentrated under reduced pressure to obtain the title compound (hereinafter referred to as the compound of Reference Example 32) (0.370 g, 2.04 mmol, quantitative) as a colorless oil.
$^1$H-NMR (400MHz, $CDCl_3$) δ: 0.94-1.05(m, 2H), 1.26-1.38(m, 3H), 1.43-1.53(m, 1H), 1.91-2.02(m, 5H), 3,49(t, J=5.7Hz, 2H).

(Reference Example 33) Synthesis of trans-4-(trifluoromethyl)cyclohexane-1-carbaldehyde:

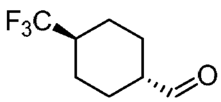

The compound of Reference Example 32 (0.370 g, 2.04 mmol) was dissolved in dichloromethane (10 mL) and Dess-Martin periodinane (0.950 g, 2.24 mmol) was added at 0°C. After stirring the reaction mixture at room temperature for 3 hours, an aqueous sodium thiosulfate solution and an aqueous saturated sodium hydrogen carbonate solution were added to the reaction mixture, and then the aqueous layer was extracted with dichloromethane. The organic layer was washed with brine, dried over anhydrous sodium sulfate and filtered, and then the filtrate was concentrated under reduced pressure. The obtained residue was purified by silica gel column chromatography (n-hexane/ethyl acetate = 90/10 to 80/20) to obtain the title compound (hereinafter referred to as the compound of Reference Example 33) (0.314 g, 1.74 mmol, 86%) as a colorless oil.
$^1$H-NMR (400MHz, $CDCl_3$) δ: 1.23-1.43(m, 4H), 1.96-2.16(m, 5H),2.20-2.28(m, 1H), 9.65(d, J=0.9Hz, 1H).

(Reference Example 34) Synthesis of 5-nitro-1-((trans-4-(trifluoromethyl)cyclohexyl)methyl)indoline:

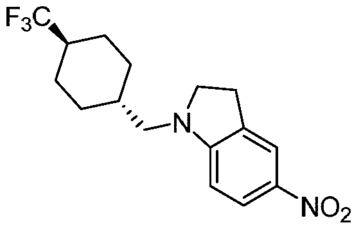

The compound of Reference Example 33 (0.100 g, 0.555 mmol) and 5-nitroindoline (0.0910 g, 0.555 mmol) were dissolved in dichloromethane (1.4 mL), and acetic acid (0.0159 mL, 0.278 mmol) and sodium triacetoxyborohydride (0.176 g, 0.833 mmol) were added at room temperature. After stirring the reaction mixture at the same temperature for 7 hours, an aqueous saturated sodium hydrogen carbonate solution was added to the reaction mixture, and then the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 90/10 to 80/20) to obtain the title compound (hereinafter referred to as the compound of Reference Example 34) (0.103 g, 0.314 mmol, 57%) as a yellow solid.
$^1$H-NMR (400MHz, $CDCl_3$) δ: 0.98-1.08(m, 2H), 1.28-1.39(m, 2H), 1.66-1.75(m, 1H), 1.91(d, J=12.8Hz, 2H), 2.01(d, J=9.1Hz, 3H), 3.07-3.12(m, 4H), 3.68(t, J=8.7Hz, 2H), 6.22(d, J=8.7Hz, 1H), 7.88-7.89(m, 1H), 8.04(dd, J=9.1, 2.3Hz, 1H).

(Reference Example 35) Synthesis of 7-chloro-5-nitro-1-((trans-4-(trifluoromethyl)cyclohexyl)methyl)ind oline:

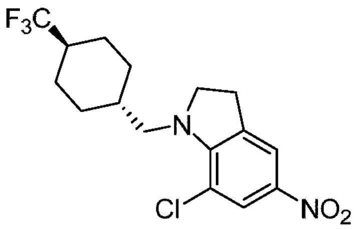

The compound of Reference Example 34 (0.100 g, 0.305 mmol) was dissolved in DMF (1.5 mL) and NCS (0.0447 g, 0.335 mmol) was added at room temperature. After stiring the reaction mixture at the same temperature for 24 hours, an aqueous sodium thiosulfate solution was added to the reaction mixture, and then the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 80/20 to 60/40) to obtain the title compound (hereinafter referred to as the compound of Reference Example 35) (0.0913 g, 0.252 mmol, 83%) as a reddish brown solid.
$^1$H-NMR (400MHz, $CDCl_3$) δ: 1.03-1.12(m, 2H), 1.28-1.39(m, 2H), 1.75-1.82(m, 1H), 1.91(d, J=13.3Hz, 2H), 1.97-2.03(m, 3H), 3.10(t, J=8.5Hz, 2H), 3,57(d, J=7.3Hz, 2H), 3.73(t, J=9.1Hz, 2H), 7.74-7.75(m, 1H), 8.00(d, J=2.3Hz, 1H).

(Reference Example 36) Synthesis of 7-chloro-1-((trans-4-(trifluoromethyl)cyclohexyl)methyl)indoline-5-amine:

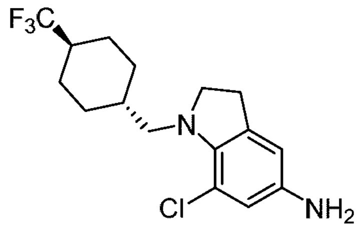

The compound of Reference Example 35 (0.0910 g, 0.251 mmol) was dissolved in a mixed solution of THF (0.50 mL), ethanol (0.50 mL) and distilled water (0.50 mL), and acetic acid (0.0718 g, 1.25 mmol) and iron powder (0.0700 g, 1.25 mmol) were added at room temperature. After stirring the reaction mixture at 50°C for 3 hours, an aqueous saturated sodium hydrogen carbonate solution was added to the reaction mixture and filtered, and then distilled water was added to the filtrate and the aqueous layer was extracted with ethyl acetate. The organic layer was washed with brine, dried over anhydrous sodium sulfate and filtered, and then the filtrate was concentrated under reduced pressure. The obtained residue was purified by silica gel column chromatography (n-hexane/ethyl acetate = 72/28 to 51/49) to obtain the title compound (hereinafter referred to as the compound of Reference Example 36) (0.0645 g, 0.194 mmol, 77%) as a brown oil.
$^1$H-NMR (400MHz, $CDCl_3$) δ: 0.97-1.07(m, 2H), 1.24-1.37(m, 2H), 1.60-1.68(m, 1H), 1.97-2.02(m, 5H), 2.93(t, J=8.5Hz, 2H), 3.16(d, J=7.3Hz, 2H), 3.34(brs, 2H), 3.36(t, J=8.5Hz, 2H), 6.42(d, J=0.9Hz, 2H).
ESI-MS:m/z = 333(M+H)$^+$.

(Example 9) Synthesis of (R)-1-acetyl-N-(7-chloro-1-((trans-4-(trifluoromethyl)cyclohexyl)m ethyl)indolin-5-yl)piperidine-2-carboxamide:

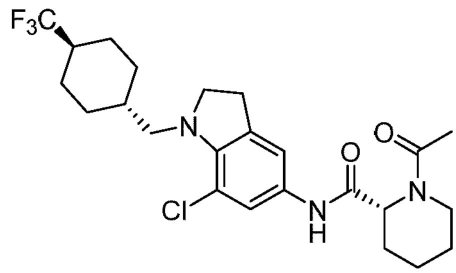

The compound of Reference Example 36 (0.0300 g, 0.0901 mmol) and the compound of Reference Example 28 (0.0185 g, 0.108 mmol) were dissolved in DMF (0.30 mL), and HATU (0.0411 g, 0.108 mmol) and diisopropylethylamine (0.0236 mL, 0.135 mmol) were added at 0°C. After stirring the reaction mixture at room temperature for 23 hours, distilled water was added to the reaction mixture, and then the aqueous layer was extracted with ethyl acetate. The organic layer was washed with an aqueous saturated sodium hydrogen carbonate solution and brine, dried over anhydrous sodium sulfate and filtered, and then the filtrate was concentrated under reduced pressure. The obtained residue was purified by silica gel column chromatography (n-hexane/ethyl acetate = 20/80 to 0/100) and recrystallized from ethyl acetate/diethyl ether to obtain the title compound (hereinafter referred to as the compound of Example 9) (0.0162 g, 0.0333 mmol, 37%) as a white crystal.
$^1$H-NMR (400MHz, $CDCl_3$) δ: 0.97-1.08(m, 2H), 1.26-1.37(m, 2H), 1.48-1.55(m, 2H), 1.65-1.76(m, 3H), 1.94-2.00(m, 6H), 2.20(s, 3H), 2.27(d, J=13.3Hz, 1H), 2.97(t, J=8.7Hz, 2H), 3.15(td, J=13.3, 2.7Hz, 1H), 3.28(d, J=7.3Hz, 2H), 3.44(t, J=8.7Hz, 2H), 3.75(d, J=14.6Hz, 1H), 5.24(d, J=5.5Hz, 1H), 7.14(d, J=2.3Hz, 1H), 7.15(d, J=2.3Hz, 1H), 8.07(brs, 1H).
ESI-MS:m/z = 486(M+H)$^+$.

(Reference Example 37) Synthesis of tert-butyl 2-((7-chloro-1-(4-(trifluoromethyl)benzyl)indolin-5-yl)carbamoyl)pi peridine-1-carboxylate:

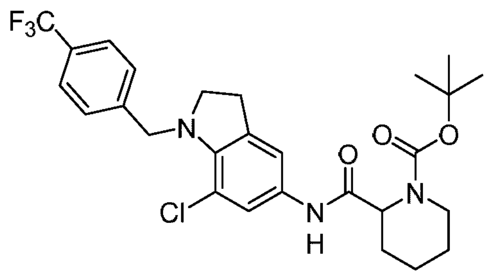

According to the same procedure as in Reference Example 17, except that 1-(tert-butoxycarbonyl)piperidine-2-carboxylic acid was used in place of (R)-1-(tert-butoxycarbonyl)piperidine-2-carboxylic acid, the title compound (hereinafter referred to as the compound of Reference Example 37) (0.495 g, 0.920 mmol, 77%) was obtained as a white amorphous.
$^1$H-NMR (400MHz, $CDCl_3$) δ: 1.46-1.69(m, 5H), 1.52(s, 9H), 2.32-2.35(m, 1H), 2.79-2.86(m, 1H), 2.95-3.00(m, 2H), 3.35-3.40(m, 2H), 4.06(brs, 1H), 4.77(s, 2H), 4.84(brs, 1H), 7.19(d, J=1.8Hz, 1H), 7.22(d, J=1.8Hz, 1H), 7.46(d, J=8.2Hz, 2H), 7.58(d, J=8.2Hz, 2H).
ESI-MS:m/z = 538(M+H)$^+$.

(Example 10) Synthesis of 1-acetyl-N-(7-chloro-1-(4-(trifluoromethyl)benzyl)indolin-5-yl)piper idine-2-carboxamide:

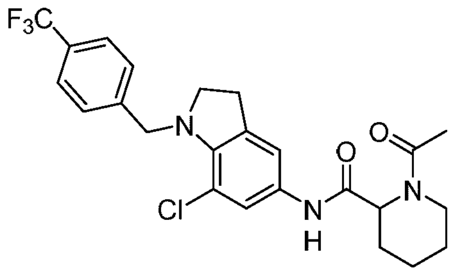

According to the same procedure as in Example 4 (Production method 1), except that the compound of Reference Example 37 was used in place of the compound of Reference Example 17, the title compound (hereinafter referred to as the compound of Example 10) (0.182 g, 0.379 mmol, 91%) was obtained as a white solid.
$^1$H-NMR (400MHz, $CDCl_3$) δ: 1.46-1.59(m, 2H), 1.70-1.77(m, 2H), 1.89-2.01(m, 1H), 2.20(s, 3H),2.26-2.29(m, 1H), 2.96(t, J=8.8Hz, 2H), 3.13-3.21(m, 1H), 3.34-3.39(m, 2H), 3.74-3.78(m, 1H), 4.75(s, 2H), 5.24-5.26(m, 1H), 7.20(brs, 1H), 7.22(brs, 1H), 7.46(d, J=8.2Hz, 2H), 7.58(d, J=8.2Hz, 2H), 8.17(s, 1H).
ESI-MS:m/z = 480(M+H)$^+$.

(Example 11) Inhibitory Effect on RORy-Coactivator Binding:

The inhibitory effect of the cyclic amine derivative (I) or a pharmacologically acceptable salt thereof on the binding between a ligand-binding domain of RORγ (hereinafter referred to as RORγ-LBD) and a coactivator was evaluated using Invitrogen's LanthaScreen™ TR-FRET Retinoid-Related Orphan Receptor (ROR) gamma Coactivator Assay kit utilizing time-resolved fluorescence energy transfer (TR-FRET).

A test compound was dissolved in DMSO and diluted with TR-FRET Coregulator Buffer D (Invitrogen) containing 5 mmol/L of DTT so as to have a final DMSO concentration of 1% before use. To each well of a 384-well black plate (Corning Inc.), 4 nmol/L of GST-fused RORγ-LBD (Invitrogen) diluted with the buffer mentioned above and the test compound were added. A well without addition of the test compound and without addition of GST-fused RORγ-LBD (background) and a well without addition of the test compound and with addition of GST-fused RORγ-LBD (control) were prepared. Next, 150 nmol/L of fluorescein-labeled TRAP220/DRIP-2 (Invitrogen) diluted with the buffer mentioned above and 32 nmol/L of terbium-labeled anti-GST antibody (Invitrogen) were added to each well. After incubating the plate at room temperature for 16 to 24 hours, the fluorescence at 495 nm and 520 nm when excited at 320 nm was measured for each well and the ratio (fluorescence value at 520 nm/fluorescence value at 495 nm) was calculated.

The fold change with addition of the test compound (ratio with addition of the test compound/ratio of the background), the fold change of the control (ratio of the control/ratio of the background), and the fold change of the background

(ratio of the background/ratio of the background) were calculated, and then the inhibition rate of binding between RORγ-LBD and a coactivator (hereinafter referred to as RORγ-coactivator binding inhibition rate) (%) was calculated from the following formula 1:

[Formula 1]

RORγ-coactivator binding inhibition rate (%) = (1 - ((Fold change with addition of the test compound) - (Fold change of the background))/((Fold change of the control) - (Fold change of the background))) × 100

The RORγ-coactivator binding inhibition rate (%) at 33 μmol/L of the test compound is shown in Table 2.

[Table 2]

| Test compound | RORγ-coactivator binding inhibition rate (%) |
|---|---|
| Compound of Example 1 | 96.9 |
| Compound of Example 2 | 94.3 |
| Compound of Example 3 | 99.5 |
| Compound of Example 4 | 96.7 |
| Compound of Example 5 | 87.9 |
| Compound of Example 6 | 85.2 |
| Compound of Example 7 | 96.0 |
| Compound of Example 8 | 99.4 |
| Compound of Example 9 | 96.8 |
| Compound of Example 10 | 99.8 |

These results revealed that the cyclic amine derivative (I) or a pharmacologically acceptable salt thereof markedly inhibits the binding between RORγ-LBD and a coactivator.

(Example 12) Suppressive Effect on IL-17 Production in Mouse Splenocytes:

Using mouse splenocytes, the suppressive effect of the cyclic amine derivative (I) or a pharmacologically acceptable salt thereof on IL-17 production by IL-23 stimulation was evaluated by a partially modified method mentioned in The Journal of Biological Chemistry, 2003, Vol. 278, No. 3, p.1910-1914.

A single cell suspension was prepared from the spleen of C57BL/6J mice (male or female, 6 to 30 weeks old) (Charles River Laboratories Japan, Inc. or CLEA Japan, Inc.) and splenocytes were prepared using Histopaque-1083 (Sigma-Aldrich Japan). The culture medium was used by adding 10% FBS (Gibco), 50 U/mL of penicillin·50 μg/mL of streptomycin (Gibco), 50 μmol/L of 2-mercaptoethanol (Gibco), and 100 U/mL of human IL-2 (Cell Science & Technology Institute, Inc.) to RPMI1640 medium (Gibco). A test compound was dissolved in DMSO and then diluted with the culture medium so as to have a final concentration of DMSO of 0.1% before use. Splenocytes ($3 \times 10^5$ cells/well) prepared in the culture medium were seeded in wells of a 96-well flat-bottom plate (Corning Incorporated), the test compound and 10 ng/mL of human IL-23 (R & D systems, Inc.) were added thereto, and the cells were cultured at 37°C under 5% $CO_2$ for 3 days. A well without addition of human IL-23 and without addition of the test compound and a well with addition of human IL-23 and without addition of the test compound were prepared. After completion of the culture, the culture supernatant was collected and the IL-17 production amount in the supernatant was determined by ELISA method (R & D systems, Inc.).

The IL-17 production inhibition rate (%) was calculated from the following Formula 2.

[Formula 2]

IL-17 production inhibition rate (%) = (1 - ((IL-17 production amount with addition of IL-23 and with addition of the test compound) - (IL-17 production amount without addition of IL-23 and without addition of the test compound))/((IL-17 production amount with addition of IL-23 and without addition of the test compound) - (IL-17 production amount without addition of IL-23 and without addition of the test compound))) × 100

The IL-17 production inhibition rate (%) at 5 μmol/L of the test compound is shown in Table 3.

[Table 3]

| Test compound | IL-17 production inhibition rate (%) |
|---|---|
| Compound of Example 1 | 98.0 |
| Compound of Example 2 | 98.4 |
| Compound of Example 3 | 99.7 |
| Compound of Example 4 | 99.9 |
| Compound of Example 5 | 87.5 |
| Compound of Example 6 | 91.6 |
| Compound of Example 7 | 98.6 |
| Compound of Example 8 | 100.0 |
| Compound of Example 9 | 99.5 |
| Compound of Example 10 | 99.5 |

These results revealed that the cyclic amine derivative (I) or a pharmacologically acceptable salt thereof suppresses IL-17 production.

(Example 13) Symptom Suppressive Effect on Imiquimod-induced Mouse Psoriasis Model:

Using increase in the ear thickness as an index of exacerbation of symptoms, the effect of the cyclic amine derivative (I) or a pharmacologically acceptable salt thereof in an imiquimod-induced mouse psoriasis model was evaluated. The imiquimod-induced mouse psoriasis model was prepared by a partially modified method by Schaper et al. (The Journal of Dermatological Science, 2013, Vol. 71, No. 1, p.29-36).

BALB/c mice (male, 7 weeks old) (Charles River Laboratories Japan, Inc.) were used at 8 to 9 weeks old after preliminary breeding. To induce psoriasis-like symptoms, 5 mg each of BESELNA CREAM 5% was applied once daily to the outside of the right and left auricles of the mice for 8 days from the day of first administration of imiquimod (hereinafter referred to as induction day) to 7 days after induction (dose of imiquimod, 0.5 mg/body/day).

A test compound at a dose of 10 mg/kg was administered to the mice once daily for 5 days from 3 days after induction to 7 days after induction. As the test compound, the compound of Example 1, the compound of Example 4, and the compound of Example 9 were used. The compound of Example 1, the compound of Example 4, and the compound of Example 9 were respectively suspended in a 0.5 w/v% methylcellulose solution and orally administered. The group in which the compound of Example 1 was administered to mice was defined as the Example-1 compound administration group, the group in which the compound of Example 4 was administered was defined as the Example-4 compound administration group, and the group in which the compound of Example 9 was administered was defined as the Example-9 compound administration group. In the vehicle administration group, a vehicle of each test compound (0.5 w/v% methylcellulose solution) was similarly administered.

The right and left ear thickness before administration of imiquimod (before induction) on the induction day and

the right and left ear thickness on the 8th day after induction were measured with a digital micrometer (Mitutoyo Corporation). The mean of the right and left ear thickness was regarded as ear thickness, and the change in the ear thickness (ear thickness on the 8th day after induction - ear thickness before induction) was used as an index of the drug efficacy evaluation.

The results are shown in FIGS. 1, 2, and 3. The vertical axis represents the change in the ear thickness (mm) (mean ± standard error, n = 6). "Vehicle" on the horizontal axis represents the vehicle administration group, the "Example-1 compound" represents the Example-1 compound administration group, the "Example-4 compound" represents the Example-4 compound administration group, and the "Example-9 compound" represents the Example-9 compound administration group. The mark of asterisk (*) indicates statistical significance by comparison with the vehicle administration group (Student's t-test) (*: P < 0.05).

Induction by imiquimod increased the ear thickness on the 8th day after induction in the vehicle administration group by 0.24 mm to 0.28 mm compared with the ear thickness before induction. This increase in the ear thickness was statistically significantly suppressed by administration of the compound of Example 1, the compound of Example 4, or the compound of Example 9.

These results revealed that the cyclic amine derivative (I) or a pharmacologically acceptable salt thereof shows marked symptom suppressive effect on psoriasis.

(Example 14) Symptom Suppressive Effect on DNFB-induced Mouse Allergic Dermatitis Model:

Using increase in the ear swelling rate as an index of exacerbation of symptoms, the effect of the cyclic amine derivative (I) or a pharmacologically acceptable salt thereof in a DNFB-induced mouse allergic dermatitis model was evaluated. The DNFB-induced mouse allergic dermatitis model was prepared by a partially modified method by Curzytek et al. (Pharmacological Reports, 2013, Vol. 65, p.1237-1246).

BALB/c mice (female, 6 weeks old) (Charles River Laboratories Japan, Inc.) were used at 8 weeks old after preliminary breeding. To the back of the mice, 25 μL of a 0.5 v/v% DNFB solution dissolved in acetone:olive oil (4:1) was applied. The next day, the same operation was repeated to sensitize the mice. Four days after the sensitization, 10 μL each of a 0.2 v/v% DNFB solution dissolved in acetone:olive oil (4:1) was applied to both sides of the right auricle of the sensitized mice to induce inflammation.

One hour before induction, a test compound at a dose of 10 mg/kg was administered to the mice. As the test compound, the compound of Example 1, the compound of Example 4, and the compound of Example 9 were used. The compound of Example 1, the compound of Example 4, and the compound of Example 9 were respectively suspended in a 0.5 w/v% methylcellulose solution and orally administered. The group in which the compound of Example 1 was administered to mice was defined as the Example-1 compound administration group, the group in which the compound of Example 4 was administered was defined as the Example-4 compound administration group, and the group in which the compound of Example 9 was administered was defined as the Example-9 compound administration group. In the vehicle administration group, a vehicle of each test compound (0.5 w/v% methylcellulose solution) was similarly administered.

The right ear thickness before application of the DNFB solution (before induction) on the induction day and the right ear thickness at the 24th hour after induction were measured with a digital micrometer (Mitutoyo Corporation). The swelling rate of the auricle was calculated by the following Formula 3 and used as an index of the drug efficacy evaluation.

[Formula 3]

Ear swelling rate (%) = ((Right ear thickness at the 24th hour after induction) - (Right ear thickness before induction))/Right ear thickness before induction × 100

The results are shown in FIGS. 4, 5, and 6. The vertical axis represents the ear swelling rate (%) (mean ± standard error, n = 6). "Vehicle" on the horizontal axis represents the vehicle administration group, the "Example-1 compound" represents the Example-1 compound administration group, the "Example-4 compound" represents the Example-4 compound administration group, and the "Example-9 compound" represents the Example-9 compound administration group. The mark of asterisk (*) indicates statistical significance by comparison with the vehicle administration group (Student's t-test) (*: P < 0.05).

The ear swelling rate in the vehicle administration group due to application of the DNFB solution to the auricle was 41.6%. This increase in the ear swelling rate was statistically significantly suppressed by administration of the compound of Example 1, the compound of Example 4, or the compound of Example 9.

This result revealed that the cyclic amine derivative (I) or a pharmacologically acceptable salt thereof shows marked symptom suppressive effect on allergic dermatitis, particularly contact dermatitis.

(Example 15) Symptom Suppressive Effect on Oxazolone-induced Mouse Atopic Dermatitis Model:

Using increase in the ear thickness as an index of exacerbation of symptoms, the effect of the cyclic amine derivative (I) or a pharmacologically acceptable salt thereof in an oxazolone-induced mouse atopic dermatitis model was evaluated. The oxazolone-induced mouse atopic dermatitis model was prepared by a partially modified method by Nakajima et al. (Journal of Investigative Dermatology, 2014, Vol. 134, p.2122-2130).

BALB/c mice (female, 7 weeks old) (Charles River Laboratories Japan, Inc.) were used at 8 weeks old after preliminary breeding. To the back of the mice, 25 μL of a 3 w/v% oxazolone solution dissolved in ethanol was applied to sensitize the mice. Every other day from 5 days to 13 days after the sensitization, 10 μL each of a 0.6 w/v% oxazolone solution dissolved in ethanol was applied to both sides of the right auricle of the sensitized mice to induce inflammation.

A test compound at a dose of 10 mg/kg was administered to the mice once daily for 15 days from the sensitization day to 14 days after sensitization. As the test compound, the compound of Example 1, the compound of Example 4, and the compound of Example 9 were used. The compound of Example 1, the compound of Example 4, and the compound of Example 9 were suspended in a 0.5 w/v% methylcellulose solution and orally administered. The group in which the compound of Example 1 was administered to mice was defined as the Example-1 compound administration group, the group in which the compound of Example 4 was administered was defined as the Example-4 compound administration group, and the group in which the compound of Example 9 was administered was defined as the Example-9 compound administration group. In the vehicle administration group, a vehicle of each test compound (0.5 w/v% methylcellulose solution) was similarly administered.

The right ear thickness before application of the oxazolone solution (before sensitization) on the sensitization day and the right ear thickness on the next day of final induction were measured with a digital micrometer (Mitutoyo Corporation). The change in the ear thickness (right ear thickness on the next day of final induction - right ear thickness before sensitization) was used as an index of the drug efficacy evaluation.

The results are shown in FIGS. 7, 8, and 9. The vertical axis represents the change in the ear thickness (mm) (mean ± standard error, n = 6). "Vehicle" on the horizontal axis represents the vehicle administration group, the "Example-1 compound" represents the Example-1 compound administration group, the "Example-4 compound" represents the Example-4 compound administration group, and the "Example-9 compound" represents the Example-9 compound administration group. The mark of asterisk (*) indicates statistical significance by comparison with the vehicle administration group (Aspin-Welch's t-test or Student's t-test) (*: $P < 0.05$).

Application of the oxazolone solution to the auricle increased the ear thickness on the next day of final induction in the vehicle administration group by 0.68 mm compared with the ear thickness before sensitization. This increase in the ear thickness was statistically significantly suppressed by administration of the compound of Example 1, the compound of Example 4, or the compound of Example 9.

This result revealed that the cyclic amine derivative (I) or a pharmacologically acceptable salt thereof shows marked symptom suppressive effect on allergic dermatitis, particularly atopic dermatitis.

(Example 16) Symptom Suppressive Effect on Mouse Alopecia Areata Model:

The effect of the cyclic amine derivative (I) or a pharmacologically acceptable salt thereof in a mouse alopecia areata model was evaluated using increase in the hair loss score as an index of worsening of symptoms. The mouse alopecia areata model was prepared by a partially modified method by Wang et al. (Journal of Investigative Dermatology, 2015, vol.135, p.2530-2532).

Female C3H/HeJ mice (CLEA Japan, Inc.) with spontaneous hair loss in 70% or more of the body surface were used as donor mice. The donor mice were euthanized by cervical dislocation, and then the groin, axilla, and auricular lymph nodes were aseptically removed. The lymph nodes were filtered through a 70 μm cell strainer to isolate lymphocytes. The lymphocytes were washed with Advanced RPMI media (containing 10% fetal bovine serum,2 mM Gluta Max, and 100 U/mL penicillin streptomycin), and then suspended in Advanced RPMI media to which Human rIL-2 (Roche; final concentration of 30 U/mL), Mouse rIL-7 (R&D Systems, Inc.; final concentration of 25 ng/mL), and Mouse rIL-15 (R&D Systems, Inc.; final concentration of 50 ng/mL) were added so that the concentration was $2 \times 10^6$ cells/mL. The lymphocytes were seeded in 1 mL portions on 24-well plates, and 500 μL of Dynabead mouse T-activator CD3/CD28 (Life Technologies) was added, followed by culture in a $CO_2$ incubator. Culture was performed for 6 days so that the cell density was 1.5 to $2.0 \times 10^6$ cells/mL during culture.

The day of transplantation of lymphocytes derived from donor mice was regarded as Day 0. Two days before the day of transplantation (Day -2), hair on the back, which would be a transplantation site, of 10-week old female C3H/HeJ mice without hair loss (CLEA Japan, Inc.) was removed with an electric hair clipper under isoflurane anesthesia

(1.5 cm × 1.5 cm). Furthermore, grouping was performed using the body weight on Day -1 as an index.

On Day 0, lymphocytes in which beads were removed with EasySep magnet (Stemcell Technologies Inc.) were collected into new tubes. The collected lymphocytes were suspended in PBS(-) so that the concentration was 10 × $10^7$ cells/mL, filled in a 1 mL syringe with a 26G injection needle, and stored on ice until transplantation. Under isoflurane anesthesia, the filled lymphocyte suspension was intradermally administered to the hair removal site in 100 μL/body portions. PBS(-) was intradermally administered in 100 μL/body portions to mice without transplantation of lymphocytes. Mice without transplantation of lymphocytes were regarded as the normal group. Mice with and without transplantation of lymphocytes were maintained on a low-fat diet (CR-LPF; Oriental Yeast Co., Ltd.) after Day 0 until Day 49.

On Day 49, photographs of the back and the abdomen of the mice were taken under isoflurane anesthesia. The status of hair loss was evaluated by a partially modified method by Alli et al. (Journal of Immunology, 2012, vol.188, p.477-486). In other words, the ratio of the area of hair loss site to the body surface area was scored in accordance with the criteria mentioned in Table 4, and regarded as the hair loss score. Specifically, the hair loss score was determined based on the numerical value of the ratio calculated from the following formula 4:

[Formula 4]

$$\text{Ratio of hair loss site (\%)} = (\text{Area of hair loss site/body surface area}) \times 100$$

[Table 4]

| Hair loss score | Ratio of hair loss site (%) (area of hair loss site/body surface area × 100) |
|---|---|
| 0 | ≤ 5 |
| 1 | > 5 to ≤ 10 |
| 2 | > 10 to ≤ 15 |
| 3 | > 15 to ≤ 20 |
| 4 | >20 to ≤ 25 |
| 5 | >25 to ≤ 30 |
| 6 | > 30 to ≤ 35 |
| 7 | >35 to ≤ 40 |
| 8 | > 40 to ≤ 45 |
| 9 | > 45 to ≤ 50 |
| 10 | > 50% |

Grouping was performed using the mouse hair loss score calculated on Day 49 as an index (the hair loss score on Day 49 (mean ± standard error): 3.6 ± 0.66). A test compound was administered to mice with hair loss symptoms at a dose of 10 mg/kg once daily for 42 days from Day 49 to Day 90. The compound of Example 4 was used as the test compound. The compound of Example 4 was orally administered after suspended in a 0.5 w/v% methylcellulose solution. The group in which the compound of Example 4 was administered was defined as the Example-4 compound administration group. A vehicle (0.5 w/v% methylcellulose solution) of each test compound was administered in the same manner to the normal group and the disease group (vehicle administration group).

On Day 91, the mouse hair loss score was calculated. The results are shown in FIG. 10. The vertical axis represents the hair loss score (mean ± standard error) of each group. On the horizontal axis, "Normal" represents the normal group (number of individuals: n = 3), "Vehicle" represents the disease group (vehicle administration group) (n = 6), and the "Example-4 compound" represents the Example-4 compound administration group (n = 5). Compared with the mean hair loss score on Day 49, the hair loss score on Day 91 was remarkably increased in the disease group (vehicle administration group). This increase in the hair loss score was suppressed by administration of the compound of Example 4.

These results revealed that the cyclic amine derivative (I) or a pharmacologically acceptable salt thereof shows marked symptom suppressive effect on hair loss symptoms.

INDUSTRIAL APPLICABILITY

Since the cyclic amine derivative (I) or a pharmacologically acceptable salt thereof according to the present invention has excellent RORγ antagonist activity, it can be used as a medicament for diseases in which improvement in the pathological state or remission of symptoms can be expected by suppression of the function of RORγ. Particularly, the cyclic amine derivative (I) or a pharmacologically acceptable salt thereof according to the present invention can be used as a therapeutic agent or preventive agent for autoimmune diseases, such as psoriasis, or allergic diseases, such as allergic dermatitis.

## Claims

**1.** A cyclic amine derivative represented by the following general formula (I):

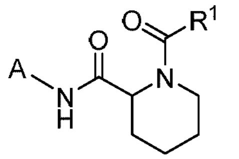 (I)

wherein

$R^1$ represents an alkyl group having 1 to 3 carbon atoms;
A represents a group represented by the following general formula (II-1), (II-2), or (II-3):

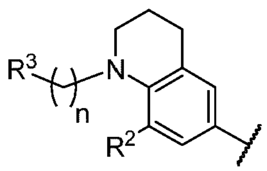
(II-1)

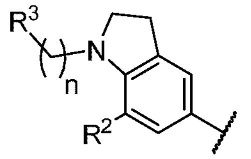
(II-2)

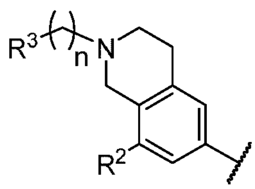
(II-3)

$R^2$ represents a hydrogen atom or a halogen atom;
$R^3$ represents an aryl group or a cycloalkyl group having 4 to 6 carbon atoms, wherein any 1 or 2 hydrogen atoms of the aryl or cycloalkyl group represented by $R^3$ may be each independently substituted with an alkyl group having 1 to 3 carbon atoms or an alkyloxy group having 1 to 3 carbon atoms, and wherein any 1 to 3 hydrogen atoms of the alkyl or alkyloxy group having 1 to 3 carbon atoms which can be a substituent of the aryl or cycloalkyl group may be each independently substituted with a halogen atom;
n represents 1 or 2;
a wavy line represents the point of attachment to the general formula (I),

or a pharmacologically acceptable salt thereof.

**2.** The cyclic amine derivative according to claim 1, wherein:

$R^2$ is a hydrogen atom, a fluorine atom, or a chlorine atom; and
$R^3$ is an aryl group or a cycloalkyl group having 4 to 6 carbon atoms, wherein any 1 or 2 hydrogen atoms of the aryl or cycloalkyl group represented by $R^3$ may be each independently substituted with a methyl group or a methoxy group, and wherein any 1 to 3 hydrogen atoms of the methyl or methoxy group which can be a substituent of the aryl or cycloalkyl group may be each independently substituted with a fluorine atom or a chlorine atom,

or a pharmacologically acceptable salt thereof.

**3.** The cyclic amine derivative according to claim 1, wherein:

$R^2$ is a fluorine atom or a chlorine atom;
$R^3$ is a phenyl group or a cyclohexyl group, wherein any 1 or 2 hydrogen atoms of the phenyl or cyclohexyl group represented by $R^3$ may be each independently substituted with a methyl group or a methoxy group, and wherein any 1 to 3 hydrogen atoms of the methyl or methoxy group which can be a substituent of the phenyl or cyclohexyl group may be each independently substituted with a fluorine atom or a chlorine atom; and
n is 1,

or a pharmacologically acceptable salt thereof.

**4.** The cyclic amine derivative according to claim 1, wherein:

$R^1$ is a methyl group;
A is a group represented by the following general formula (II-1) or (II-2):

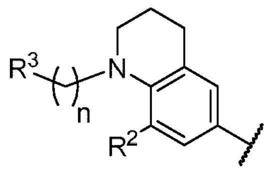

(II-1)

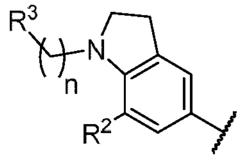

(II-2)

$R^2$ is a chlorine atom;
$R^3$ is a phenyl group or a cyclohexyl group, wherein any 1 hydrogen atom of the phenyl or cyclohexyl group represented by $R^3$ may be each independently substituted with a trifluoromethyl group or a trifluoromethoxy group;
n is 1; and
a wavy line represents the point of attachment to the general formula (I),

or a pharmacologically acceptable salt thereof.

**5.** A medicament, comprising the cyclic amine derivative according to any one of claims 1 to 4 or a pharmacologically acceptable salt thereof as an active ingredient.

**6.** A retinoid-related orphan receptor γ antagonist, comprising the cyclic amine derivative according to any one of claims 1 to 4 or a pharmacologically acceptable salt thereof as an active ingredient.

**7.** A therapeutic agent or preventive agent for an autoimmune disease, comprising the cyclic amine derivative according to any one of claims 1 to 4 or a pharmacologically acceptable salt thereof as an active ingredient.

**8.** A therapeutic agent or preventive agent for psoriasis or alopecia areata, comprising the cyclic amine derivative according to any one of claims 1 to 4 or a pharmacologically acceptable salt thereof as an active ingredient.

**9.** A therapeutic agent or preventive agent for an allergic disease, comprising the cyclic amine derivative according to any one of claims 1 to 4 or a pharmacologically acceptable salt thereof as an active ingredient.

**10.** A therapeutic agent or preventive agent for allergic dermatitis, comprising the cyclic amine derivative according to any one of claims 1 to 4 or a pharmacologically acceptable salt thereof as an active ingredient.

**11.** A therapeutic agent or preventive agent for contact dermatitis or atopic dermatitis, comprising the cyclic amine derivative according to any one of claims 1 to 4 or a pharmacologically acceptable salt thereof as an active ingredient.

FIG. 1

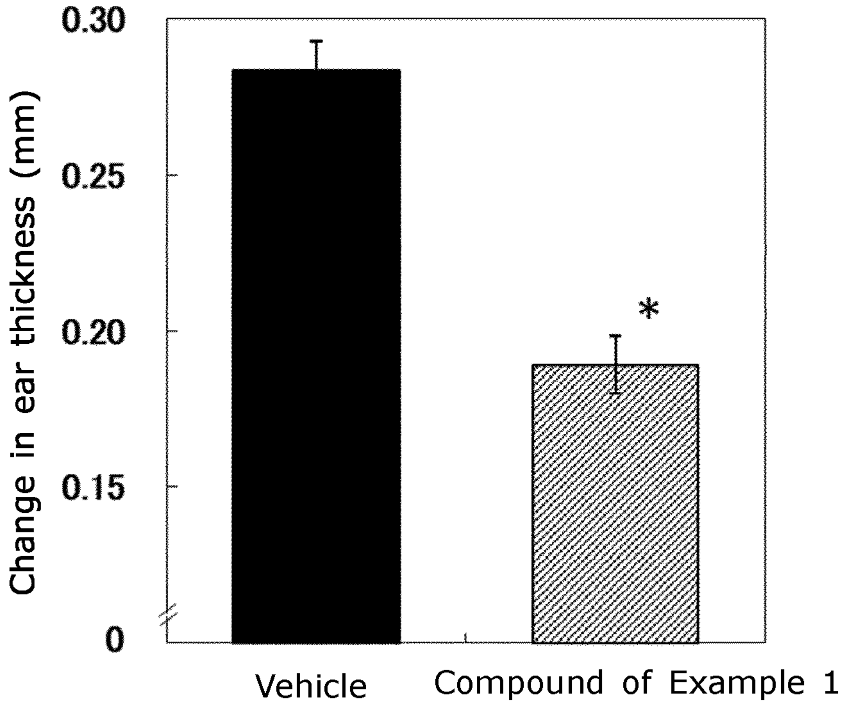
Change in ear thickness (mm)
0.30
0.25
0.20
0.15
0
*
Vehicle
Compound of Example 1

FIG. 2

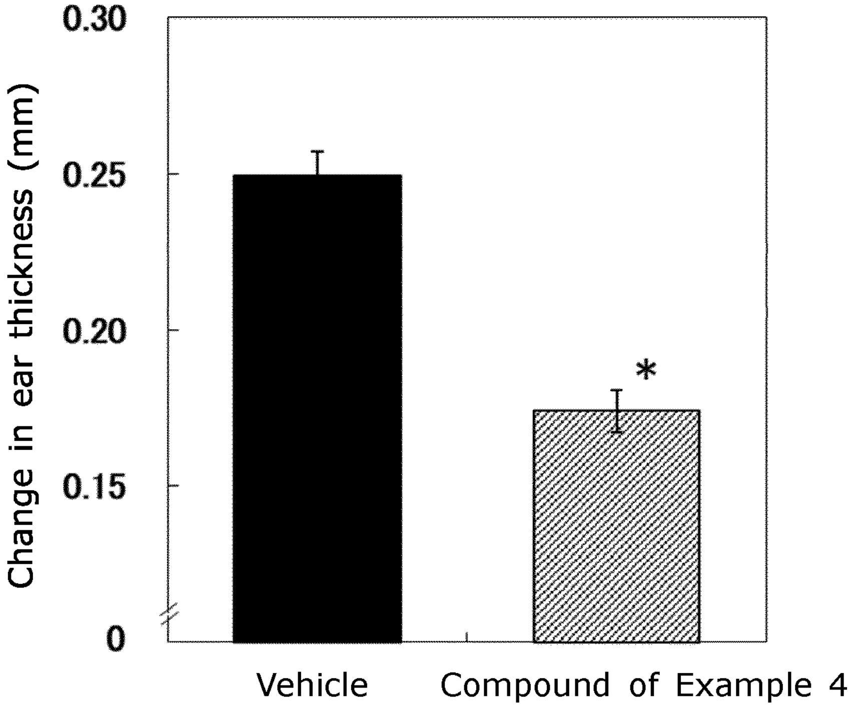
0.30
0.25
0.20
0.15
0
Change in ear thickness (mm)
*
Vehicle
Compound of Example 4

FIG. 3

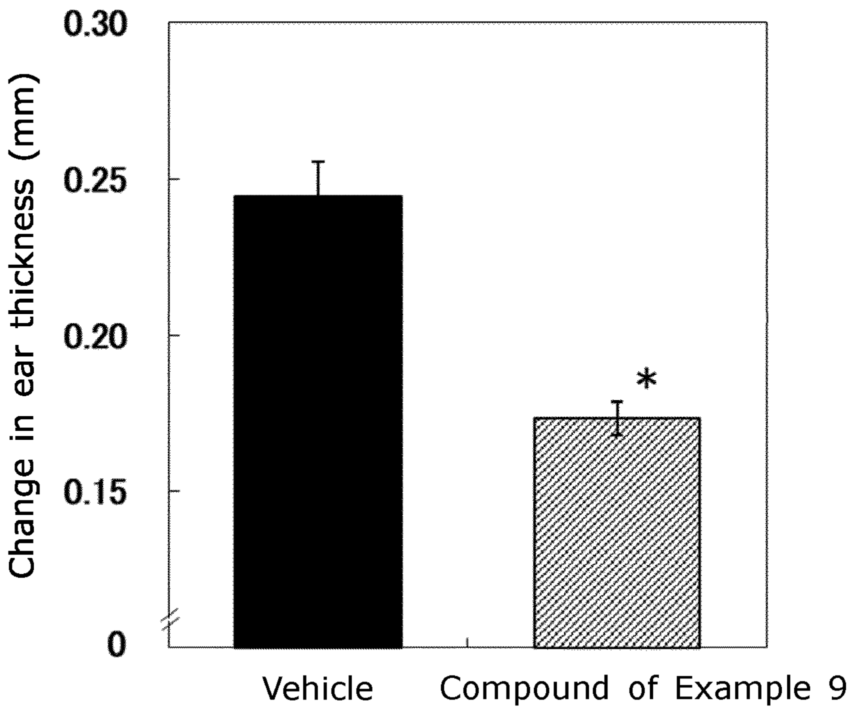
0.30
0.25
0.20
0.15
0
Change in ear thickness (mm)
*
Vehicle
Compound of Example 9

FIG. 4

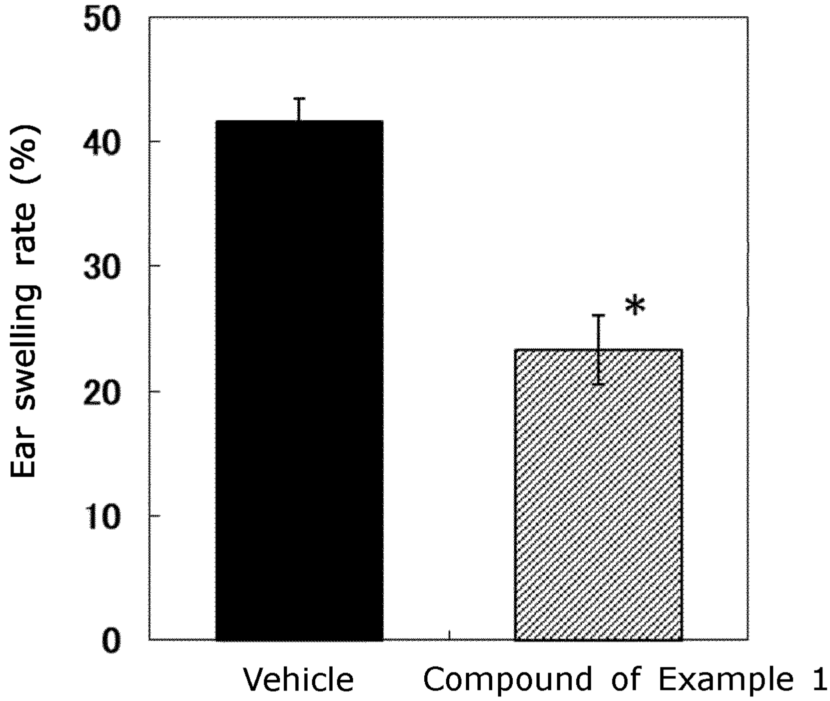
50
40
30
20
10
0
Ear swelling rate (%)
*
Vehicle
Compound of Example 1

FIG. 5

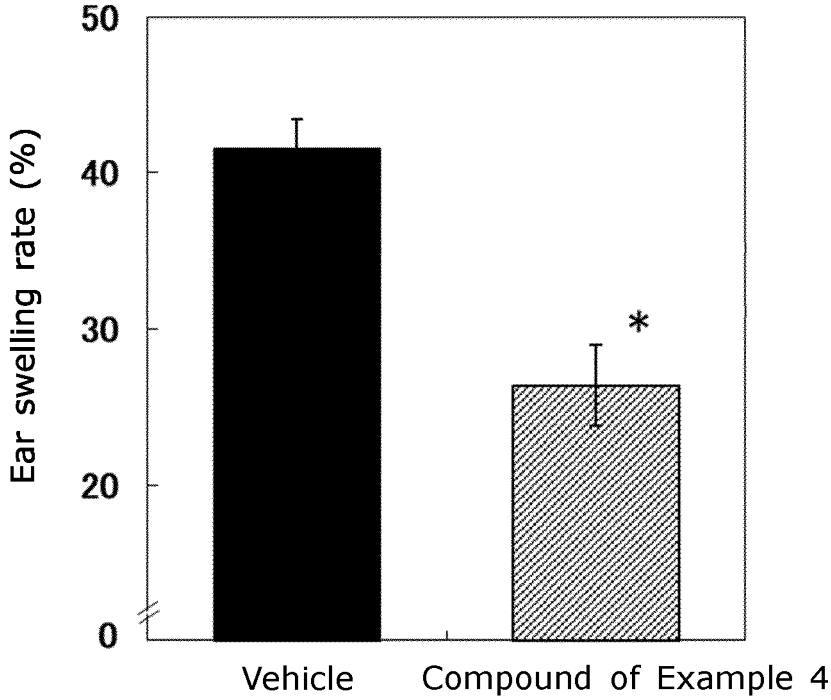

FIG. 6

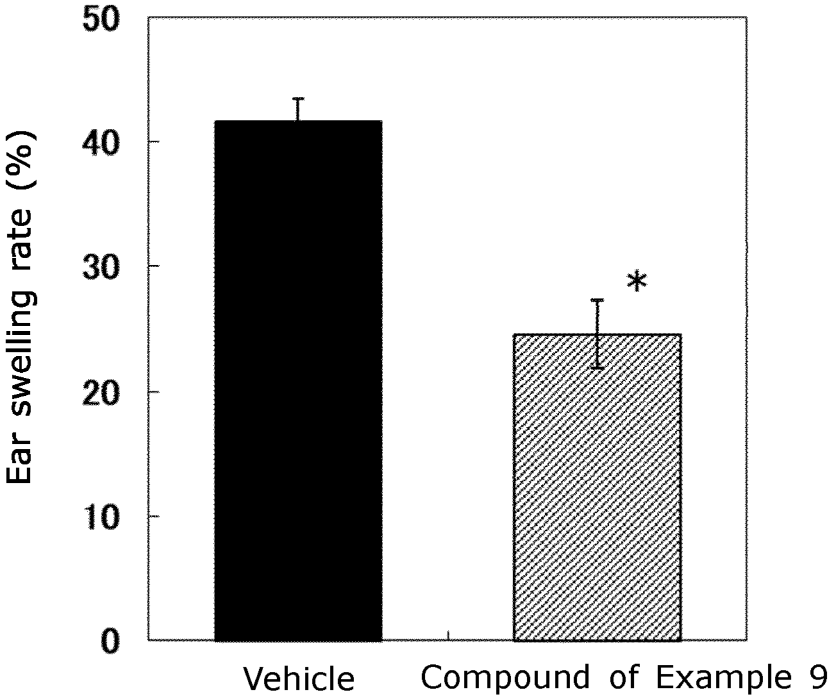
50
40
30
20
10
0
Ear swelling rate (%)
*
Vehicle
Compound of Example 9

FIG. 7

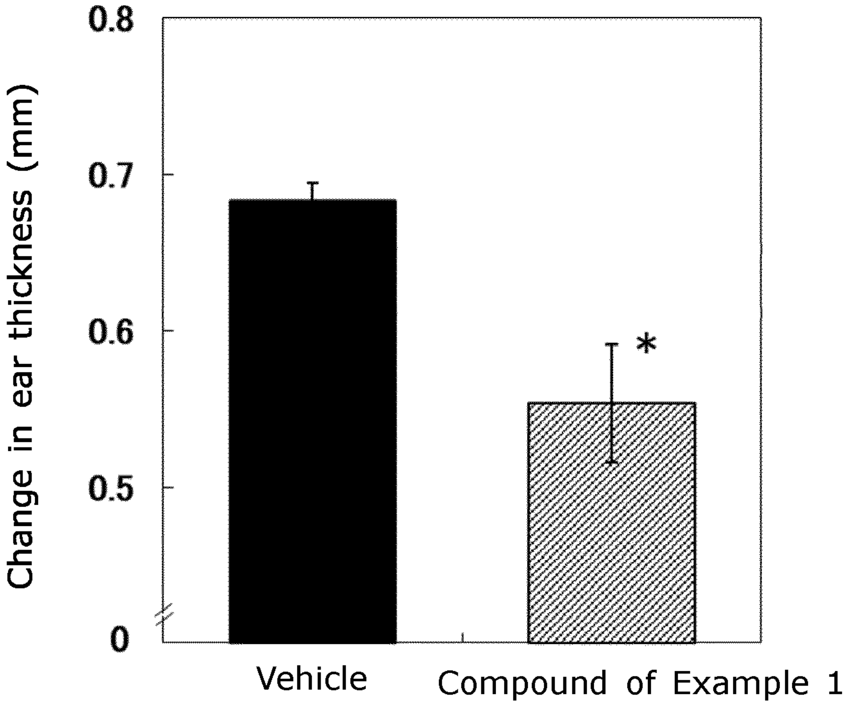
0.8
0.7
0.6
0.5
0
Change in ear thickness (mm)
*
Vehicle
Compound of Example 1

FIG. 8

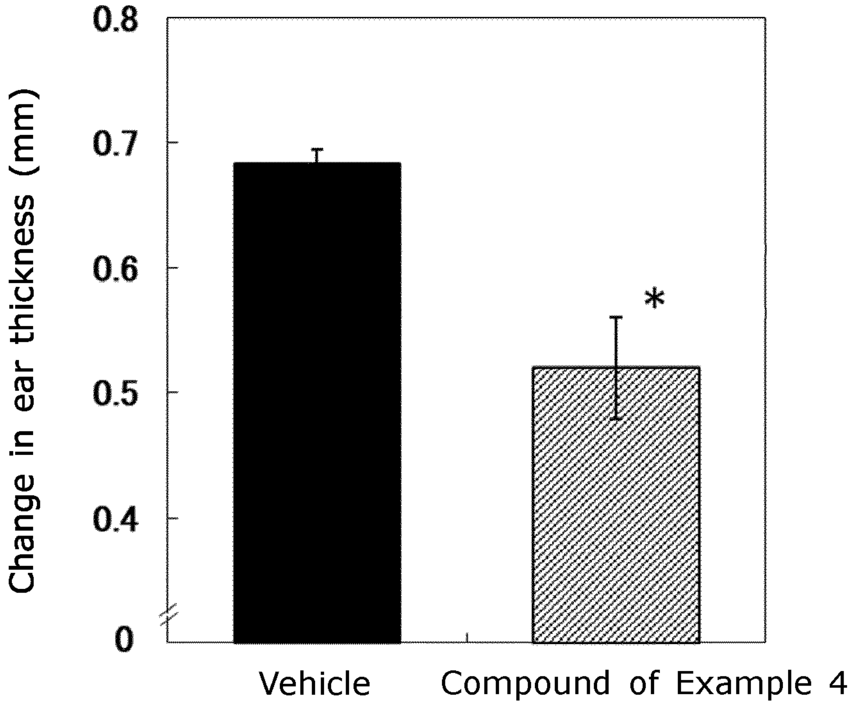
0.8
0.7
0.6
0.5
0.4
0
Change in ear thickness (mm)
*
Vehicle
Compound of Example 4

FIG. 9

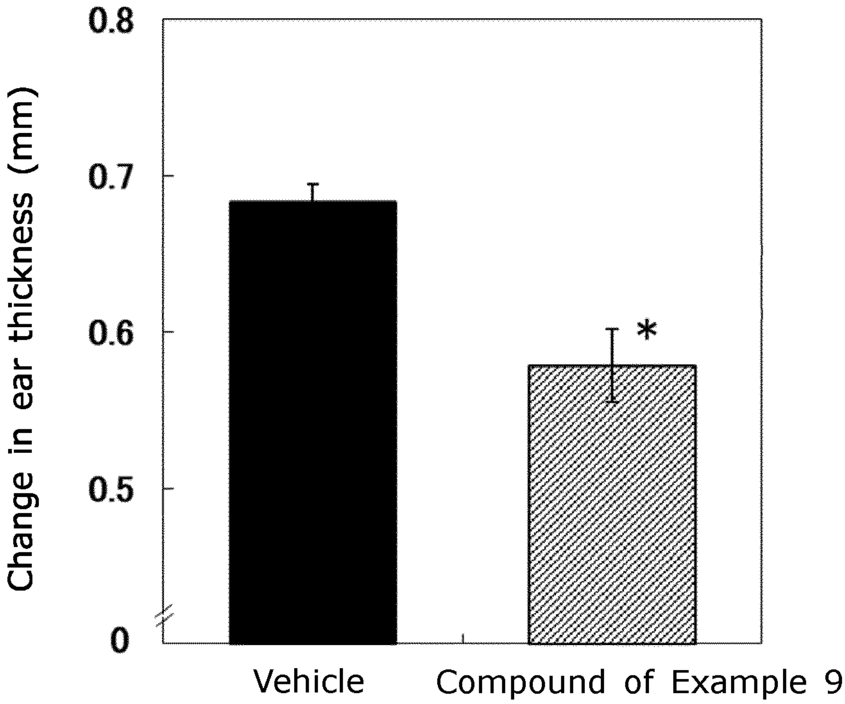
Change in ear thickness (mm)
0.8
0.7
0.6
0.5
0
*
Vehicle
Compound of Example 9

FIG. 10

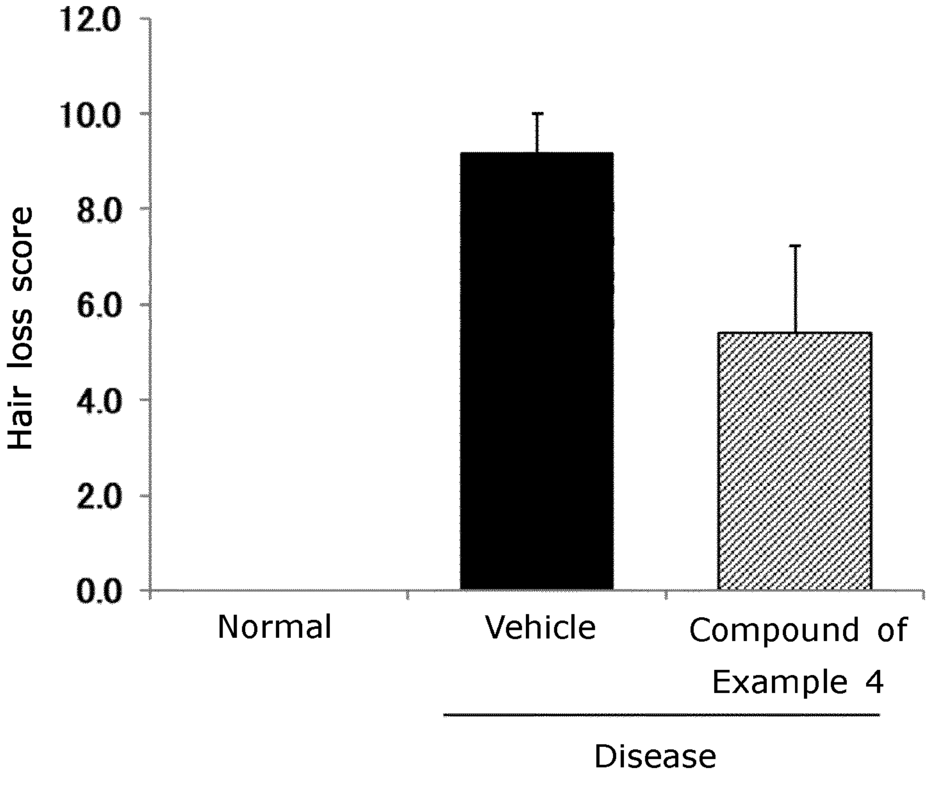
12.0
10.0
8.0
6.0
4.0
2.0
0.0
Hair loss score
Normal
Vehicle
Compound of Example 4
Disease

## INTERNATIONAL SEARCH REPORT

International application No.
PCT/JP2019/003046

A. CLASSIFICATION OF SUBJECT MATTER
Int.Cl. C07D401/12(2006.01)i, A61K31/454(2006.01)i, A61K31/4709(2006.01)i, A61K31/4725(2006.01)i, A61P17/06(2006.01)i, A61P37/02(2006.01)i, A61P43/00(2006.01)i

According to International Patent Classification (IPC) or to both national classification and IPC

B. FIELDS SEARCHED
Minimum documentation searched (classification system followed by classification symbols)
Int.Cl. C07D401/12, A61K31/454, A61K31/4709, A61K31/4725, A61P17/06, A61P37/02, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2015-124178 A (TORAY INDUSTRIES, INC.) 06 July 2015, entire text, particularly, a cyclic amine derivative represented by formula (I) (Family: none) | 1-11 |
| A | WO 2016/072402 A1 (DAIICHI SANKYO CO., LTD.) 12 May 2016, entire text, particularly, a compound represented by formula (I) & TW 201625588 A | 1-11 |
| A | WO 2017/131156 A1 (TORAY INDUSTRIES, INC.) 03 August 2017, entire text, particularly, a cyclic amine derivative represented by formula (I) & CA 3009971 A & TW 201731816 A & AU 2017210685 A & MX 2018008454 A & CN 108473426 A & KR 10-2018-0100573 A & EP 3409660 A1 & US 2018/0370916 A1 | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29.03.2019 | 09.04.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/003046

C (Continuation). DOCUMENTS CONSIDERED TO BE RELEVANT

| Category* | Citation of document, with indication, where appropriate, of the relevant passages | Relevant to claim No. |
|---|---|---|
| A | WO 2016/014910 A1 (INNOV17 LLC) 28 January 2016, entire text, particularly, a compound of formula (I) & US 2016/0024056 A1 & EP 3172195 A1 | 1-11 |
| E, A | WO 2019/022223 A1 (TORAY INDUSTRIES, INC.) 31 January 2019, entire text, particularly, a cyclic amine derivative represented by formula (I) (Family: none) | 1-11 |
| E, A | WO 2019/044940 A1 (TORAY INDUSTRIES, INC.) 07 March 2019, entire text, particularly, a cyclic amine derivative represented by formula (I) (Family: none) | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012236822 A
- WO 2013029338 A
- US 20160122318 A
- WO 2017131156 A
- WO 2010026113 A
- WO 2005051934 A
- WO 2012158784 A
- WO 2013018695 A
- WO 2012064744 A


### Non-patent literature cited in the description


- **CHEN et al.** *International Immunopharmacology,* 2011, vol. 11, 536-542
- **HOFMANN et al.** *Current Opinion in Allergy and Clinical Immunology,* 2016, vol. 16, 451-457
- **IVANOV et al.** *Cell,* 2006, vol. 126, 1121-1133
- **JETTEN.** *Nuclear Receptor Signaling,* 2009, vol. 7, e003
- **HAMZAOUI et al.** *Medical Science Monitor,* 2011, vol. 17, CR227-234
- **MA et al.** *Journal of the European Academy of Dermatology and Venereology,* 2014, vol. 28, 1079-1086
- **LEPPKES et al.** *Gastroenterology,* 2009, vol. 136, 257-267
- **JIN et al.** *Molecular Endocrinology,* 2010, vol. 24, 923-929
- **SOLT et al.** *Nature,* 2011, vol. 472, 491-494
- **ZHAO et al.** *British Journal of Dermatology,* 2009, vol. 161, 1301-1306
- **JETTEN et al.** *The Journal of Immunology,* 2007, vol. 178, 3208-3218
- **BRITTAIN, H.G.** Polymorphism in Pharmaceutical Solids. CRC Press
- **GREENE, TW.** Greene's Protective Groups in Organic Synthesis. Wiley-Interscience
- *The Journal of Biological Chemistry,* 2003, vol. 278 (3), 1910-1914
- *Journal of Neuroscience Research,* 2006, vol. 84, 1225-1234
- *Journal of Immunology,* 2009, vol. 182, 5836-5845
- *Annual Review of Immunology,* 1984, vol. 2, 199-218
- *Nature,* 2000, vol. 404, 995-999
- *European Journal of Pharmacology,* 2001, vol. 431, 103-110
- *Arthritis Research & Therapy,* 2012, vol. 14, 253-265
- *Journal of Immunology,* 2006, vol. 36, 3071-3081
- *Journal of Investigative Dermatology,* 1999, vol. 112, 456-462
- *The Journal of Clinical Investigation,* 2002, vol. 110, 955-963
- *The Journal of Clinical Investigation,* 2000, vol. 105, 625-631
- *Experimental Dermatology,* 2012, vol. 21, 901-905
- *American Journal of Pathology,* 1985, vol. 120, 323-325
- *European Journal of Dermatology,* 2005, vol. 15, 459-464
- *Pigment Cell & Melanoma Research,* 2014, vol. 27, 1075-1085
- *Journal of Investigative Dermatology,* 2015, vol. 135, 2530-2532
- *Nature Medicine,* 2014, 1043-1049
- *Journal of Clinical Investigation Insight,* 2016, e89790
- *Pharmacological Reports,* 2013, vol. 65, 1237-1246
- *Journal of Investigative Dermatology,* 2014, vol. 134, 2122-2130
- *Journal of Animal Science,* 2010, vol. 81, 699-705
- *British Journal of Ophthalmology,* 2012, vol. 96, 1332-1336
- *Gastroenterology,* 1997, vol. 113, 1560-1569
- *American Journal of Respiratory and Critical Care Medicine,* 1997, vol. 156, 766-775
- *Clinical & Experimental Allergy,* 2005, vol. 35, 461-466
- **SCHAPER et al.** *The Journal of Dermatological Science,* 2013, vol. 71 (1), 29-36
- **CURZYTEK et al.** *Pharmacological Reports,* 2013, vol. 65, 1237-1246
- **NAKAJIMA et al.** *Journal of Investigative Dermatology,* 2014, vol. 134, 2122-2130
- **WANG et al.** *Journal of Investigative Dermatology,* 2015, vol. 135, 2530-2532

- **ALLI et al.** *Journal of Immunology,* 2012, vol. 188, 477-486